(19) Europäisches Patentamt
European Patent Office
Office européen des brevets

(11) Veröffentlichungsnummer: 0 303 118
A2

(12) **EUROPÄISCHE PATENTANMELDUNG**

(21) Anmeldenummer: 88112290.7

(22) Anmeldetag: 29.07.88

(51) Int. Cl.⁴: C07D 231/44 , C07D 231/18 , A01N 43/56

(30) Priorität: 10.08.87 DE 3726529

(43) Veröffentlichungstag der Anmeldung: 15.02.89 Patentblatt 89/07

(84) Benannte Vertragsstaaten: BE CH DE ES FR GB IT LI NL

(71) Anmelder: BAYER AG
Konzernverwaltung RP Patentabteilung
D-5090 Leverkusen 1 Bayerwerk(DE)

(72) Erfinder: Gehring, Reinhold, Dr.
Dasnöckel 49
D-5600 Wuppertal 11(DE)
Erfinder: Jensen-Korte, Uta, Dr.
Geibelstrasse 9
D-4000 Düsseldorf 1(DE)
Erfinder: Schallner, Otto, Dr.
Noldeweg 22
D-4019 Monheim(DE)
Erfinder: Stetter, Jörg, Dr.
Gellertweg 4
D-5600 Wuppertal 1(DE)
Erfinder: Wroblowsky, Heinz-Jürgen, Dr.
Gladbacher Strasse 34
D-4018 Langenfeld(DE)
Erfinder: Marhold, Albrecht, Dr.
Carl-Duisberg-Strasse 329
D-5090 Leverkusen 1(DE)
Erfinder: Becker, Benedikt, Dr.
Metzkausener Strasse 14
D-4020 Mettmann(DE)
Erfinder: Behrenz, Wolfgang, Dr.
Untergründemich 14
D-5063 Overath(DE)
Erfinder: Homeyer, Bernhard, Dr.
Obere Strasse 28
D-5090 Leverkusen 3(DE)
Erfinder: Stendel, Wilhelm, Dr.
In den Birken 55
D-5600 Wuppertal 1(DE)
Erfinder: Andrews, Dr.
Gellertweg 2
D-5600 Wuppertal 1(DE)

(54) **1-Arylpyrazole.**

(57) Es werden neue 1-Arylpyrazole der Formel (I)

EP 0 303 118 A2

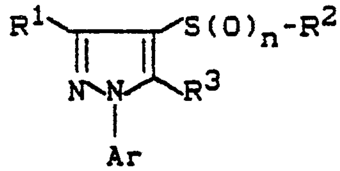

(I)

bereitgestellt, in welcher
$R^1$ für Wasserstoff oder Alkyl steht,
$R^2$ für Alkyl oder Halogenalkyl steht,
$R^3$ für Wasserstoff, Halogen,
oder für einen Rest

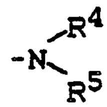

steht,
wobei
$R^4$ und $R^5$ unabhängig voneinander jeweils für Wasserstoff, Alkyl, Alkenyl oder Alkinyl stehen,
Ar für einen der Reste

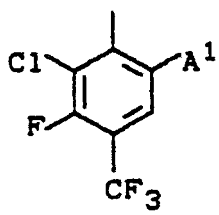 ; 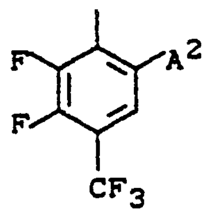 ; 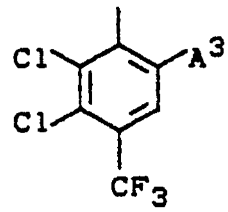 ;

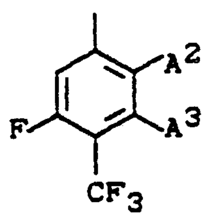 ; 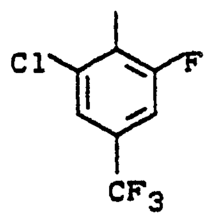 oder 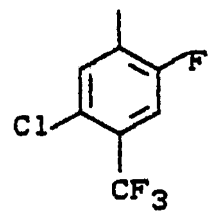

steht, wobei
$A^1$ für Wasserstoff, Fluor oder Chlor steht,
$A^2$ für Fluor oder Chlor steht und
$A^3$ für Wasserstoff oder Fluor steht und
n für eine Zahl 0, 1 oder 2 steht,
mit Ausnahme der Verbindung 5-Amino-1-(2,5-difluor-4-trifluormethyl-phenyl)-4-dichlorfluormethylthio-pyrazol.

Die neuen Verbindungen der Formel (I) zeigen eine ausgezeichnete Wirksamkeit gegenüber Insekten und/oder Nematoden und können als Wirkstoff in Schädlingsbekämpfungsmitteln eingesetzt werden.

# 1-Arylpyrazole

Die Erfindung betrifft neue 1-Arylpyrazole, mehrere Verfahren zu ihrer Herstellung sowie ihre Verwendung als Schädlingsbekämpfungsmittel.

Es ist bereits bekannt, daß bestimmte 1-Arylpyrazole, wie beispielsweise das 5-Methylamino-4-trifluormethylthio-1-(2,6-dichlor-4-trifluormethylphenyl)-pyrazol oder das 5-Methylamino-4-dichlorfluormethylthio-1-(2,6-dichlor-4-trifluorphenyl)-pyrazol insektizide Eigenschaften besitzen (vergl. EP 201 852).

Die Wirksamkeit dieser vorbekannten Verbindungen ist jedoch insbesondere bei niedrigen Aufwandmengen und -konzentrationen nicht in allen Anwendungsbereichen völlig zufriedenstellend.

Es wurden neue 1-Arylpyrazole der allgemeinen Formel (I),

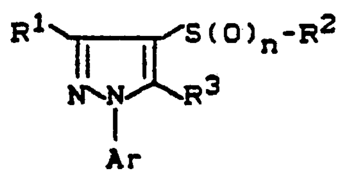

(I)

in welcher

$R^1$ für Wasserstoff oder Alkyl steht,

$R^2$ für Alkyl oder Halogenalkyl steht,

$R^3$ für Wasserstoff, Halogen, oder für einen Rest

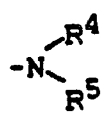

steht,

wobei

$R^4$ und $R^5$ unabhängig voneinander jeweils für Wasserstoff, Alkyl, Alkenyl oder Alkinyl stehen,

Ar für einen der Reste

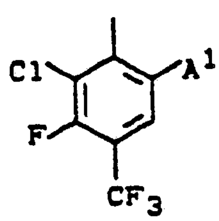 ; 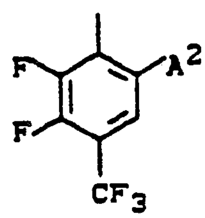 ; 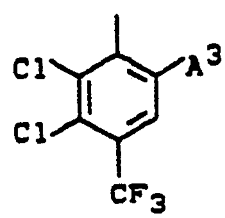 ;

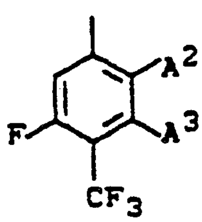 ; 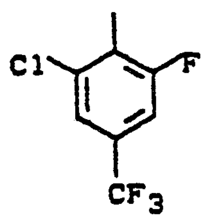 oder 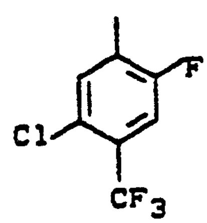

steht, wobei

$A^1$ für Wasserstoff, Fluor oder Chlor steht,

$A^2$ für Fluor oder Chlor steht und

$A^3$ für Wasserstoff oder Fluor steht und

n für eine Zahl 0, 1 oder 2 steht,

wobei jedoch die Verbindung 5-Amino-1-(2,5-difluor-4-trifluormethyl-phenyl)-4-dichlorfluormethylthio-pyrazol

ausgenommen ist,
gefunden.

Weiterhin wurde gefunden, daß man die neuen 1-Arylpyrazole der allgemeinen Formel (I),

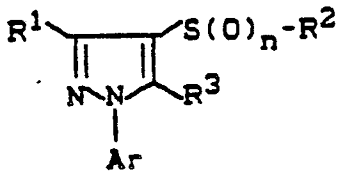

(I)

in welcher
$R^1$ für Wasserstoff oder Alkyl steht,
$R^2$ für Alkyl oder Halogenalkyl steht,
$R^3$ für Wasserstoff, Halogen,
oder für einen Rest

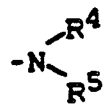

steht,
wobei
$R^4$ und $R^5$ unabhängig voneinander jeweils für Wasserstoff, Alkyl, Alkenyl oder Alkinyl stehen,
Ar für einen der Reste

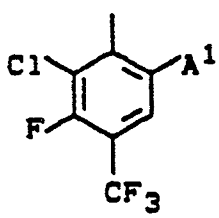 ; 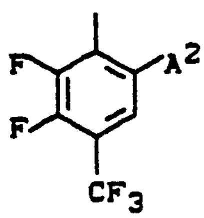 ; 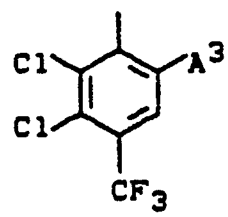 ;

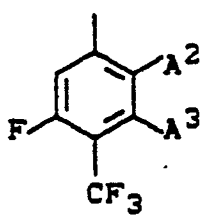 ; 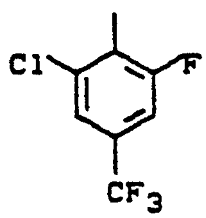 oder 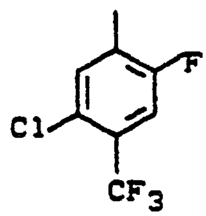

steht, wobei
$A^1$ für Wasserstoff, Fluor oder Chlor steht,
$A^2$ für Fluor oder Chlor steht und
$A^3$ für Wasserstoff oder Fluor steht und
n für eine Zahl 0, 1 oder 2 steht,
wobei jedoch die Verbindung 5-Amino-1-(2,5-difluor-4-trifluormethyl-phenyl)-4-dichlorfluormethylthio-pyrazol ausgenommen ist,
nach einem der im Folgenden beschriebenen Verfahren erhält:

(a) Man erhält 1-Arylpyrazole der Formel (Ia),

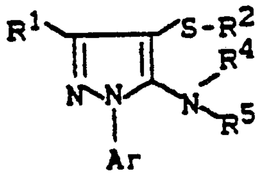

(Ia)

in welcher
$R^1$, $R^2$, $R^4$, $R^5$ und Ar die oben angegebene Bedeutung haben,
wenn man 4-unsubstituierte 1-Arylpyrazole der Formel (II),

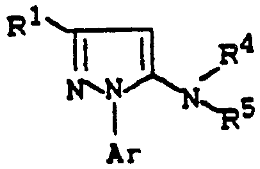

(II)

in welcher
$R^1$, $R^4$, $R^5$ und Ar die oben angegebene Bedeutung haben,
mit Sulfenylhalogeniden der Formel (III),

$R^2$-S-$Hal^1$ (III)

in welcher
$Hal^1$ für Halogen steht und
$R^2$ die oben angegebene Bedeutung hat,
gegebenenfalls in Gegenwart eines Verdünnungsmittels und gegebenenfalls in Gegenwart eines Reaktionshilfsmittels umsetzt;

(b) man erhält 1-Arylpyrazole der Formel (Ib),

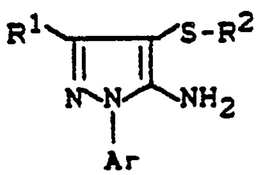

(Ib)

in welcher
$R^1$, $R^2$ und Ar die oben angegebene Bedeutung haben,
alternativ auch, wenn man
($\alpha$) Thiocyanate der Formel (IV),

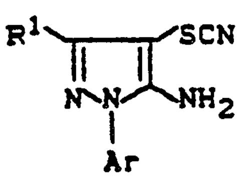

(IV)

in welcher
$R^1$ und Ar die oben angegebene Bedeutung haben,
oder
($\beta$) Disulfide der Formel (V),

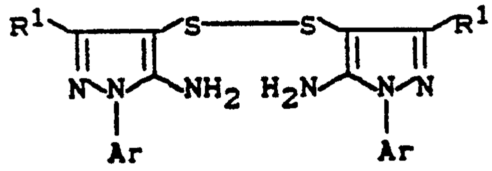

(V)

in welcher
$R^1$ und Ar die oben angegebene Bedeutung haben,
mit Halogeniden der Formel (VI),

$R^2$-$Hal^2$ (VI)

in welcher
$Hal^2$ für Halogen steht und
$R^2$ die oben angegebene Bedeutung hat,
in Gegenwart eines geeigneten Reduktionsmittels gegebenenfalls in Gegenwart eines Verdünnungsmittels und gegebenenefalls in Gegenwart eines Reaktionshilfsmittels umsetzt;

(c) man erhält 1-Arylpyrazole der Formel (Ic),

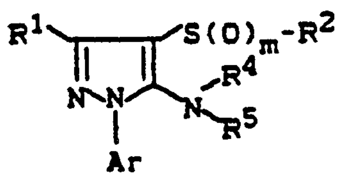

(Ic)

in welcher
$R^1$, $R^2$, $R^4$, $R^5$ und Ar die oben angegebene Bedeutung haben und
m für eine Zahl 1 oder 2 steht,
wenn man 1-Arylpyrazole der Formel (Ia),

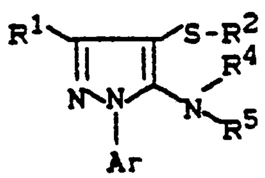

(Ia)

in welcher
$R^1$, $R^2$, $R^4$, $R^5$ und Ar die oben angegebene Bedeutung haben,
mit einem geeigneten Oxidationsmittel, gegebenenfalls in Gegenwart eines Verdünnungsmittels und gegebenenfalls in Gegenwart eines Reaktionshilfsmittels sowie gegebenenfalls in Gegenwart eines geeigneten Katalysators, umsetzt;

(d) man erhält 1-Arylpyrazole der Formel (Id),

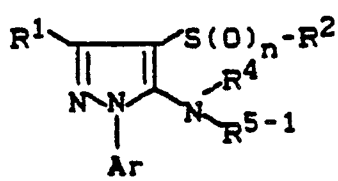

(Id)

in welcher
$R^1$, $R^2$, $R^4$, Ar und n die oben angegebene Bedeutung haben und
$R^{5-1}$ für Alkyl, Alkenyl oder Alkinyl steht,
wenn man 1-Arylpyrazole der Formel (Ig),

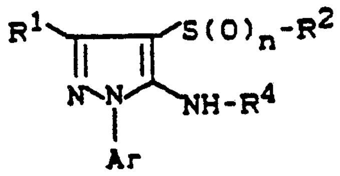

(Ig)

in welcher
$R^1$, $R^2$, $R^4$, Ar und n die oben angegebene Bedeutung haben,
mit Alkylierungsmitteln der formel (VII),

$R^{5-1}$-E (VII)

in welcher
$R^{5-1}$ für Alkyl, Alkenyl oder Alkinyl steht und
E für eine elektronenanziehende Abgangsgruppe steht,
gegebenenfalls in Gegenwart eines Verdünnungsmittels und gegebenenfalls in Gegenwart eines Reaktionshilfsmittels umsetzt;

(e) man erhält 1-Arylpyrazole der Formel (Ie),

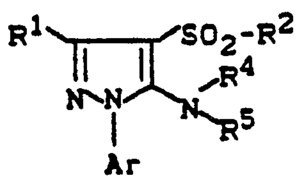

(Ie)

in welcher
$R^1$, $R^2$, $R^4$, $R^5$ und Ar die oben angegebene Bedeutung haben,
wenn man 1-Arylpyrazole der Formel (Ih),

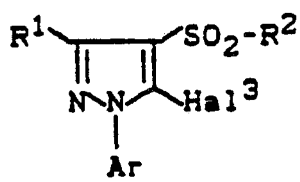

(Ih)

in welcher
$Hal^3$ für Halogen steht und
$R^1$,$R^2$ und Ar die oben angegebene Bedeutung haben,
mit Aminen der Formel (VIII),

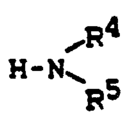

(VIII)

in welcher
$R^4$ und $R^5$ die oben angegebene Bedeutung haben,
gegebenenfalls in Gegenwart eines Verdünnungsmittels und gegebenenfalls in Gegenwart eines Reaktionshilfsmittels umsetzt;

(f) man erhält 1-Arylpyrazole der Formel (If),

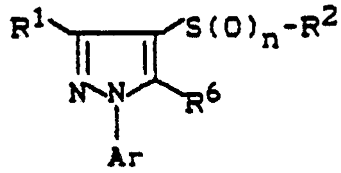

(If)

in welcher
$R^1$, $R^2$, Ar und n die oben angegebene Bedeutung haben und
$R^6$ für Wasserstoff oder Halogen steht,
wenn man 1-Arylpyrazole der Formel (Ii),

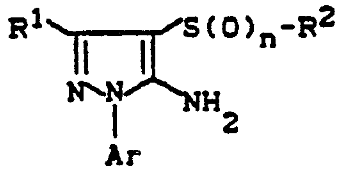

(Ii)

in welcher
$R^1$, $R^2$, Ar und n die oben angegebene Bedeutung haben,
mit einem anorganischen oder organischen Nitrit, gegebenenfalls in Gegenwart eins Reaktionshilfsmittels und gegebenenfalls in Gegenwart einer Halogenwasserstoffsäure sowie gegebenenfalls in Gegenwart eines Verdünnungsmittels, umsetzt.

Schließlich wurde gefunden, daß die neuen 1-Arylpyrazole der allgemeinen Formel (I) eine gute insektizide Wirksamkeit besitzen.

Überraschenderweise zeigen die erfindungsgemäßen 1-Arylpyrazole der allgemeinen Formel (I) eine erheblich bessere insektizide Wirksamkeit als die aus dem Stand der Technik bekannten 1-Arylpyrazole, wie beispielsweise das 5-Methylamino-4-trifluormethylthio-1-(2,6-dichlor-4-trifluormethylphenyl)-pyrazol oder das 5-Methylamino-4-dichlorfluormethylthio-1-(2,6-dichlor-4-trifluormethylphenyl)-pyrazol welches chemisch und wirkungsmäßig naheliegende Verbindungen sind.

Die erfindungsgemäßen 1-Arylpyrazole sind durch die Formel (I) allgemein definiert. Bevorzugt sind Verbindungen der Formel (I), bei welchen
$R^1$ für Wasserstoff oder für geradkettiges oder verzweigtes Alkyl mit 1 bis 4 Kohlenstoffatomen steht,
$R^2$ für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,
$R^3$ für Wasserstoff, Fluor, Chlor, Brom oder für einen Rest

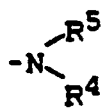

steht, wobei
$R^4$ und $R^5$ unabhängig voneinander jeweils für Wasserstoff oder für jeweils geradkettiges oder verzweigtes Alkyl, Alkenyl oder Alkinyl mit jeweils bis zu 6 Kohlenstoffatomen stehen,
Ar für einen der Reste

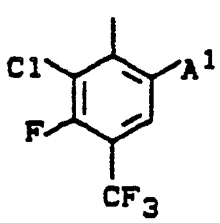 ; 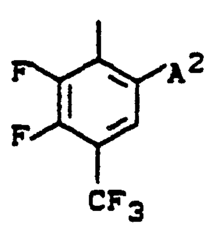 ; 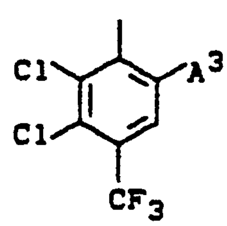 ;

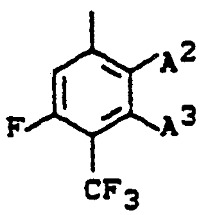 ; 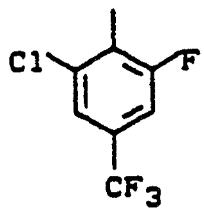 oder 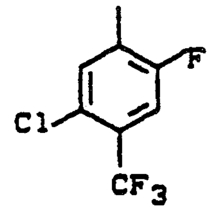

steht, wobei
$A^1$ für Wasserstoff, Fluor oder Chlor steht,
$A^2$ für Fluor oder Chlor steht und
$A^3$ für Wasserstoff oder Fluor steht und
n für eine Zahl 0, 1 oder 2 steht,
wobei jedoch die Verbindung 5-Amino-1-(2,5-difluor-4-trifluormethyl-phenyl)-4-dichlorfluormethylthio-pyrazol ausgenommen ist.

Besonders bevorzugt sind Verbindngen der Formel (I), bei welchen
$R^1$ für Wasserstoff, Methyl oder Ethyl steht,
$R^2$ für Methyl, Ethyl, n- oder i-Propyl, n-, i-, s-oder t-Butyl, Chlormethyl, Difluormethyl, Difluorchlormethyl, Fluordichlormethyl, Trifluormethyl, Pentafluorethyl, Pentachlorethyl, Fluortetrachlor ethyl, Difluortrichlorethyl, Trifluordichlorethyl, Tetrafluorchlorethyl, Heptafluorpropyl, Chlorethyl, Brommethyl, Chlorpropyl oder Brompropyl steht,
$R^3$ für Wasserstoff, Fluor, Chlor oder Brom oder für einen Rest

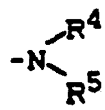

steht,
wobei
$R^4$ und $R^5$ unabhängig voneinander für Wasserstoff, Methyl, Ethyl, n- oder i-Propyl, n-, i-, s-oder t-Butyl, Allyl, n- oder i-Butenyl, Propargyl sowie n- oder i-Butinyl stehen,
Ar für einen der Reste

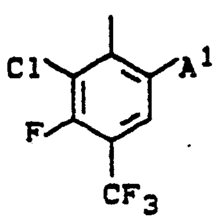 ; 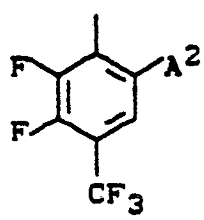 ; 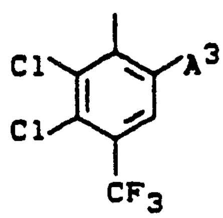 ;

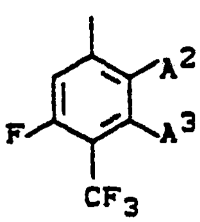 ; 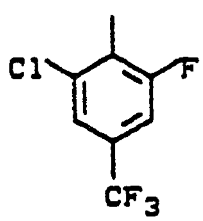 oder 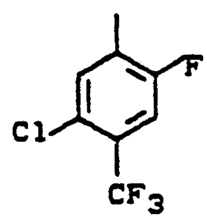

steht, wobei
$A^1$ für Wasserstoff, Fluor oder Chlor steht,
$A^2$ für Fluor oder Chlor steht und
$A^3$ für Wasserstoff oder Fluor steht und
n für eine Zahl 0, 1 oder 2 steht,
wobei jedoch die Verbindung 5-Amino-1-(2,5-difluor-4-trifluormethyl-phenyl)-4-dichlorfluormethylthio-pyrazol ausgenommen ist.

Ganz besonders bevorzugt sind Verbindungen der Formel (I), bei welchen
$R^1$ für Wasserstoff oder Methyl steht,
$R^2$ für Methyl, Ethyl, Trifluormethyl, Dichlorfluormethyl oder Difluorchlormethyl steht,
$R^3$ für Wasserstoff, Chlor, Brom oder für einen Rest

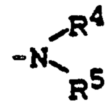

steht,
$R^4$ und $R^5$ unabhängig voneinander für Wasserstoff, Methyl, Ethyl, n- oder i-Propyl, n-, i-, s-Butyl, Allyl, n- oder i-Butenyl, Propargyl sowie n- oder i-Butinyl stehen,
Ar für einen der Reste

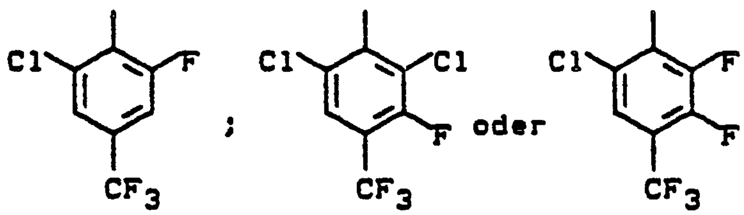

steht und n für eine Zahl 0, 1 oder 2 steht.

Im einzelnen seien außer den bei der Herstellungsbeispielen genannten Verbindungen die folgenden 1-Arylpyrazole der allgemeinen Formel (I) genannt:

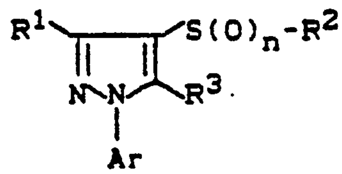

(I)

| $R^1$ | $R^2$ | $R^3$ | Ar | n |
|---|---|---|---|---|
| H | $CF_3$ | $-NH-C_2H_5$ | 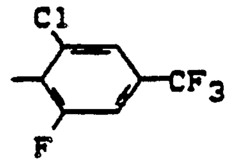 | 0 |
| H | $CF_3$ | $-NH-CH_3$ | 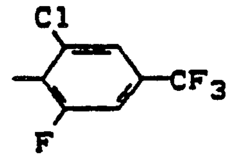 | 0 |
| H | $CF_3$ | $-N(CH_3)_2$ | 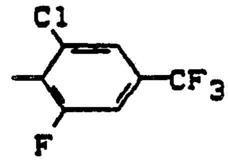 | 0 |
| H | $CF_3$ | $-N(C_2H_5)_2$ | 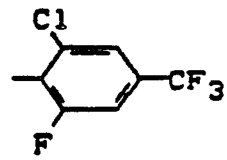 | 0 |
| H | $CF_3$ | 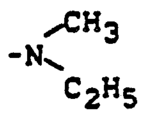 | 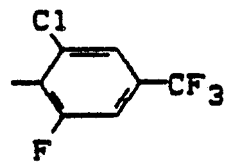 | 0 |
| H | $CF_3$ | $-NH-CH_2-CH=CH_2$ | 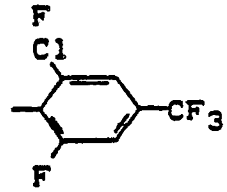 | 0 |
| H | $CF_3$ | $-N(CH_2-CH=CH_2)_2$ | 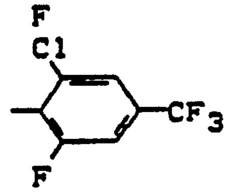 | 0 |
| H | $CF_3$ | $-N(CH_2-C\equiv CH)_2$ | 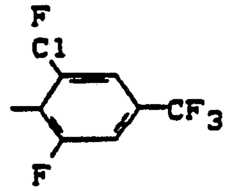 | 0 |

| $R^1$ | $R^2$ | $R^3$ | Ar | n |
|---|---|---|---|---|
| H | $CF_3$ | $-NH-CH_2-C\equiv CH$ | 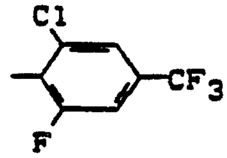 | 0 |
| H | $CF_3$ | $-N(CH_3)(CH_2-CH=CH_2)$ | 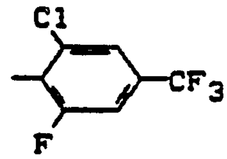 | 0 |
| H | $CF_3$ | $-N(CH_3)(CH_2-C\equiv CH)$ | 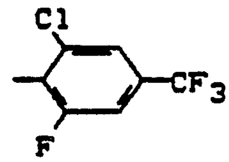 | 0 |
| H | $CF_3$ | $-NH-CH_2CH_2-CH_3$ | 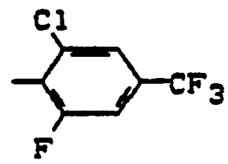 | 0 |
| H | $CF_3$ | $-NH-CH(CH_3)_2$ | 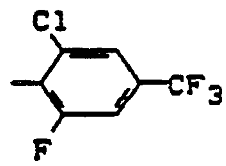 | 0 |
| H | $CF_3$ | $-N(CH_3)(CH_2-CH_2-CH_3)$ | 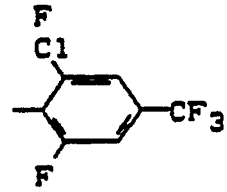 | 0 |
| H | $CF_3$ | $-N(CH_2-CH_2-CH_3)_2$ | 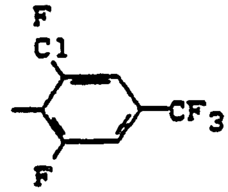 | 0 |
| H | $CF_3$ | $-NH-CH_2-CH=CH-CH_3$ | 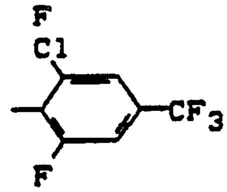 | 0 |
| H | $CF_3$ | $-NH-CH_2-C\equiv C-CH_3$ | 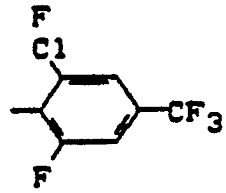 | 0 |

| $R^1$ | $R^2$ | $R^3$ | Ar | n |
|---|---|---|---|---|
| H | $CClF_2$ | $-NH-C_2H_5$ | 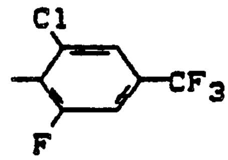 | 0 |
| H | $CClF_2$ | $-NH-CH_3$ | 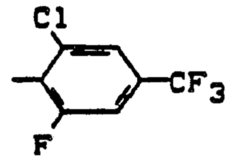 | 0 |
| H | $CClF_2$ | $-N(CH_3)_2$ | 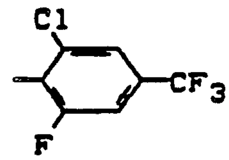 | 0 |
| H | $CClF_2$ | $-N(C_2H_5)_2$ | 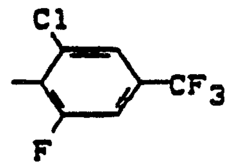 | 0 |
| H | $CClF_2$ | 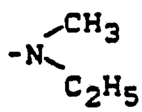 | 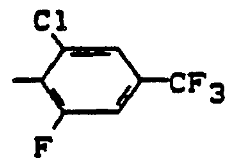 | 0 |
| H | $CClF_2$ | $-NH-CH_2-CH=CH_2$ | 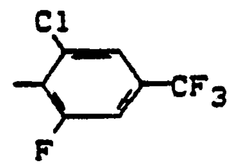 | 0 |
| H | $CClF_2$ | $-N(CH_2-CH=CH_2)_2$ | 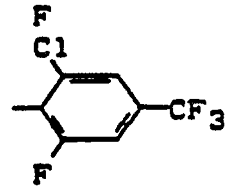 | 0 |
| H | $CClF_2$ | $-N(CH_2-C\equiv CH)_2$ | 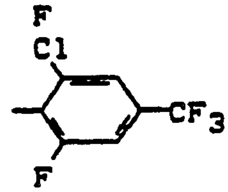 | 0 |

| $R^1$ | $R^2$ | $R^3$ | Ar | n |
|---|---|---|---|---|
| H | $CClF_2$ | $-NH-CH_2-C\equiv CH$ | 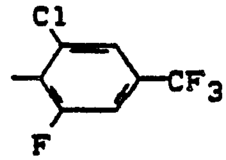 | 0 |
| H | $CClF_2$ | $CH_3$<br>$-N$<br>$CH_2-CH=CH_2$ | 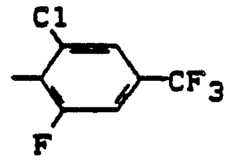 | 0 |
| H | $CClF_2$ | $CH_3$<br>$-N$<br>$CH_2-C\equiv CH$ | 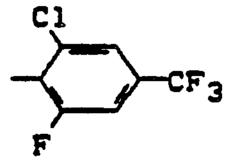 | 0 |
| H | $CClF_2$ | $-NH-CH_2CH_2-CH_3$ | 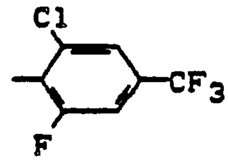 | 0 |
| H | $CClF_2$ | $-NH-CH(CH_3)_2$ | 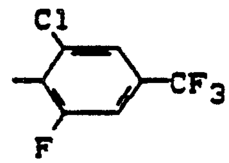 | 0 |
| H | $CClF_2$ | $CH_3$<br>$-N$<br>$CH_2-CH_2-CH_3$ | 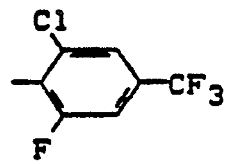 | 0 |
| H | $CClF_2$ | $-N(CH_2-CH_2-CH_3)_2$ | 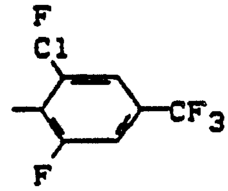 | 0 |
| H | $CClF_2$ | $-NH-CH_2-CH=CH-CH_3$ | 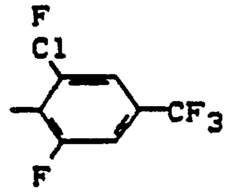 | 0 |
| H | $CClF_2$ | $-NH-CH_2-C\equiv C-CH_3$ | 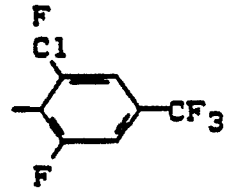 | 0 |

| $R^1$ | $R^2$ | $R^3$ | Ar | n |
|---|---|---|---|---|
| H | $CCl_2F$ | $-NH-C_2H_5$ | 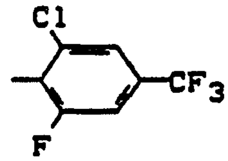 | 0 |
| H | $CCl_2F$ | $-NH-CH_3$ | 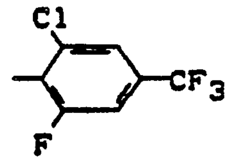 | 0 |
| H | $CCl_2F$ | $-N(CH_3)_2$ | 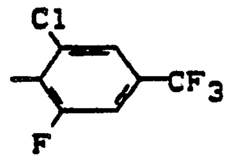 | 0 |
| H | $CCl_2F$ | $-N(C_2H_5)_2$ | 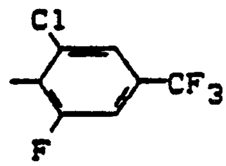 | 0 |
| H | $CCl_2F$ | 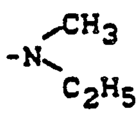 | 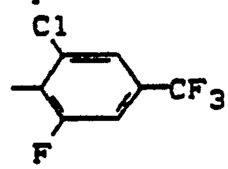 | 0 |
| H | $CCl_2F$ | $-NH-CH_2-CH=CH_2$ | 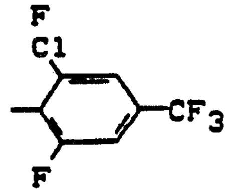 | 0 |
| H | $CCl_2F$ | $-N(CH_2-CH=CH_2)_2$ | 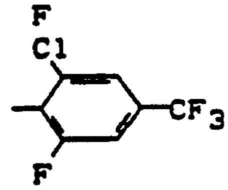 | 0 |
| H | $CCl_2F$ | $-N(CH_2-C\equiv CH)_2$ | 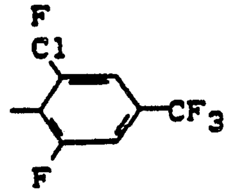 | 0 |

| $R^1$ | $R^2$ | $R^3$ | Ar | n |
|---|---|---|---|---|
| H | $CCl_2F$ | $-NH-CH_2-C{\equiv}CH$ | 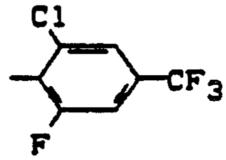 | 0 |
| H | $CCl_2F$ | $-N(CH_3)(CH_2-CH{=}CH_2)$ | 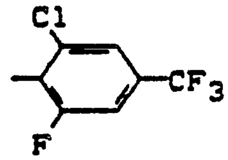 | 0 |
| H | $CCl_2F$ | $-N(CH_3)(CH_2-C{\equiv}CH)$ | 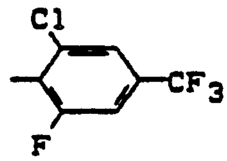 | 0 |
| H | $CCl_2F$ | $-NH-CH_2CH_2-CH_3$ | 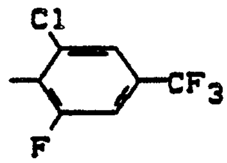 | 0 |
| H | $CCl_2F$ | $-NH-CH(CH_3)_2$ | 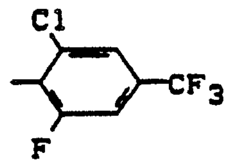 | 0 |
| H | $CCl_2F$ | $-N(CH_3)(CH_2-CH_2-CH_3)$ | 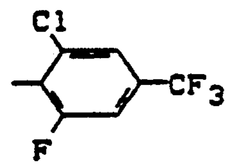 | 0 |
| H | $CCl_2F$ | $-N(CH_2-CH_2-CH_3)_2$ | 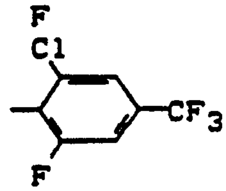 | 0 |
| H | $CCl_2F$ | $-NH-CH_2-CH{=}CH-CH_3$ | 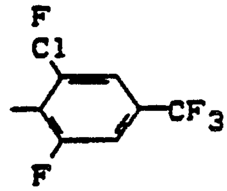 | 0 |
| H | $CCl_2F$ | $-NH-CH_2-C{\equiv}C-CH_3$ | 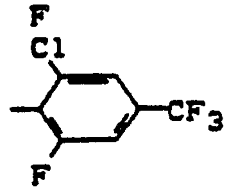 | 0 |

| $R^1$ | $R^2$ | $R^3$ | Ar | n |
|---|---|---|---|---|
| $CH_3$ | $CF_3$ | $-NH-C_2H_5$ | 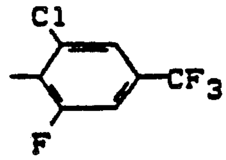 | 0 |
| $CH_3$ | $CF_3$ | $-NH-CH_3$ | 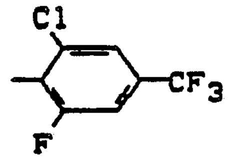 | 0 |
| $CH_3$ | $CF_3$ | $-N(CH_3)_2$ | 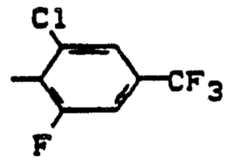 | 0 |
| $CH_3$ | $CF_3$ | $-N(C_2H_5)_2$ | 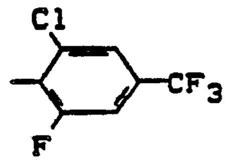 | 0 |
| $CH_3$ | $CF_3$ | 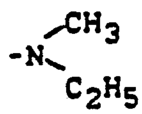 | 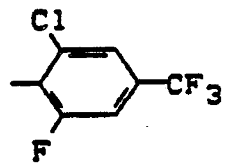 | 0 |
| $CH_3$ | $CF_3$ | $-NH-CH_2-CH{=}CH_2$ | 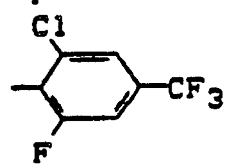 | 0 |
| $CH_3$ | $CF_3$ | $-N(CH_2-CH{=}CH_2)_2$ | 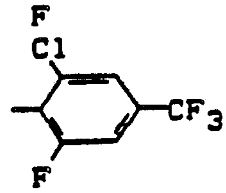 | 0 |
| $CH_3$ | $CF_3$ | $-N(CH_2-C{\equiv}CH)_2$ | 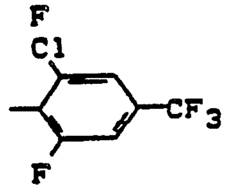 | 0 |

| $R^1$ | $R^2$ | $R^3$ | Ar | n |
|---|---|---|---|---|
| $CH_3$ | $CF_3$ | $-NH-CH_2-C\equiv CH$ | 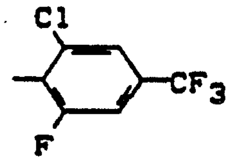 | 0 |
| $CH_3$ | $CF_3$ | $-N(CH_3)CH_2-CH=CH_2$ | 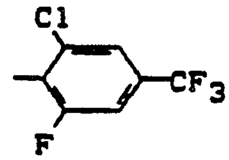 | 0 |
| $CH_3$ | $CF_3$ | $-N(CH_3)CH_2-C\equiv CH$ | 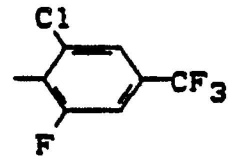 | 0 |
| $CH_3$ | $CF_3$ | $-NH-CH_2CH_2-CH_3$ | 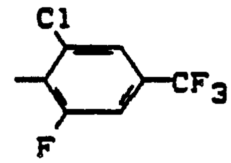 | 0 |
| $CH_3$ | $CF_3$ | $-NH-CH(CH_3)_2$ | 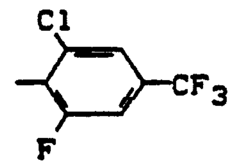 | 0 |
| $CH_3$ | $CF_3$ | $-N(CH_3)CH_2-CH_2-CH_3$ | 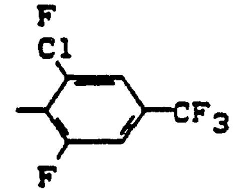 | 0 |
| $CH_3$ | $CF_3$ | $-N(CH_2-CH_2-CH_3)_2$ | 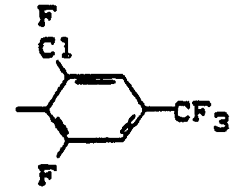 | 0 |
| $CH_3$ | $CF_3$ | $-NH-CH_2-CH=CH-CH_3$ | 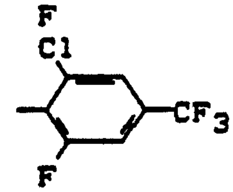 | 0 |
| $CH_3$ | $CF_3$ | $-NH-CH_2-C\equiv C-CH_3$ | 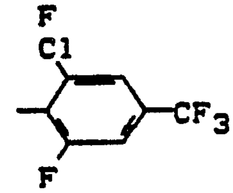 | 0 |

| $R^1$ | $R^2$ | $R^3$ | Ar | n |
|---|---|---|---|---|
| $CH_3$ | $CClF_2$ | $-NH-C_2H_5$ | 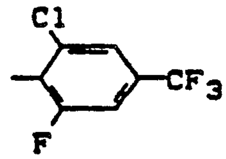 | 0 |
| $CH_3$ | $CClF_2$ | $-NH-CH_3$ | 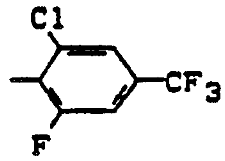 | 0 |
| $CH_3$ | $CClF_2$ | $-N(CH_3)_2$ | 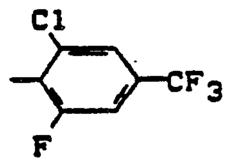 | 0 |
| $CH_3$ | $CClF_2$ | $-N(C_2H_5)_2$ | 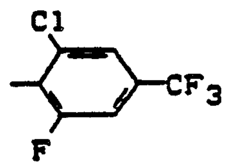 | 0 |
| $CH_3$ | $CClF_2$ | 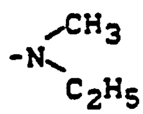 | 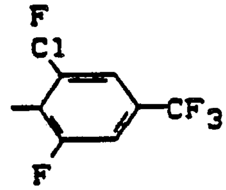 | 0 |
| $CH_3$ | $CClF_2$ | $-NH-CH_2-CH=CH_2$ | 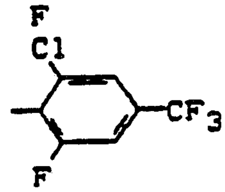 | 0 |
| $CH_3$ | $CClF_2$ | $-N(CH_2-CH=CH_2)_2$ | 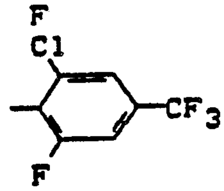 | 0 |
| $CH_3$ | $CClF_2$ | $-N(CH_2-C\equiv CH)_2$ | 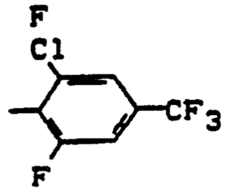 | 0 |

| $R^1$ | $R^2$ | $R^3$ | Ar | n |
|---|---|---|---|---|
| $CH_3$ | $CClF_2$ | $-NH-CH_2-C{\equiv}CH$ | 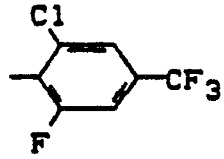 | 0 |
| $CH_3$ | $CClF_2$ | $CH_3$<br>-N<br>$CH_2-CH{=}CH_2$ | 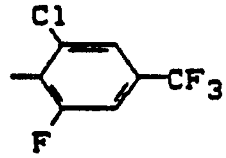 | 0 |
| $CH_3$ | $CClF_2$ | $CH_3$<br>-N<br>$CH_2-C{\equiv}CH$ | 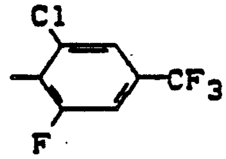 | 0 |
| $CH_3$ | $CClF_2$ | $-NH-CH_2CH_2-CH_3$ | 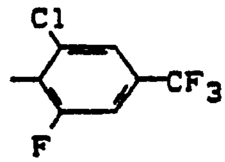 | 0 |
| $CH_3$ | $CClF_2$ | $-NH-CH(CH_3)_2$ | 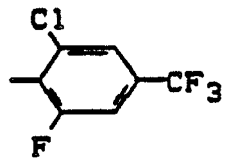 | 0 |
| $CH_3$ | $CClF_2$ | $CH_3$<br>-N<br>$CH_2-CH_2-CH_3$ | 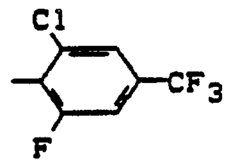 | 0 |
| $CH_3$ | $CClF_2$ | $-N(CH_2-CH_2-CH_3)_2$ | 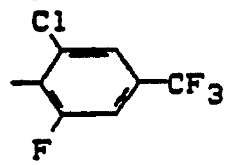 | 0 |
| $CH_3$ | $CClF_2$ | $-NH-CH_2-CH{=}CH-CH_3$ | 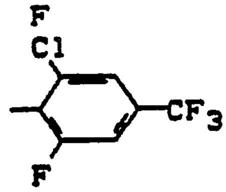 | 0 |
| $CH_3$ | $CClF_2$ | $-NH-CH_2-C{\equiv}C-CH_3$ | 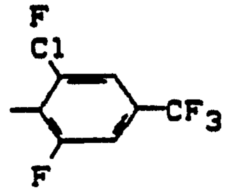 | 0 |

| $R^1$ | $R^2$ | $R^3$ | Ar | n |
|---|---|---|---|---|
| $CH_3$ | $CCl_2F$ | $-NH-C_2H_5$ | 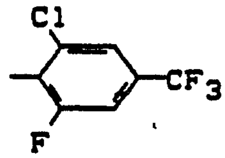 | 0 |
| $CH_3$ | $CCl_2F$ | $-NH-CH_3$ | 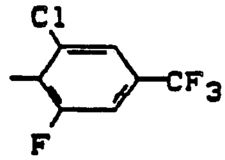 | 0 |
| $CH_3$ | $CCl_2F$ | $-N(CH_3)_2$ | 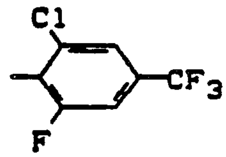 | 0 |
| $CH_3$ | $CCl_2F$ | $-N(C_2H_5)_2$ | 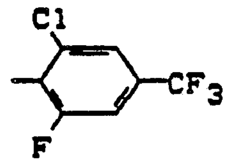 | 0 |
| $CH_3$ | $CCl_2F$ | 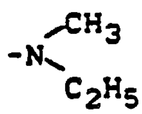 | 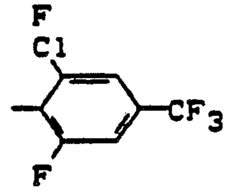 | 0 |
| $CH_3$ | $CCl_2F$ | $-NH-CH_2-CH=CH_2$ | 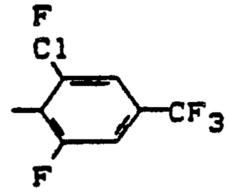 | 0 |
| $CH_3$ | $CCl_2F$ | $-N(CH_2-CH=CH_2)_2$ | 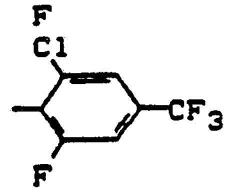 | 0 |
| $CH_3$ | $CCl_2F$ | $-N(CH_2-C\equiv CH)_2$ | 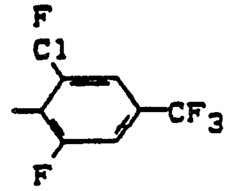 | 0 |

| $R^1$ | $R^2$ | $R^3$ | Ar | n |
|---|---|---|---|---|
| $CH_3$ | $CCl_2F$ | $-NH-CH_2-C\equiv CH$ | 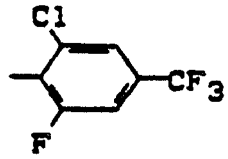 | 0 |
| $CH_3$ | $CCl_2F$ | $CH_3$<br>$-N$<br>$CH_2-CH=CH_2$ | 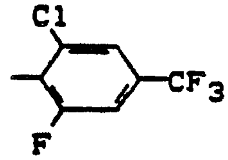 | 0 |
| $CH_3$ | $CCl_2F$ | $CH_3$<br>$-N$<br>$CH_2-C\equiv CH$ | 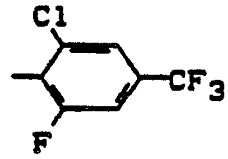 | 0 |
| $CH_3$ | $CCl_2F$ | $-NH-CH_2CH_2-CH_3$ | 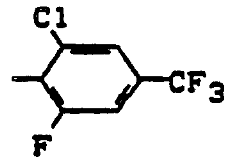 | 0 |
| $CH_3$ | $CCl_2F$ | $-NH-CH(CH_3)_2$ | 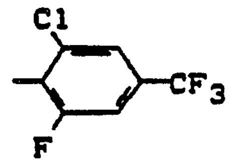 | 0 |
| $CH_3$ | $CCl_2F$ | $CH_3$<br>$-N$<br>$CH_2-CH_2-CH_3$ | 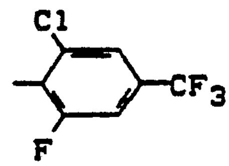 | 0 |
| $CH_3$ | $CCl_2F$ | $-N(CH_2-CH_2-CH_3)_2$ | 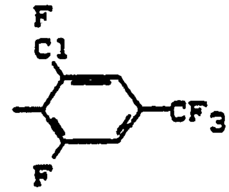 | 0 |
| $CH_3$ | $CCl_2F$ | $-NH-CH_2-CH=CH-CH_3$ | 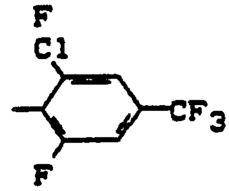 | 0 |
| $CH_3$ | $CCl_2F$ | $-NH-CH_2-C\equiv C-CH_3$ | 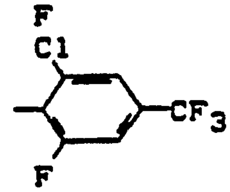 | 0 |

| $R^1$ | $R^2$ | $R^3$ | Ar | n |
|---|---|---|---|---|
| H | $CF_3$ | $-NH-C_2H_5$ | 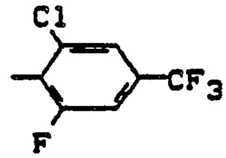 | 1 |
| H | $CF_3$ | $-NH-CH_3$ | 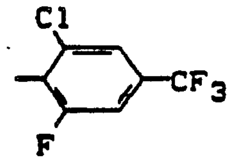 | 1 |
| H | $CF_3$ | $-N(CH_3)_2$ | 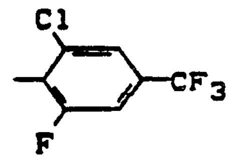 | 1 |
| H | $CF_3$ | $-N(C_2H_5)_2$ | 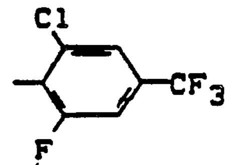 | 1 |
| H | $CF_3$ | $-N(CH_3)(C_2H_5)$ | 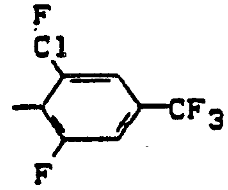 | 1 |
| H | $CF_3$ | $-NH-CH_2-CH=CH_2$ | 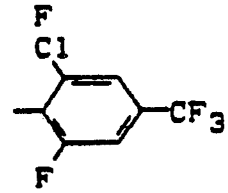 | 1 |
| H | $CF_3$ | $-N(CH_2-CH=CH_2)_2$ | 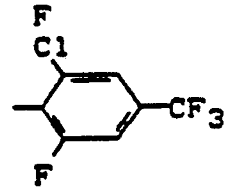 | 1 |
| H | $CF_3$ | $-N(CH_2-C{\equiv}CH)_2$ | 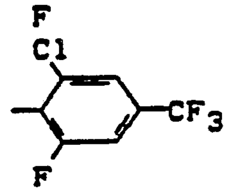 | 1 |

| $R^1$ | $R^2$ | $R^3$ | Ar | n |
|---|---|---|---|---|
| H | $CF_3$ | $-NH-CH_2-C\equiv CH$ | 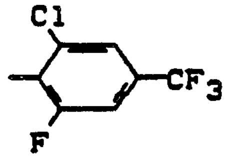 | 1 |
| H | $CF_3$ | $CH_3$<br>$-N$<br>$CH_2-CH=CH_2$ | 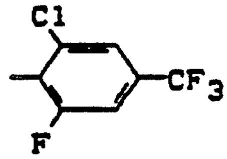 | 1 |
| H | $CF_3$ | $CH_3$<br>$-N$<br>$CH_2-C\equiv CH$ | 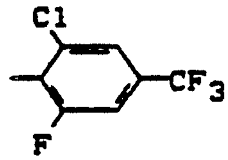 | 1 |
| H | $CF_3$ | $-NH-CH_2CH_2-CH_3$ | 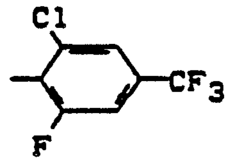 | 1 |
| H | $CF_3$ | $-NH-CH(CH_3)_2$ | 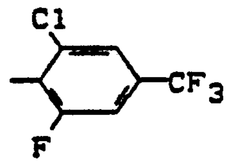 | 1 |
| H | $CF_3$ | $CH_3$<br>$-N$<br>$CH_2-CH_2-CH_3$ | 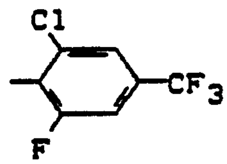 | 1 |
| H | $CF_3$ | $-N(CH_2-CH_2-CH_3)_2$ | 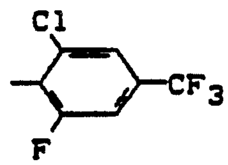 | 1 |
| H | $CF_3$ | $-NH-CH_2-CH=CH-CH_3$ | 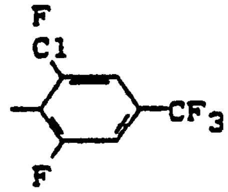 | 1 |
| H | $CF_3$ | $-NH-CH_2-C\equiv C-CH_3$ | 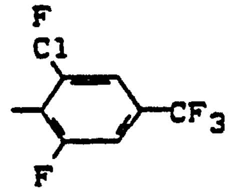 | 1 |

| $R^1$ | $R^2$ | $R^3$ | Ar | n |
|---|---|---|---|---|
| H | $CClF_2$ | $-NH-C_2H_5$ | 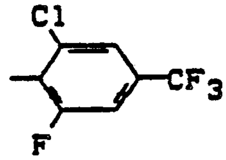 | 1 |
| H | $CClF_2$ | $-NH-CH_3$ | 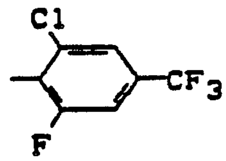 | 1 |
| H | $CClF_2$ | $-N(CH_3)_2$ | 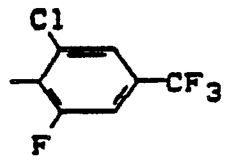 | 1 |
| H | $CClF_2$ | $-N(C_2H_5)_2$ | 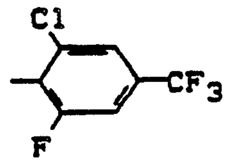 | 1 |
| H | $CClF_2$ | 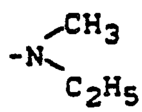 | 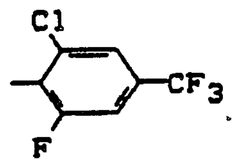 | 1 |
| H | $CClF_2$ | $-NH-CH_2-CH=CH_2$ | 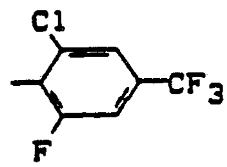 | 1 |
| H | $CClF_2$ | $-N(CH_2-CH=CH_2)_2$ | 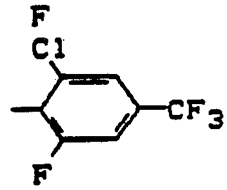 | 1 |
| H | $CClF_2$ | $-N(CH_2-C\equiv CH)_2$ | 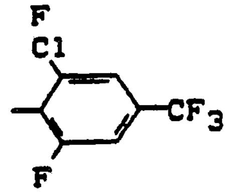 | 1 |

| $R^1$ | $R^2$ | $R^3$ | Ar | n |
|---|---|---|---|---|
| H | $CClF_2$ | $-NH-CH_2-C≡CH$ | 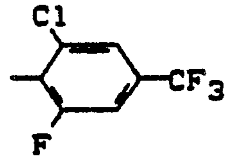 | 1 |
| H | $CClF_2$ | $-N(CH_3)(CH_2-CH=CH_2)$ | 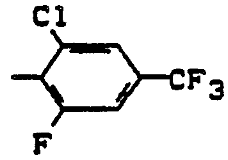 | 1 |
| H | $CClF_2$ | $-N(CH_3)(CH_2-C≡CH)$ | 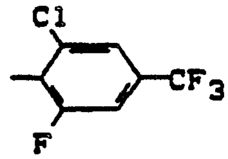 | 1 |
| H | $CClF_2$ | $-NH-CH_2CH_2-CH_3$ | 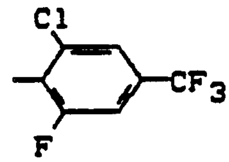 | 1 |
| H | $CClF_2$ | $-NH-CH(CH_3)_2$ | 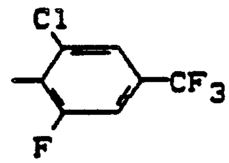 | 1 |
| H | $CClF_2$ | $-N(CH_3)(CH_2-CH_2-CH_3)$ | 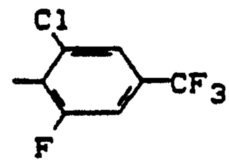 | 1 |
| H | $CClF_2$ | $-N(CH_2-CH_2-CH_3)_2$ | 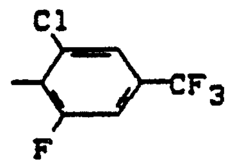 | 1 |
| H | $CClF_2$ | $-NH-CH_2-CH=CH-CH_3$ | 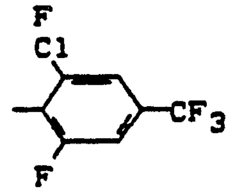 | 1 |
| H | $CClF_2$ | $-NH-CH_2-C≡C-CH_3$ | 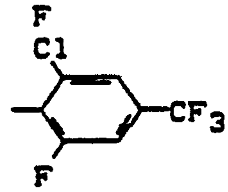 | 1 |

| $R^1$ | $R^2$ | $R^3$ | Ar | n |
|---|---|---|---|---|
| H | $CCl_2F$ | $-NH-C_2H_5$ | 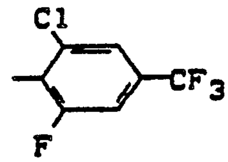 | 1 |
| H | $CCl_2F$ | $-NH-CH_3$ | 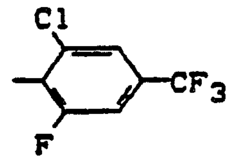 | 1 |
| H | $CCl_2F$ | $-N(CH_3)_2$ | 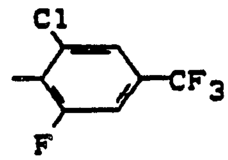 | 1 |
| H | $CCl_2F$ | $-N(C_2H_5)_2$ | 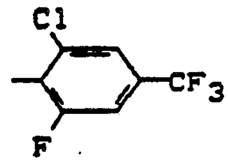 | 1 |
| H | $CCl_2F$ | 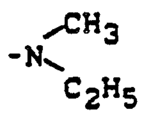 | 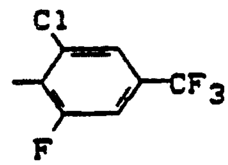 | 1 |
| H | $CCl_2F$ | $-NH-CH_2-CH=CH_2$ | 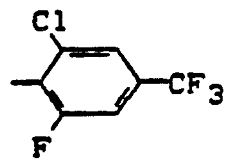 | 1 |
| H | $CCl_2F$ | $-N(CH_2-CH=CH_2)_2$ | 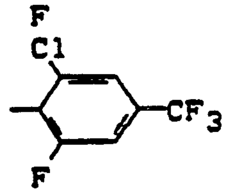 | 1 |
| H | $CCl_2F$ | $-N(CH_2-C\equiv CH)_2$ | 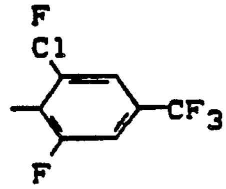 | 1 |

| $R^1$ | $R^2$ | $R^3$ | Ar | n |
|---|---|---|---|---|
| H | $CCl_2F$ | $-NH-CH_2-C\equiv CH$ | 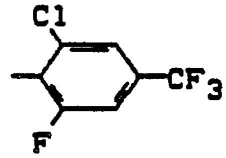 | 1 |
| H | $CCl_2F$ | 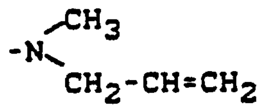 | 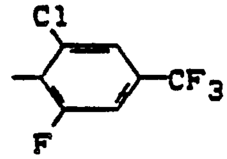 | 1 |
| H | $CCl_2F$ | 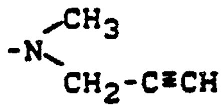 | 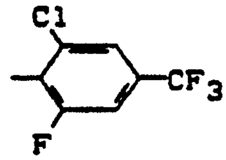 | 1 |
| H | $CCl_2F$ | $-NH-CH_2CH_2-CH_3$ | 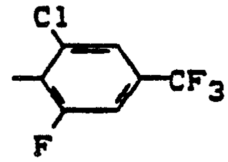 | 1 |
| H | $CCl_2F$ | $-NH-CH(CH_3)_2$ | 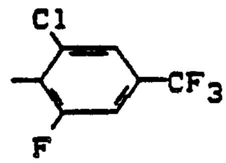 | 1 |
| H | $CCl_2F$ | 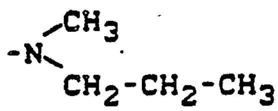 | 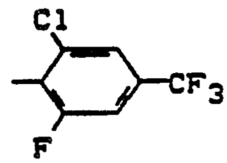 | 1 |
| H | $CCl_2F$ | $-N(CH_2-CH_2-CH_3)_2$ | 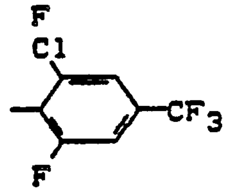 | 1 |
| H | $CCl_2F$ | $-NH-CH_2-CH=CH-CH_3$ | 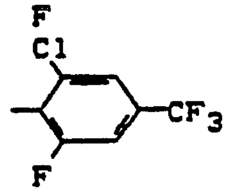 | 1 |
| H | $CCl_2F$ | $-NH-CH_2-C\equiv C-CH_3$ | 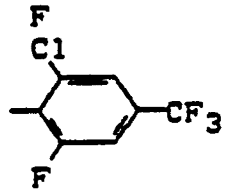 | 1 |

| $R^1$ | $R^2$ | $R^3$ | Ar | n |
|---|---|---|---|---|
| $CH_3$ | $CF_3$ | $-NH-C_2H_5$ | 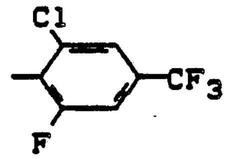 | 1 |
| $CH_3$ | $CF_3$ | $-NH-CH_3$ | 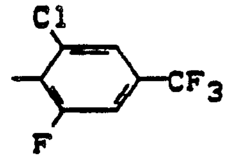 | 1 |
| $CH_3$ | $CF_3$ | $-N(CH_3)_2$ | 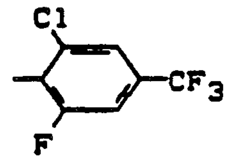 | 1 |
| $CH_3$ | $CF_3$ | $-N(C_2H_5)_2$ | 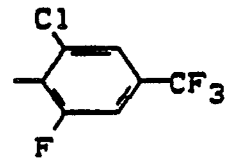 | 1 |
| $CH_3$ | $CF_3$ | 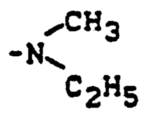 | 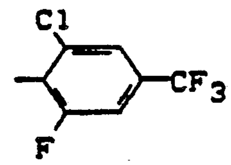 | 1 |
| $CH_3$ | $CF_3$ | $-NH-CH_2-CH=CH_2$ | 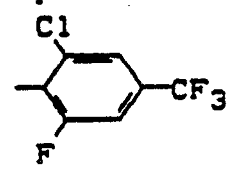 | 1 |
| $CH_3$ | $CF_3$ | $-N(CH_2-CH=CH_2)_2$ | 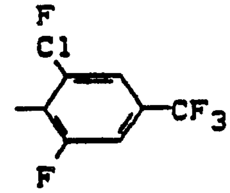 | 1 |
| $CH_3$ | $CF_3$ | $-N(CH_2-C\equiv CH)_2$ | 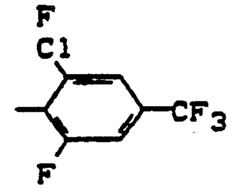 | 1 |

| $R^1$ | $R^2$ | $R^3$ | Ar | n |
|---|---|---|---|---|
| $CH_3$ | $CF_3$ | $-NH-CH_2-C\equiv CH$ | 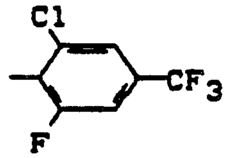 | 1 |
| $CH_3$ | $CF_3$ | $CH_3$<br>$-N$<br>$CH_2-CH=CH_2$ | 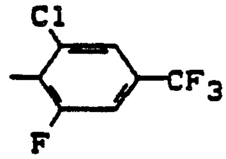 | 1 |
| $CH_3$ | $CF_3$ | $CH_3$<br>$-N$<br>$CH_2-C\equiv CH$ | 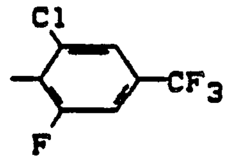 | 1 |
| $CH_3$ | $CF_3$ | $-NH-CH_2CH_2-CH_3$ | 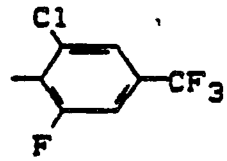 | 1 |
| $CH_3$ | $CF_3$ | $-NH-CH(CH_3)_2$ | 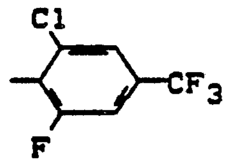 | 1 |
| $CH_3$ | $CF_3$ | $CH_3$<br>$-N$<br>$CH_2-CH_2-CH_3$ | 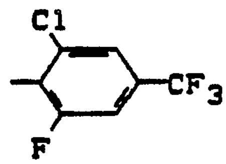 | 1 |
| $CH_3$ | $CF_3$ | $-N(CH_2-CH_2-CH_3)_2$ | 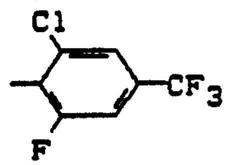 | 1 |
| $CH_3$ | $CF_3$ | $-NH-CH_2-CH=CH-CH_3$ | 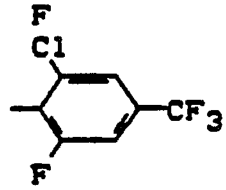 | 1 |
| $CH_3$ | $CF_3$ | $-NH-CH_2-C\equiv C-CH_3$ | 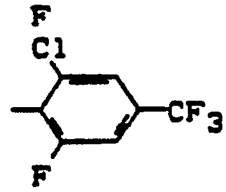 | 1 |

| $R^1$ | $R^2$ | $R^3$ | Ar | n |
|---|---|---|---|---|
| $CH_3$ | $CClF_2$ | $-NH-C_2H_5$ | 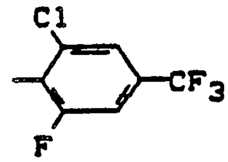 | 1 |
| $CH_3$ | $CClF_2$ | $-NH-CH_3$ | 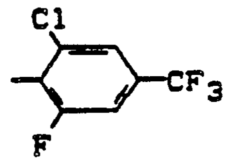 | 1 |
| $CH_3$ | $CClF_2$ | $-N(CH_3)_2$ | 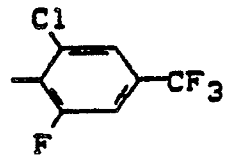 | 1 |
| $CH_3$ | $CClF_2$ | $-N(C_2H_5)_2$ | 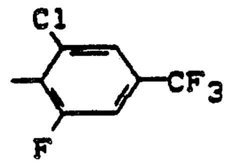 | 1 |
| $CH_3$ | $CClF_2$ | $-N(CH_3)(C_2H_5)$ | 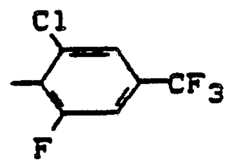 | 1 |
| $CH_3$ | $CClF_2$ | $-NH-CH_2-CH=CH_2$ | 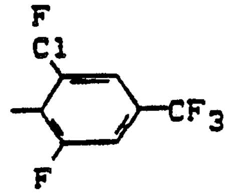 | 1 |
| $CH_3$ | $CClF_2$ | $-N(CH_2-CH=CH_2)_2$ | 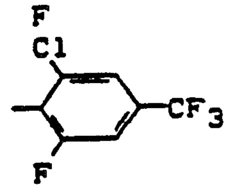 | 1 |
| $CH_3$ | $CClF_2$ | $-N(CH_2-C\equiv CH)_2$ | 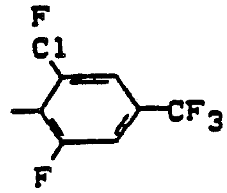 | 1 |

| $R^1$ | $R^2$ | $R^3$ | Ar | n |
|---|---|---|---|---|
| $CH_3$ | $CClF_2$ | $-NH-CH_2-C \equiv CH$ | 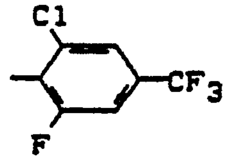 | 1 |
| $CH_3$ | $CClF_2$ | 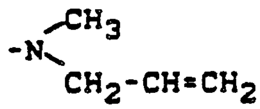 | 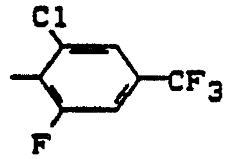 | 1 |
| $CH_3$ | $CClF_2$ | 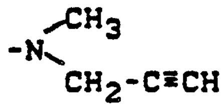 | 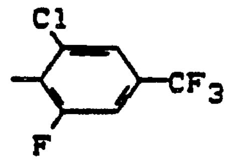 | 1 |
| $CH_3$ | $CClF_2$ | $-NH-CH_2CH_2-CH_3$ | 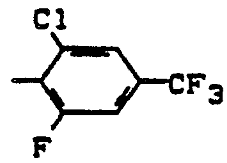 | 1 |
| $CH_3$ | $CClF_2$ | $-NH-CH(CH_3)_2$ | 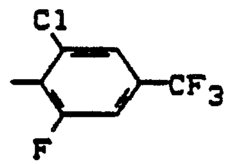 | 1 |
| $CH_3$ | $CClF_2$ | 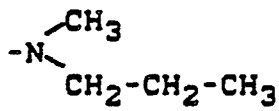 | 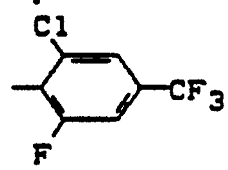 | 1 |
| $CH_3$ | $CClF_2$ | $-N(CH_2-CH_2-CH_3)_2$ | 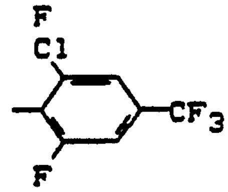 | 1 |
| $CH_3$ | $CClF_2$ | $-NH-CH_2-CH=CH-CH_3$ | 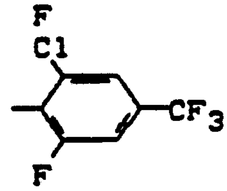 | 1 |
| $CH_3$ | $CClF_2$ | $-NH-CH_2-C \equiv C-CH_3$ | 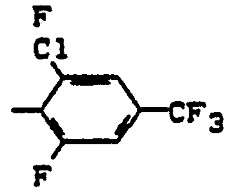 | 1 |

| $R^1$ | $R^2$ | $R^3$ | Ar | n |
|---|---|---|---|---|
| $CH_3$ | $CCl_2F$ | $-NH-C_2H_5$ | 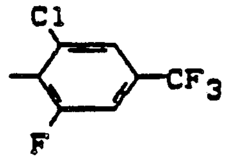 | 1 |
| $CH_3$ | $CCl_2F$ | $-NH-CH_3$ | 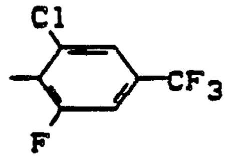 | 1 |
| $CH_3$ | $CCl_2F$ | $-N(CH_3)_2$ | 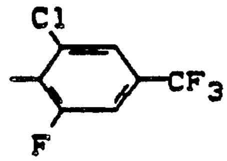 | 1 |
| $CH_3$ | $CCl_2F$ | $-N(C_2H_5)_2$ | 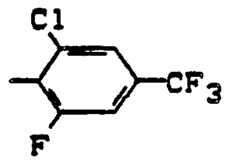 | 1 |
| $CH_3$ | $CCl_2F$ | 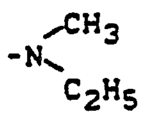 | 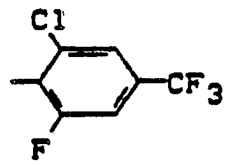 | 1 |
| $CH_3$ | $CCl_2F$ | $-NH-CH_2-CH=CH_2$ | 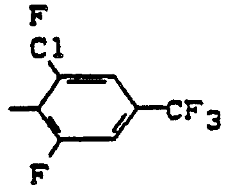 | 1 |
| $CH_3$ | $CCl_2F$ | $-N(CH_2-CH=CH_2)_2$ | 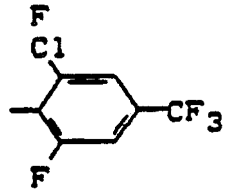 | 1 |
| $CH_3$ | $CCl_2F$ | $-N(CH_2-C≡CH)_2$ | 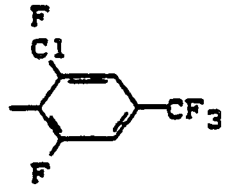 | 1 |

| $R^1$ | $R^2$ | $R^3$ | Ar | n |
|---|---|---|---|---|
| $CH_3$ | $CCl_2F$ | $-NH-CH_2-C\equiv CH$ | 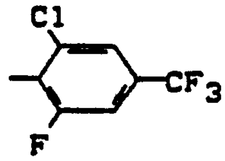 | 1 |
| $CH_3$ | $CCl_2F$ | $-N(CH_3)(CH_2-CH=CH_2)$ | 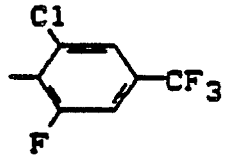 | 1 |
| $CH_3$ | $CCl_2F$ | $-N(CH_3)(CH_2-C\equiv CH)$ | 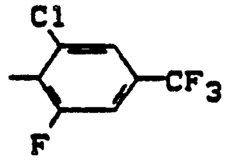 | 1 |
| $CH_3$ | $CCl_2F$ | $-NH-CH_2CH_2-CH_3$ | 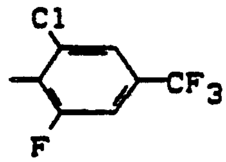 | 1 |
| $CH_3$ | $CCl_2F$ | $-NH-CH(CH_3)_2$ | 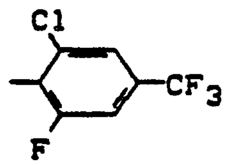 | 1 |
| $CH_3$ | $CCl_2F$ | $-N(CH_3)(CH_2-CH_2-CH_3)$ | 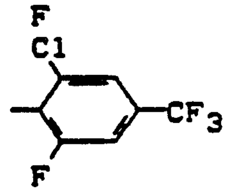 | 1 |
| $CH_3$ | $CCl_2F$ | $-N(CH_2-CH_2-CH_3)_2$ | 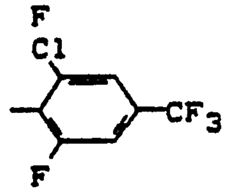 | 1 |
| $CH_3$ | $CCl_2F$ | $-NH-CH_2-CH=CH-CH_3$ | 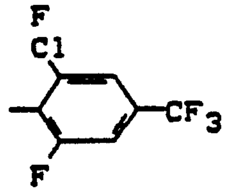 | 1 |
| $CH_3$ | $CCl_2F$ | $-NH-CH_2-C\equiv C-CH_3$ | 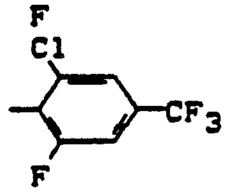 | 1 |

| $R^1$ | $R^2$ | $R^3$ | Ar | n |
|---|---|---|---|---|
| H | $CF_3$ | $-NH-C_2H_5$ | 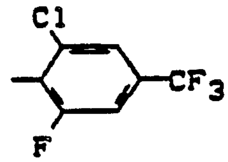 | 2 |
| H | $CF_3$ | $-NH-CH_3$ | 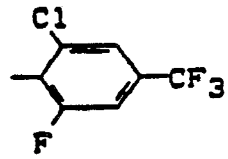 | 2 |
| H | $CF_3$ | $-N(CH_3)_2$ | 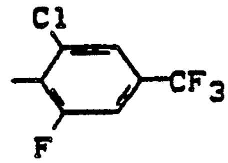 | 2 |
| H | $CF_3$ | $-N(C_2H_5)_2$ | 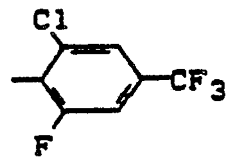 | 2 |
| H | $CF_3$ | 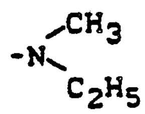 | 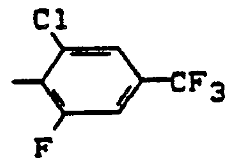 | 2 |
| H | $CF_3$ | $-NH-CH_2-CH=CH_2$ | 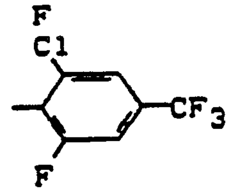 | 2 |
| H | $CF_3$ | $-N(CH_2-CH=CH_2)_2$ | 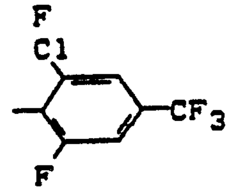 | 2 |
| H | $CF_3$ | $-N(CH_2-C\equiv CH)_2$ | 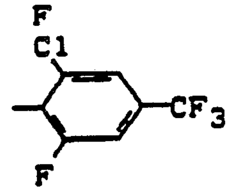 | 2 |

| $R^1$ | $R^2$ | $R^3$ | Ar | n |
|---|---|---|---|---|
| H | $CF_3$ | $-NH-CH_2-C≡CH$ | 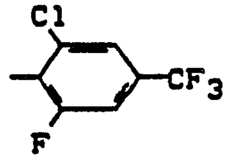 | 2 |
| H | $CF_3$ | 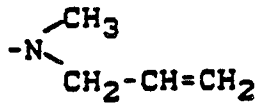 | 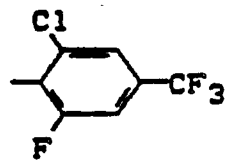 | 2 |
| H | $CF_3$ | 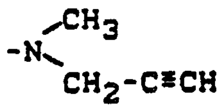 | 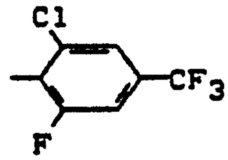 | 2 |
| H | $CF_3$ | $-NH-CH_2CH_2-CH_3$ | 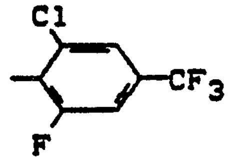 | 2 |
| H | $CF_3$ | $-NH-CH(CH_3)_2$ | 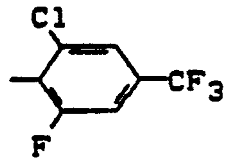 | 2 |
| H | $CF_3$ | 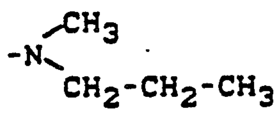 | 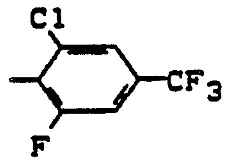 | 2 |
| H | $CF_3$ | $-N(CH_2-CH_2-CH_3)_2$ | 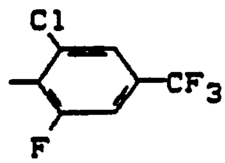 | 2 |
| H | $CF_3$ | $-NH-CH_2-CH=CH-CH_3$ | 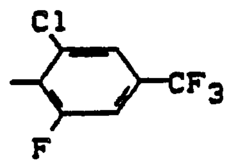 | 2 |
| H | $CF_3$ | $-NH-CH_2-C≡C-CH_3$ | 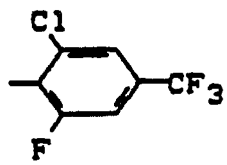 | 2 |

| $R^1$ | $R^2$ | $R^3$ | Ar | n |
|---|---|---|---|---|
| H | $CClF_2$ | $-NH-C_2H_5$ | 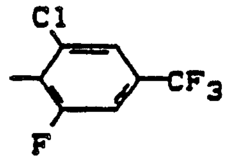 | 2 |
| H | $CClF_2$ | $-NH-CH_3$ | 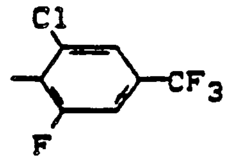 | 2 |
| H | $CClF_2$ | $-N(CH_3)_2$ | 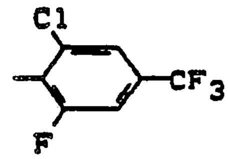 | 2 |
| H | $CClF_2$ | $-N(C_2H_5)_2$ | 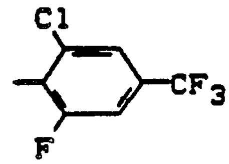 | 2 |
| H | $CClF_2$ | 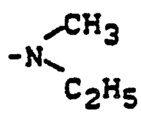 | 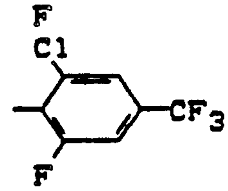 | 2 |
| H | $CClF_2$ | $-NH-CH_2-CH=CH_2$ | 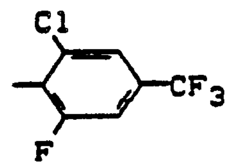 | 2 |
| H | $CClF_2$ | $-N(CH_2-CH=CH_2)_2$ | 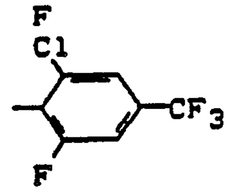 | 2 |
| H | $CClF_2$ | $-N(CH_2-C{\equiv}CH)_2$ | 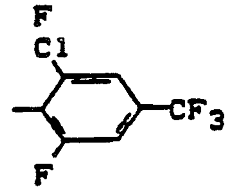 | 2 |

| $R^1$ | $R^2$ | $R^3$ | Ar | n |
|---|---|---|---|---|
| H | $CClF_2$ | $-NH-CH_2-C{\equiv}CH$ | 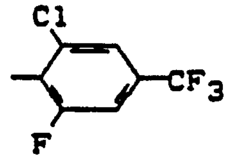 | 2 |
| H | $CClF_2$ | $CH_3$<br>$-N$<br>$CH_2-CH=CH_2$ | 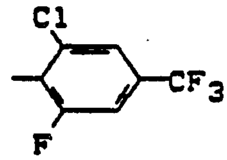 | 2 |
| H | $CClF_2$ | $CH_3$<br>$-N$<br>$CH_2-C{\equiv}CH$ | 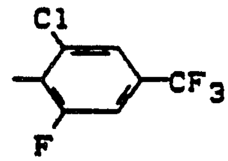 | 2 |
| H | $CClF_2$ | $-NH-CH_2CH_2-CH_3$ | 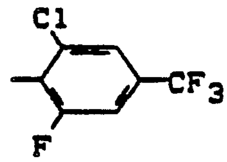 | 2 |
| H | $CClF_2$ | $-NH-CH(CH_3)_2$ | 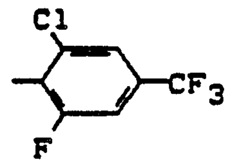 | 2 |
| H | $CClF_2$ | $CH_3$<br>$-N$<br>$CH_2-CH_2-CH_3$ | 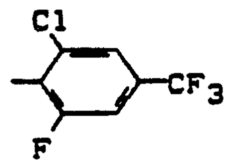 | 2 |
| H | $CClF_2$ | $-N(CH_2-CH_2-CH_3)_2$ | 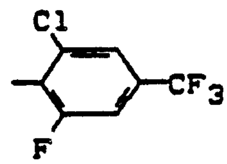 | 2 |
| H | $CClF_2$ | $-NH-CH_2-CH=CH-CH_3$ | 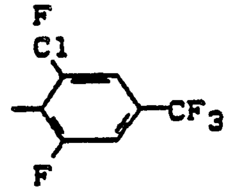 | 2 |
| H | $CClF_2$ | $-NH-CH_2-C{\equiv}C-CH_3$ | 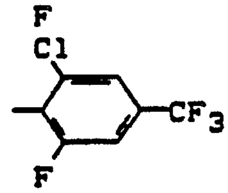 | 2 |

| $R^1$ | $R^2$ | $R^3$ | Ar | n |
|---|---|---|---|---|
| H | $CCl_2F$ | $-NH-C_2H_5$ | 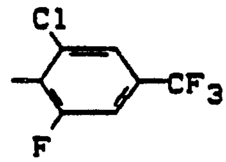 | 2 |
| H | $CCl_2F$ | $-NH-CH_3$ | 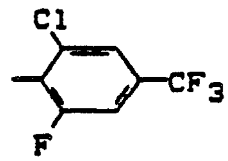 | 2 |
| H | $CCl_2F$ | $-N(CH_3)_2$ | 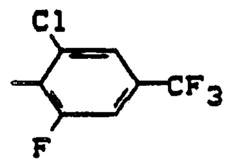 | 2 |
| H | $CCl_2F$ | $-N(C_2H_5)_2$ | 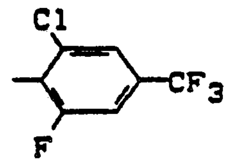 | 2 |
| H | $CCl_2F$ | 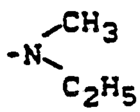 | 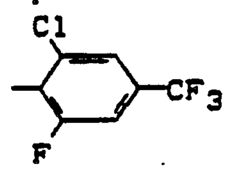 | 2 |
| H | $CCl_2F$ | $-NH-CH_2-CH=CH_2$ | 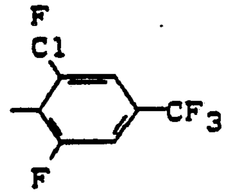 | 2 |
| H | $CCl_2F$ | $-N(CH_2-CH=CH_2)_2$ | 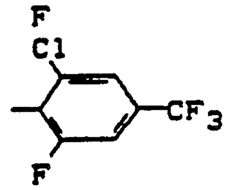 | 2 |
| H | $CCl_2F$ | $-N(CH_2-C\equiv CH)_2$ | 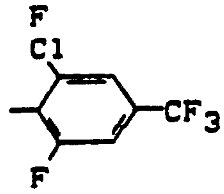 | 2 |

| $R^1$ | $R^2$ | $R^3$ | Ar | n |
|---|---|---|---|---|
| H | $CCl_2F$ | $-NH-CH_2-C\equiv CH$ | 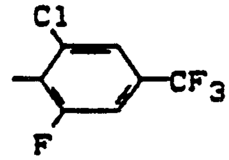 | 2 |
| H | $CCl_2F$ | 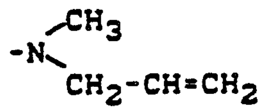 | 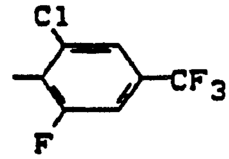 | 2 |
| H | $CCl_2F$ | 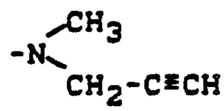 | 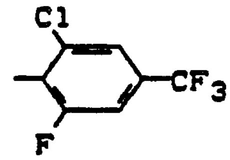 | 2 |
| H | $CCl_2F$ | $-NH-CH_2CH_2-CH_3$ | 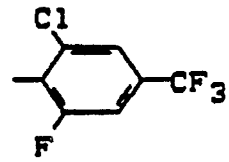 | 2 |
| H | $CCl_2F$ | $-NH-CH(CH_3)_2$ | 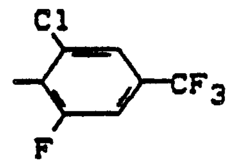 | 2 |
| H | $CCl_2F$ | 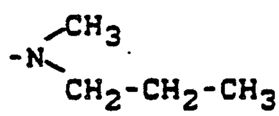 | 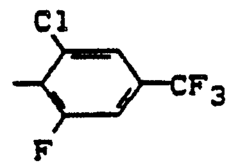 | 2 |
| H | $CCl_2F$ | $-N(CH_2-CH_2-CH_3)_2$ | 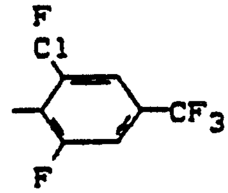 | 2 |
| H | $CCl_2F$ | $-NH-CH_2-CH=CH-CH_3$ | 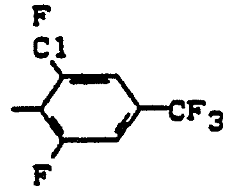 | 2 |
| H | $CCl_2F$ | $-NH-CH_2-C\equiv C-CH_3$ | 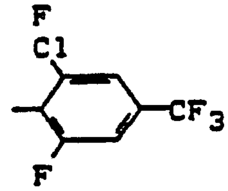 | 2 |

| $R^1$ | $R^2$ | $R^3$ | Ar | n |
|---|---|---|---|---|
| $CH_3$ | $CF_3$ | $-NH-C_2H_5$ | 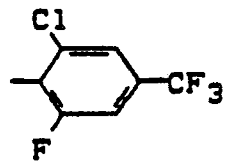 | 2 |
| $CH_3$ | $CF_3$ | $-NH-CH_3$ | 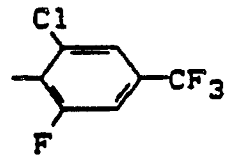 | 2 |
| $CH_3$ | $CF_3$ | $-N(CH_3)_2$ | 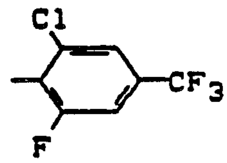 | 2 |
| $CH_3$ | $CF_3$ | $-N(C_2H_5)_2$ | 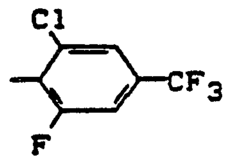 | 2 |
| $CH_3$ | $CF_3$ | 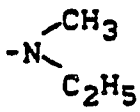 | 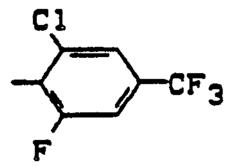 | 2 |
| $CH_3$ | $CF_3$ | $-NH-CH_2-CH=CH_2$ | 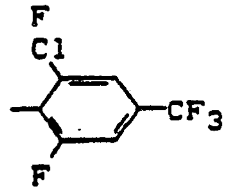 | 2 |
| $CH_3$ | $CF_3$ | $-N(CH_2-CH=CH_2)_2$ | 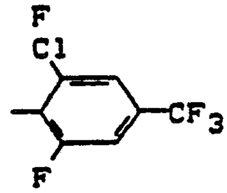 | 2 |
| $CH_3$ | $CF_3$ | $-N(CH_2-C\equiv CH)_2$ | 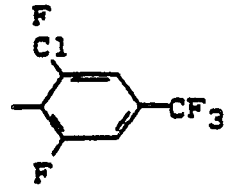 | 2 |

| $R^1$ | $R^2$ | $R^3$ | Ar | n |
|---|---|---|---|---|
| $CH_3$ | $CF_3$ | $-NH-CH_2-C{\equiv}CH$ | 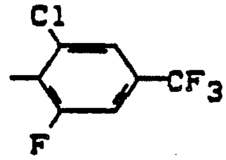 | 2 |
| $CH_3$ | $CF_3$ | 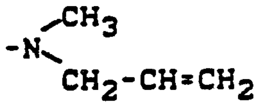 | 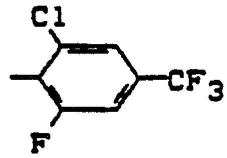 | 2 |
| $CH_3$ | $CF_3$ | 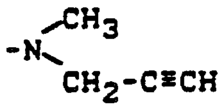 | 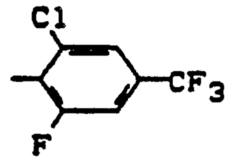 | 2 |
| $CH_3$ | $CF_3$ | $-NH-CH_2CH_2-CH_3$ | 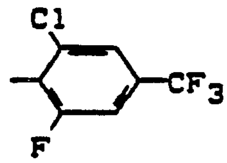 | 2 |
| $CH_3$ | $CF_3$ | $-NH-CH(CH_3)_2$ | 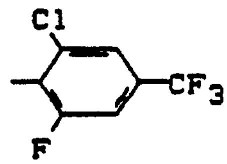 | 2 |
| $CH_3$ | $CF_3$ | 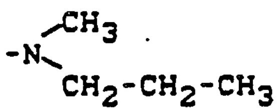 | 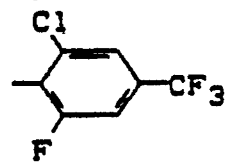 | 2 |
| $CH_3$ | $CF_3$ | $-N(CH_2-CH_2-CH_3)_2$ | 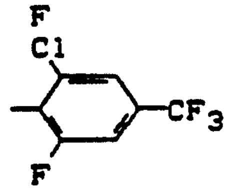 | 2 |
| $CH_3$ | $CF_3$ | $-NH-CH_2-CH{=}CH-CH_3$ | 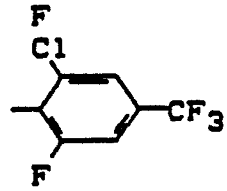 | 2 |
| $CH_3$ | $CF_3$ | $-NH-CH_2-C{\equiv}C-CH_3$ | 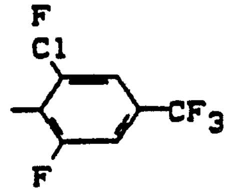 | 2 |

| $R^1$ | $R^2$ | $R^3$ | Ar | n |
|---|---|---|---|---|
| $CH_3$ | $CClF_2$ | $-NH-C_2H_5$ | 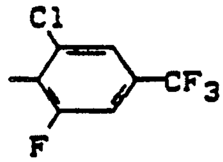 | 2 |
| $CH_3$ | $CClF_2$ | $-NH-CH_3$ | 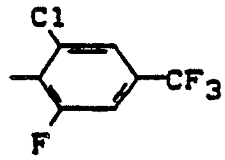 | 2 |
| $CH_3$ | $CClF_2$ | $-N(CH_3)_2$ | 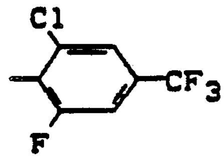 | 2 |
| $CH_3$ | $CClF_2$ | $-N(C_2H_5)_2$ | 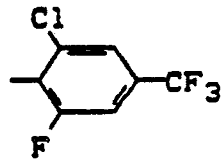 | 2 |
| $CH_3$ | $CClF_2$ | 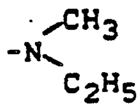 | 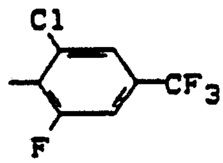 | 2 |
| $CH_3$ | $CClF_2$ | $-NH-CH_2-CH=CH_2$ | 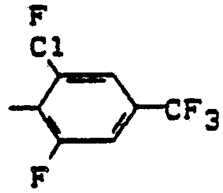 | 2 |
| $CH_3$ | $CClF_2$ | $-N(CH_2-CH=CH_2)_2$ | 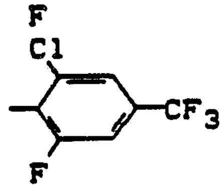 | 2 |
| $CH_3$ | $CClF_2$ | $-N(CH_2-C\equiv CH)_2$ | 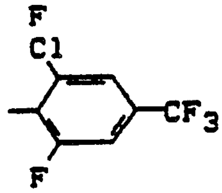 | 2 |

| $R^1$ | $R^2$ | $R^3$ | Ar | n |
|---|---|---|---|---|
| $CH_3$ | $CClF_2$ | $-NH-CH_2-C{\equiv}CH$ | 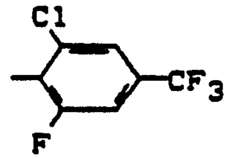 | 2 |
| $CH_3$ | $CClF_2$ | $-N\genfrac{}{}{0pt}{}{CH_3}{CH_2-CH=CH_2}$ | 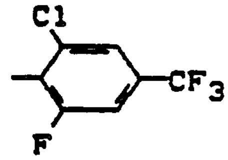 | 2 |
| $CH_3$ | $CClF_2$ | $-N\genfrac{}{}{0pt}{}{CH_3}{CH_2-C{\equiv}CH}$ | 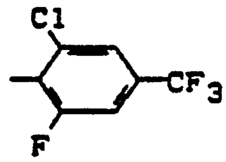 | 2 |
| $CH_3$ | $CClF_2$ | $-NH-CH_2CH_2-CH_3$ | 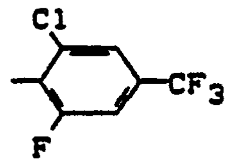 | 2 |
| $CH_3$ | $CClF_2$ | $-NH-CH(CH_3)_2$ | 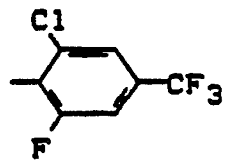 | 2 |
| $CH_3$ | $CClF_2$ | $-N\genfrac{}{}{0pt}{}{CH_3}{CH_2-CH_2-CH_3}$ | 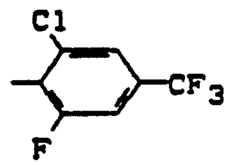 | 2 |
| $CH_3$ | $CClF_2$ | $-N(CH_2-CH_2-CH_3)_2$ | 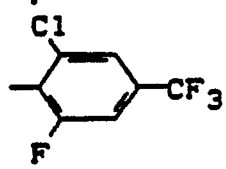 | 2 |
| $CH_3$ | $CClF_2$ | $-NH-CH_2-CH=CH-CH_3$ | 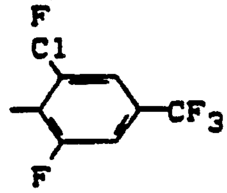 | 2 |
| $CH_3$ | $CClF_2$ | $-NH-CH_2-C{\equiv}C-CH_3$ | 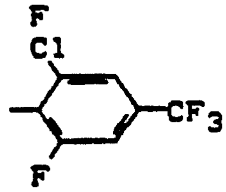 | 2 |

| $R^1$ | $R^2$ | $R^3$ | Ar | n |
|---|---|---|---|---|
| $CH_3$ | $CCl_2F$ | $-NH-C_2H_5$ | 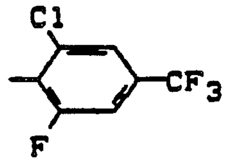 | 2 |
| $CH_3$ | $CCl_2F$ | $-NH-CH_3$ | 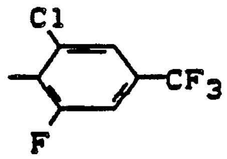 | 2 |
| $CH_3$ | $CCl_2F$ | $-N(CH_3)_2$ | 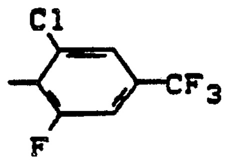 | 2 |
| $CH_3$ | $CCl_2F$ | $-N(C_2H_5)_2$ | 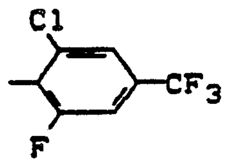 | 2 |
| $CH_3$ | $CCl_2F$ | 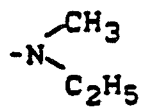 | 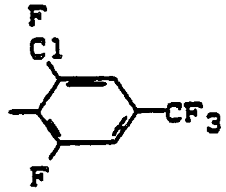 | 2 |
| $CH_3$ | $CCl_2F$ | $-NH-CH_2-CH=CH_2$ | 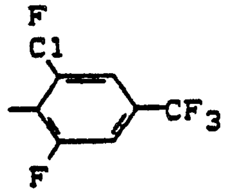 | 2 |
| $CH_3$ | $CCl_2F$ | $-N(CH_2-CH=CH_2)_2$ | 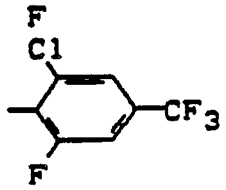 | 2 |
| $CH_3$ | $CCl_2F$ | $-N(CH_2-C\equiv CH)_2$ | 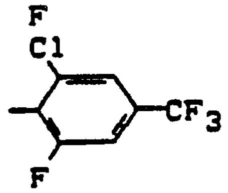 | 2 |

| $R^1$ | $R^2$ | $R^3$ | Ar | n |
|---|---|---|---|---|
| $CH_3$ | $CCl_2F$ | $-NH-CH_2-C\equiv CH$ | 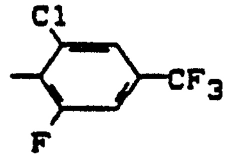 | 2 |
| $CH_3$ | $CCl_2F$ | $-N\genfrac{}{}{0pt}{}{CH_3}{CH_2-CH=CH_2}$ | 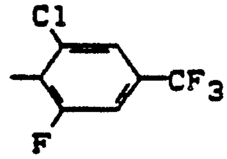 | 2 |
| $CH_3$ | $CCl_2F$ | $-N\genfrac{}{}{0pt}{}{CH_3}{CH_2-C\equiv CH}$ | 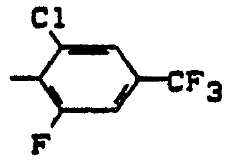 | 2 |
| $CH_3$ | $CCl_2F$ | $-NH-CH_2CH_2-CH_3$ | 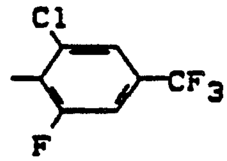 | 2 |
| $CH_3$ | $CCl_2F$ | $-NH-CH(CH_3)_2$ | 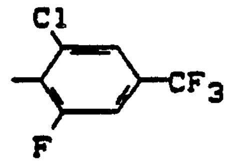 | 2 |
| $CH_3$ | $CCl_2F$ | $-N\genfrac{}{}{0pt}{}{CH_3}{CH_2-CH_2-CH_3}$ | 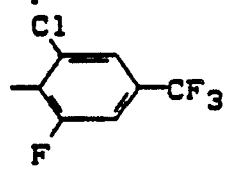 | 2 |
| $CH_3$ | $CCl_2F$ | $-N(CH_2-CH_2-CH_3)_2$ | 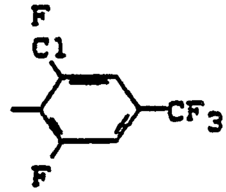 | 2 |
| $CH_3$ | $CCl_2F$ | $-NH-CH_2-CH=CH-CH_3$ | 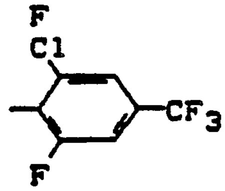 | 2 |
| $CH_3$ | $CCl_2F$ | $-NH-CH_2-C\equiv C-CH_3$ | 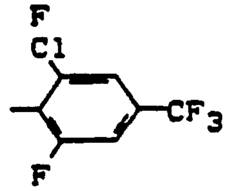 | 2 |

| $R^1$ | $R^2$ | $R^3$ | Ar | n |
|---|---|---|---|---|
| H | $CF_3$ | $-NH-C_2H_5$ | 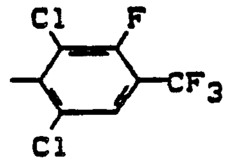 | 0 |
| H | $CF_3$ | $-NH-CH_3$ | 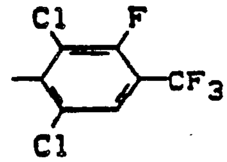 | 0 |
| H | $CF_3$ | $-N(CH_3)_2$ | 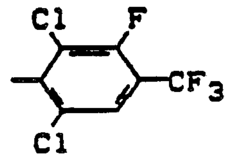 | 0 |
| H | $CF_3$ | $-N(C_2H_5)_2$ | 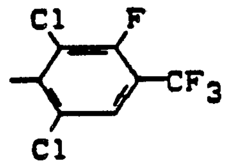 | 0 |
| H | $CF_3$ | 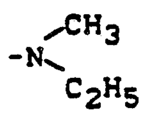 | 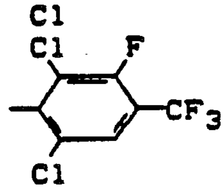 | 0 |
| H | $CF_3$ | $-NH-CH_2-CH=CH_2$ | 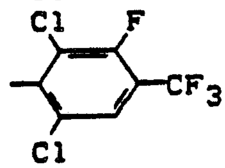 | 0 |
| H | $CF_3$ | $-N(CH_2-CH=CH_2)_2$ | 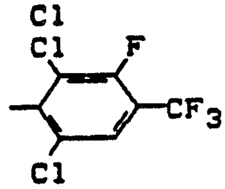 | 0 |
| H | $CF_3$ | $-N(CH_2-C\equiv CH)_2$ | 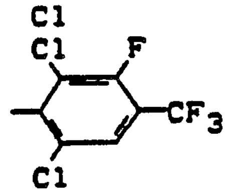 | 0 |

| $R^1$ | $R^2$ | $R^3$ | Ar | n |
|---|---|---|---|---|
| H | $CF_3$ | $-NH-CH_2-C{\equiv}CH$ | 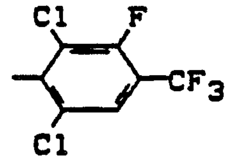 | 0 |
| H | $CF_3$ | 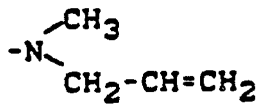 | 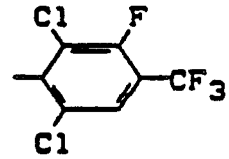 | 0 |
| H | $CF_3$ | 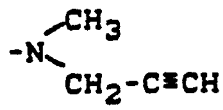 | 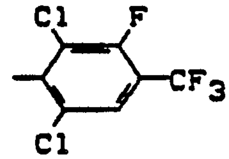 | 0 |
| H | $CF_3$ | $-NH-CH_2CH_2-CH_3$ | 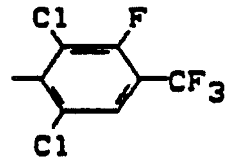 | 0 |
| H | $CF_3$ | $-NH-CH(CH_3)_2$ | 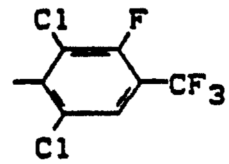 | 0 |
| H | $CF_3$ | 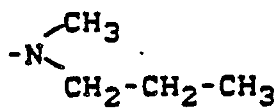 | 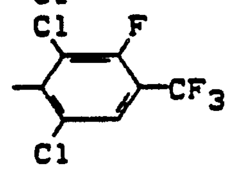 | 0 |
| H | $CF_3$ | $-N(CH_2-CH_2-CH_3)_2$ | 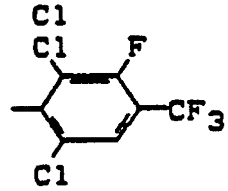 | 0 |
| H | $CF_3$ | $-NH-CH_2-CH{=}CH-CH_3$ | 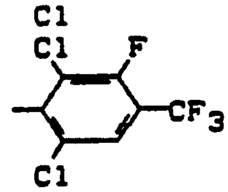 | 0 |
| H | $CF_3$ | $-NH-CH_2-C{\equiv}C-CH_3$ | 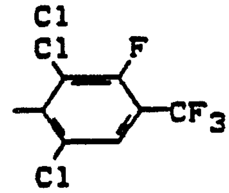 | 0 |

| $R^1$ | $R^2$ | $R^3$ | Ar | n |
|---|---|---|---|---|
| H | $CClF_2$ | $-NH-C_2H_5$ | 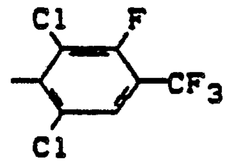 | 0 |
| H | $CClF_2$ | $-NH-CH_3$ | 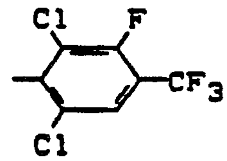 | 0 |
| H | $CClF_2$ | $-N(CH_3)_2$ | 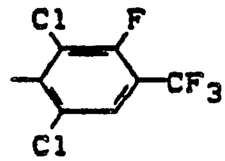 | 0 |
| H | $CClF_2$ | $-N(C_2H_5)_2$ | 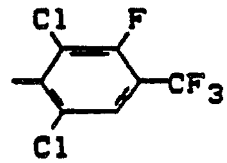 | 0 |
| H | $CClF_2$ | $-N(CH_3)(C_2H_5)$ | 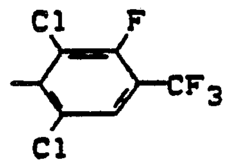 | 0 |
| H | $CClF_2$ | $-NH-CH_2-CH=CH_2$ | 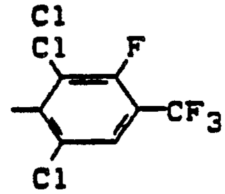 | 0 |
| H | $CClF_2$ | $-N(CH_2-CH=CH_2)_2$ | 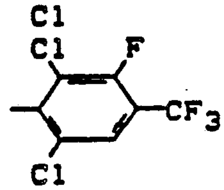 | 0 |
| H | $CClF_2$ | $-N(CH_2-C\equiv CH)_2$ | 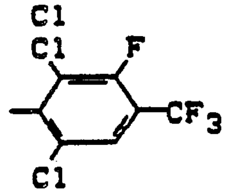 | 0 |

| $R^1$ | $R^2$ | $R^3$ | Ar | n |
|---|---|---|---|---|
| H | $CClF_2$ | $-NH-CH_2-C{\equiv}CH$ | 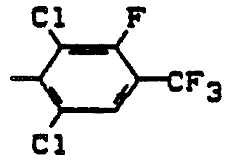 | 0 |
| H | $CClF_2$ | $-N(CH_3)CH_2-CH{=}CH_2$ | 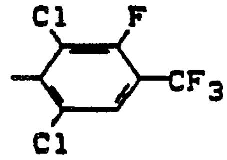 | 0 |
| H | $CClF_2$ | $-N(CH_3)CH_2-C{\equiv}CH$ | 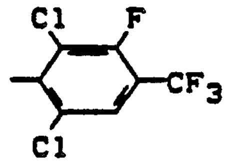 | 0 |
| H | $CClF_2$ | $-NH-CH_2CH_2-CH_3$ | 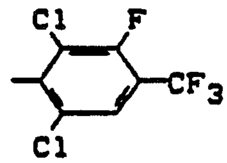 | 0 |
| H | $CClF_2$ | $-NH-CH(CH_3)_2$ | 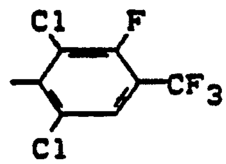 | 0 |
| H | $CClF_2$ | $-N(CH_3)CH_2-CH_2-CH_3$ | 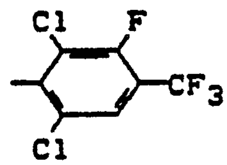 | 0 |
| H | $CClF_2$ | $-N(CH_2-CH_2-CH_3)_2$ | 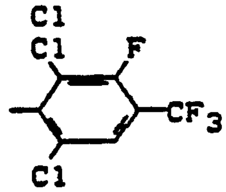 | 0 |
| H | $CClF_2$ | $-NH-CH_2-CH{=}CH-CH_3$ | 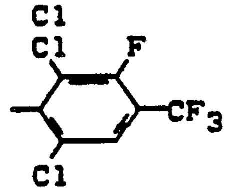 | 0 |
| H | $CClF_2$ | $-NH-CH_2-C{\equiv}C-CH_3$ | 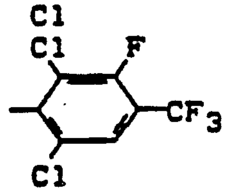 | 0 |

| $R^1$ | $R^2$ | $R^3$ | Ar | n |
|---|---|---|---|---|
| H | $CCl_2F$ | $-NH-C_2H_5$ | 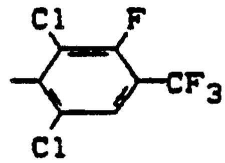 | 0 |
| H | $CCl_2F$ | $-NH-CH_3$ | 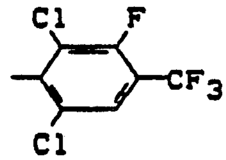 | 0 |
| H | $CCl_2F$ | $-N(CH_3)_2$ | 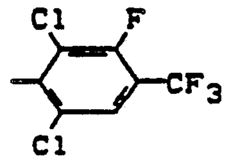 | 0 |
| H | $CCl_2F$ | $-N(C_2H_5)_2$ | 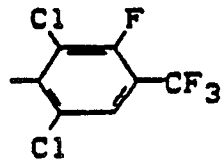 | 0 |
| H | $CCl_2F$ | $-N(CH_3)(C_2H_5)$ | 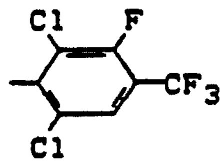 | 0 |
| H | $CCl_2F$ | $-NH-CH_2-CH{=}CH_2$ | 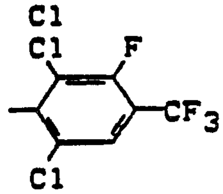 | 0 |
| H | $CCl_2F$ | $-N(CH_2-CH{=}CH_2)_2$ | 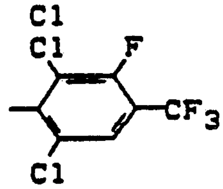 | 0 |
| H | $CCl_2F$ | $-N(CH_2-C{\equiv}CH)_2$ | 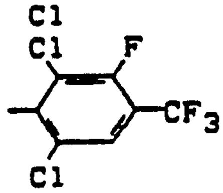 | 0 |

| $R^1$ | $R^2$ | $R^3$ | Ar | n |
|---|---|---|---|---|
| H | $CCl_2F$ | $-NH-CH_2-C{\equiv}CH$ | 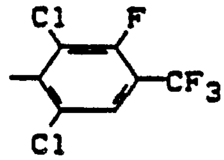 | 0 |
| H | $CCl_2F$ | $-N(CH_3)CH_2-CH{=}CH_2$ | 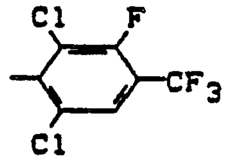 | 0 |
| H | $CCl_2F$ | $-N(CH_3)CH_2-C{\equiv}CH$ | 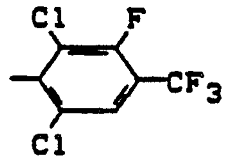 | 0 |
| H | $CCl_2F$ | $-NH-CH_2CH_2-CH_3$ | 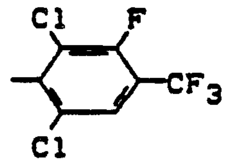 | 0 |
| H | $CCl_2F$ | $-NH-CH(CH_3)_2$ | 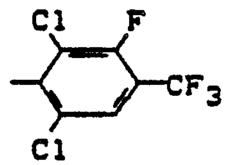 | 0 |
| H | $CCl_2F$ | $-N(CH_3)CH_2-CH_2-CH_3$ | 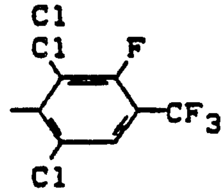 | 0 |
| H | $CCl_2F$ | $-N(CH_2-CH_2-CH_3)_2$ | 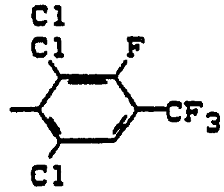 | 0 |
| H | $CCl_2F$ | $-NH-CH_2-CH{=}CH-CH_3$ | 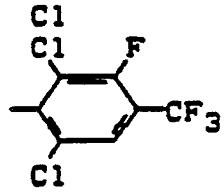 | 0 |
| H | $CCl_2F$ | $-NH-CH_2-C{\equiv}C-CH_3$ | 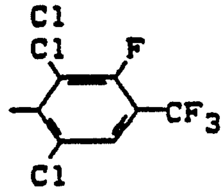 | 0 |

| $R^1$ | $R^2$ | $R^3$ | Ar | n |
|---|---|---|---|---|
| $CH_3$ | $CF_3$ | $-NH-C_2H_5$ | 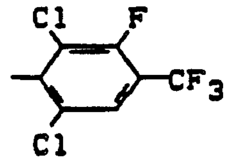 | 0 |
| $CH_3$ | $CF_3$ | $-NH-CH_3$ | 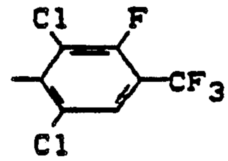 | 0 |
| $CH_3$ | $CF_3$ | $-N(CH_3)_2$ | 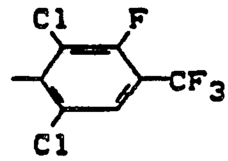 | 0 |
| $CH_3$ | $CF_3$ | $-N(C_2H_5)_2$ | 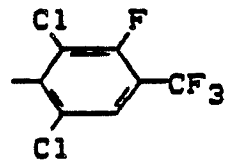 | 0 |
| $CH_3$ | $CF_3$ | 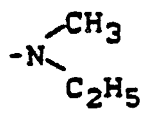 | 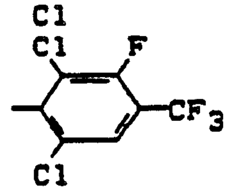 | 0 |
| $CH_3$ | $CF_3$ | $-NH-CH_2-CH=CH_2$ | 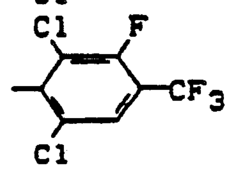 | 0 |
| $CH_3$ | $CF_3$ | $-N(CH_2-CH=CH_2)_2$ | 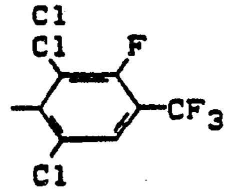 | 0 |
| $CH_3$ | $CF_3$ | $-N(CH_2-C\equiv CH)_2$ | 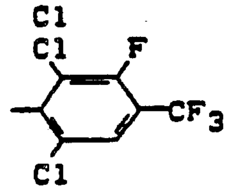 | 0 |

| $R^1$ | $R^2$ | $R^3$ | Ar | n |
|---|---|---|---|---|
| $CH_3$ | $CF_3$ | $-NH-CH_2-C{\equiv}CH$ | 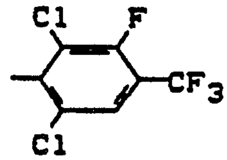 | 0 |
| $CH_3$ | $CF_3$ | $-N(CH_3)(CH_2-CH{=}CH_2)$ | 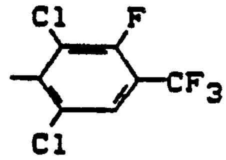 | 0 |
| $CH_3$ | $CF_3$ | $-N(CH_3)(CH_2-C{\equiv}CH)$ | 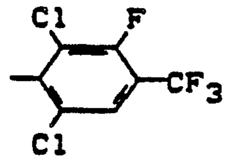 | 0 |
| $CH_3$ | $CF_3$ | $-NH-CH_2CH_2-CH_3$ | 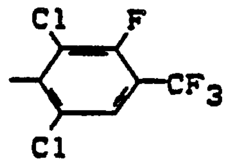 | 0 |
| $CH_3$ | $CF_3$ | $-NH-CH(CH_3)_2$ | 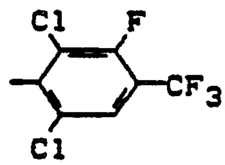 | 0 |
| $CH_3$ | $CF_3$ | $-N(CH_3)(CH_2-CH_2-CH_3)$ | 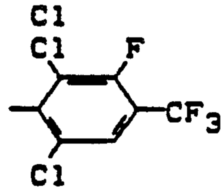 | 0 |
| $CH_3$ | $CF_3$ | $-N(CH_2-CH_2-CH_3)_2$ | 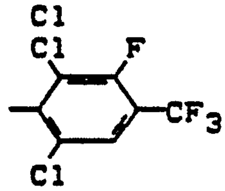 | 0 |
| $CH_3$ | $CF_3$ | $-NH-CH_2-CH{=}CH-CH_3$ | 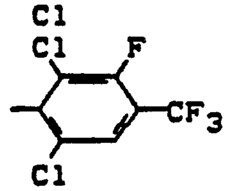 | 0 |
| $CH_3$ | $CF_3$ | $-NH-CH_2-C{\equiv}C-CH_3$ | 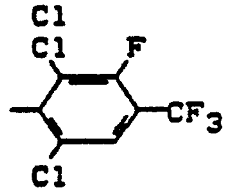 | 0 |

| $R^1$ | $R^2$ | $R^3$ | Ar | n |
|---|---|---|---|---|
| $CH_3$ | $CClF_2$ | $-NH-C_2H_5$ | 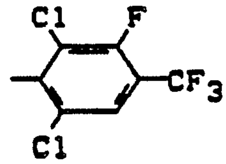 | 0 |
| $CH_3$ | $CClF_2$ | $-NH-CH_3$ | 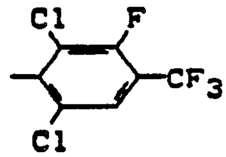 | 0 |
| $CH_3$ | $CClF_2$ | $-N(CH_3)_2$ | 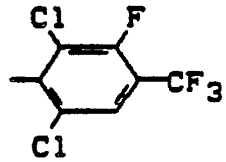 | 0 |
| $CH_3$ | $CClF_2$ | $-N(C_2H_5)_2$ | 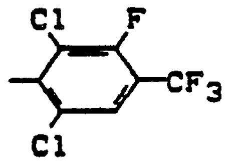 | 0 |
| $CH_3$ | $CClF_2$ | 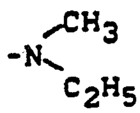 | 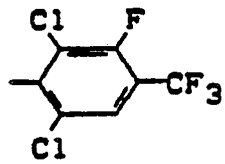 | 0 |
| $CH_3$ | $CClF_2$ | $-NH-CH_2-CH=CH_2$ | 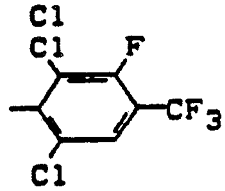 | 0 |
| $CH_3$ | $CClF_2$ | $-N(CH_2-CH=CH_2)_2$ | 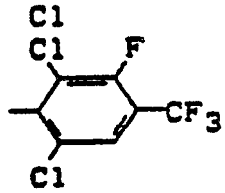 | 0 |
| $CH_3$ | $CClF_2$ | $-N(CH_2-C\equiv CH)_2$ | 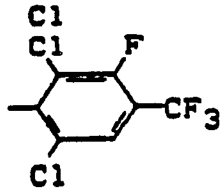 | 0 |

| $R^1$ | $R^2$ | $R^3$ | Ar | n |
|---|---|---|---|---|
| $CH_3$ | $CClF_2$ | $-NH-CH_2-C \equiv CH$ | 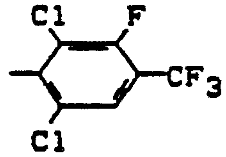 | 0 |
| $CH_3$ | $CClF_2$ | $CH_3$<br>$-N$<br>$CH_2-CH=CH_2$ | 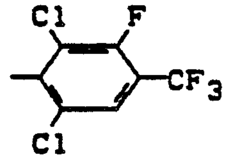 | 0 |
| $CH_3$ | $CClF_2$ | $CH_3$<br>$-N$<br>$CH_2-C \equiv CH$ | 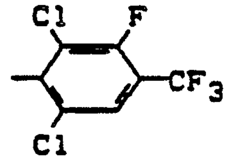 | 0 |
| $CH_3$ | $CClF_2$ | $-NH-CH_2CH_2-CH_3$ | 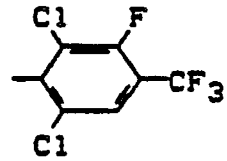 | 0 |
| $CH_3$ | $CClF_2$ | $-NH-CH(CH_3)_2$ | 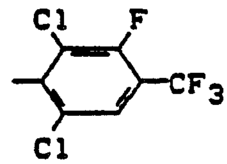 | 0 |
| $CH_3$ | $CClF_2$ | $CH_3$<br>$-N$<br>$CH_2-CH_2-CH_3$ | 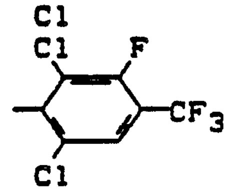 | 0 |
| $CH_3$ | $CClF_2$ | $-N(CH_2-CH_2-CH_3)_2$ | 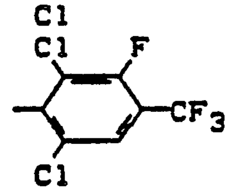 | 0 |
| $CH_3$ | $CClF_2$ | $-NH-CH_2-CH=CH-CH_3$ | 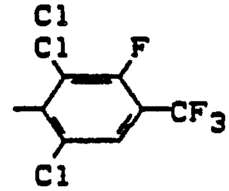 | 0 |
| $CH_3$ | $CClF_2$ | $-NH-CH_2-C \equiv C-CH_3$ | 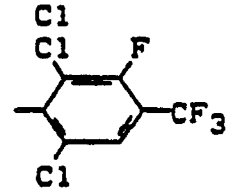 | 0 |

| $R^1$ | $R^2$ | $R^3$ | Ar | n |
|---|---|---|---|---|
| $CH_3$ | $CCl_2F$ | $-NH-C_2H_5$ | 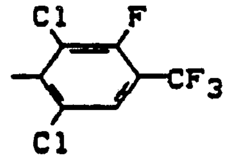 | 0 |
| $CH_3$ | $CCl_2F$ | $-NH-CH_3$ | 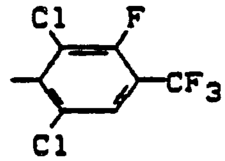 | 0 |
| $CH_3$ | $CCl_2F$ | $-N(CH_3)_2$ | 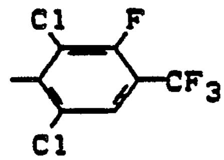 | 0 |
| $CH_3$ | $CCl_2F$ | $-N(C_2H_5)_2$ | 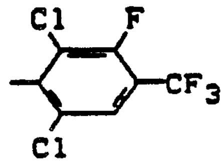 | 0 |
| $CH_3$ | $CCl_2F$ | 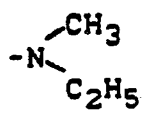 | 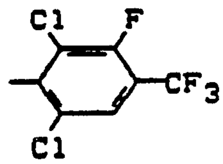 | 0 |
| $CH_3$ | $CCl_2F$ | $-NH-CH_2-CH=CH_2$ | 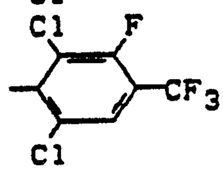 | 0 |
| $CH_3$ | $CCl_2F$ | $-N(CH_2-CH=CH_2)_2$ | 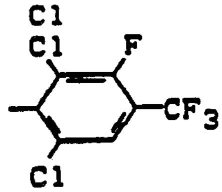 | 0 |
| $CH_3$ | $CCl_2F$ | $-N(CH_2-C\equiv CH)_2$ | 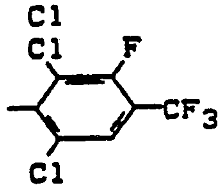 | 0 |

| $R^1$ | $R^2$ | $R^3$ | Ar | n |
|---|---|---|---|---|
| $CH_3$ | $CCl_2F$ | $-NH-CH_2-C{\equiv}CH$ | 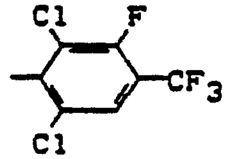 | 0 |
| $CH_3$ | $CCl_2F$ | $-N(CH_3)(CH_2-CH{=}CH_2)$ | 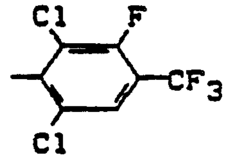 | 0 |
| $CH_3$ | $CCl_2F$ | $-N(CH_3)(CH_2-C{\equiv}CH)$ | 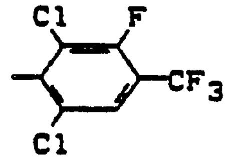 | 0 |
| $CH_3$ | $CCl_2F$ | $-NH-CH_2CH_2-CH_3$ | 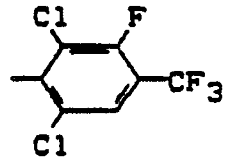 | 0 |
| $CH_3$ | $CCl_2F$ | $-NH-CH(CH_3)_2$ | 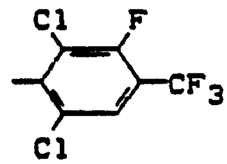 | 0 |
| $CH_3$ | $CCl_2F$ | $-N(CH_3)(CH_2-CH_2-CH_3)$ | 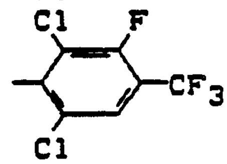 | 0 |
| $CH_3$ | $CCl_2F$ | $-N(CH_2-CH_2-CH_3)_2$ | 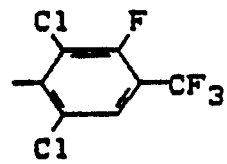 | 0 |
| $CH_3$ | $CCl_2F$ | $-NH-CH_2-CH{=}CH-CH_3$ | 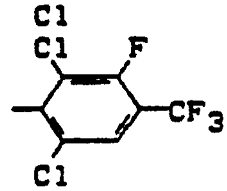 | 0 |
| $CH_3$ | $CCl_2F$ | $-NH-CH_2-C{\equiv}C-CH_3$ | 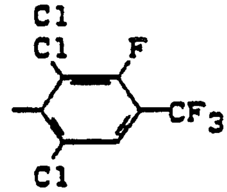 | 0 |

| $R^1$ | $R^2$ | $R^3$ | Ar | n |
|---|---|---|---|---|
| H | $CF_3$ | $-NH-C_2H_5$ | 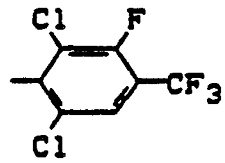 | 2 |
| H | $CF_3$ | $-NH-CH_3$ | 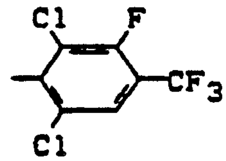 | 2 |
| H | $CF_3$ | $-N(CH_3)_2$ | 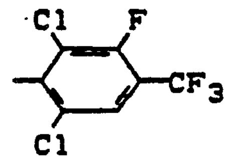 | 2 |
| H | $CF_3$ | $-N(C_2H_5)_2$ | 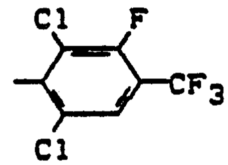 | 2 |
| H | $CF_3$ | 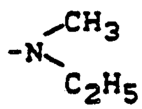 | 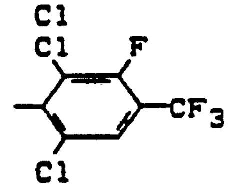 | 2 |
| H | $CF_3$ | $-NH-CH_2-CH=CH_2$ | 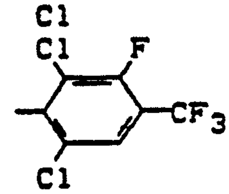 | 2 |
| H | $CF_3$ | $-N(CH_2-CH=CH_2)_2$ | 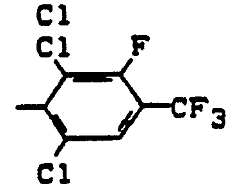 | 2 |
| H | $CF_3$ | $-N(CH_2-C{\equiv}CH)_2$ | 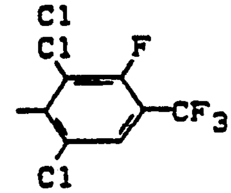 | 2 |

| $R^1$ | $R^2$ | $R^3$ | Ar | n |
|---|---|---|---|---|
| H | $CF_3$ | $-NH-CH_2-C\equiv CH$ | 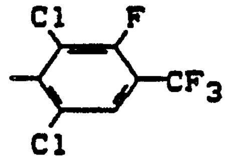 | 2 |
| H | $CF_3$ | $CH_3$<br>$-N$<br>$CH_2-CH=CH_2$ | 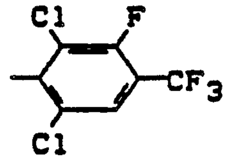 | 2 |
| H | $CF_3$ | $CH_3$<br>$-N$<br>$CH_2-C\equiv CH$ | 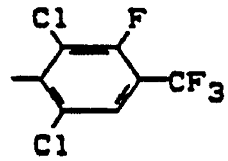 | 2 |
| H | $CF_3$ | $-NH-CH_2CH_2-CH_3$ | 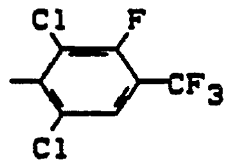 | 2 |
| H | $CF_3$ | $-NH-CH(CH_3)_2$ | 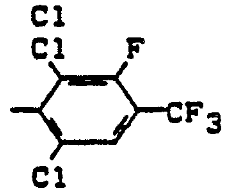 | 2 |
| H | $CF_3$ | $CH_3$<br>$-N$<br>$CH_2-CH_2-CH_3$ | 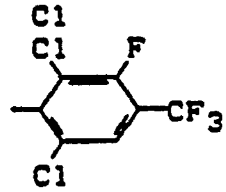 | 2 |
| H | $CF_3$ | $-N(CH_2-CH_2-CH_3)_2$ | 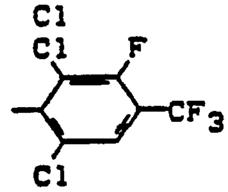 | 2 |
| H | $CF_3$ | $-NH-CH_2-CH=CH-CH_3$ | 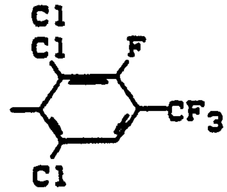 | 2 |
| H | $CF_3$ | $-NH-CH_2-C\equiv C-CH_3$ | 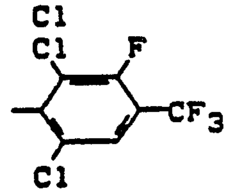 | 2 |

| $R^1$ | $R^2$ | $R^3$ | Ar | n |
|---|---|---|---|---|
| H | $CClF_2$ | $-NH-C_2H_5$ | 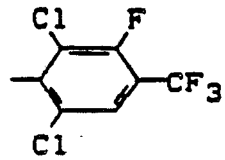 | 2 |
| H | $CClF_2$ | $-NH-CH_3$ | 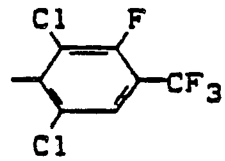 | 2 |
| H | $CClF_2$ | $-N(CH_3)_2$ | 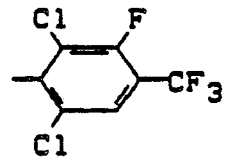 | 2 |
| H | $CClF_2$ | $-N(C_2H_5)_2$ | 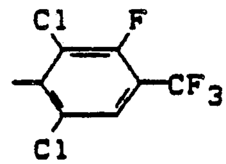 | 2 |
| H | $CClF_2$ | 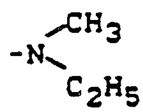 | 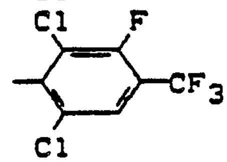 | 2 |
| H | $CClF_2$ | $-NH-CH_2-CH=CH_2$ | 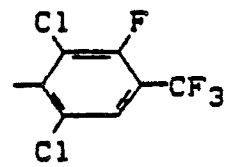 | 2 |
| H | $CClF_2$ | $-N(CH_2-CH=CH_2)_2$ | 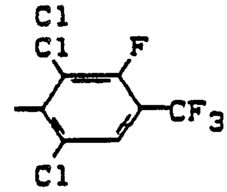 | 2 |
| H | $CClF_2$ | $-N(CH_2-C\equiv CH)_2$ | 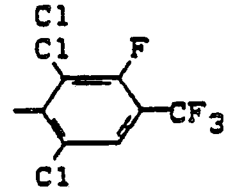 | 2 |

| $R^1$ | $R^2$ | $R^3$ | Ar | n |
|---|---|---|---|---|
| H | $CClF_2$ | $-NH-CH_2-C{\equiv}CH$ | 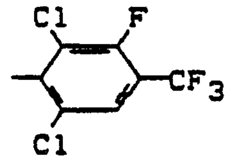 | 2 |
| H | $CClF_2$ | $-N(CH_3)CH_2-CH{=}CH_2$ | 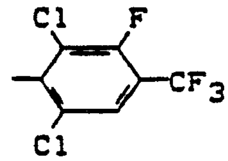 | 2 |
| H | $CClF_2$ | $-N(CH_3)CH_2-C{\equiv}CH$ | 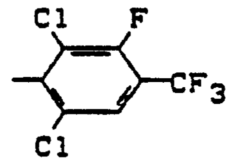 | 2 |
| H | $CClF_2$ | $-NH-CH_2CH_2-CH_3$ | 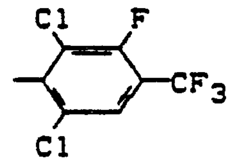 | 2 |
| H | $CClF_2$ | $-NH-CH(CH_3)_2$ | 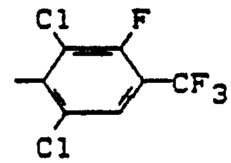 | 2 |
| H | $CClF_2$ | $-N(CH_3)CH_2-CH_2-CH_3$ | 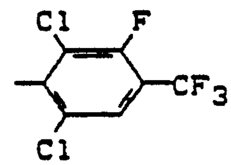 | 2 |
| H | $CClF_2$ | $-N(CH_2-CH_2-CH_3)_2$ | 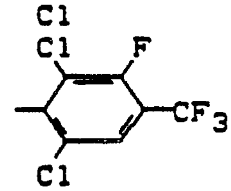 | 2 |
| H | $CClF_2$ | $-NH-CH_2-CH{=}CH-CH_3$ | 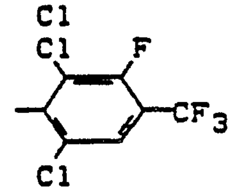 | 2 |
| H | $CClF_2$ | $-NH-CH_2-C{\equiv}C-CH_3$ | 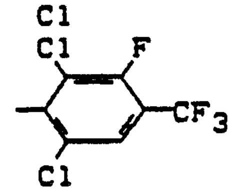 | 2 |

| $R^1$ | $R^2$ | $R^3$ | Ar | n |
|---|---|---|---|---|
| H | $CCl_2F$ | $-NH-C_2H_5$ | 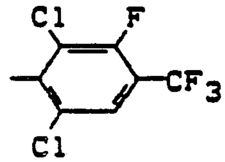 | 2 |
| H | $CCl_2F$ | $-NH-CH_3$ | 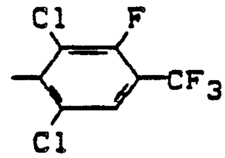 | 2 |
| H | $CCl_2F$ | $-N(CH_3)_2$ | 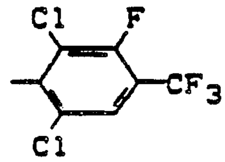 | 2 |
| H | $CCl_2F$ | $-N(C_2H_5)_2$ | 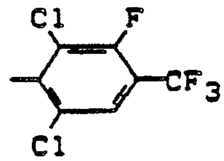 | 2 |
| H | $CCl_2F$ | 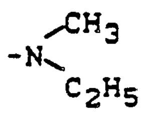 | 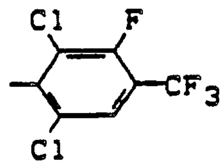 | 2 |
| H | $CCl_2F$ | $-NH-CH_2-CH=CH_2$ | 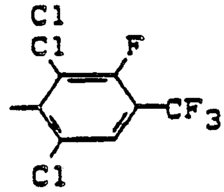 | 2 |
| H | $CCl_2F$ | $-N(CH_2-CH=CH_2)_2$ | 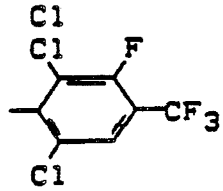 | 2 |
| H | $CCl_2F$ | $-N(CH_2-C\equiv CH)_2$ | 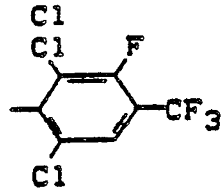 | 2 |

| $R^1$ | $R^2$ | $R^3$ | Ar | n |
|---|---|---|---|---|
| H | $CCl_2F$ | $-NH-CH_2-C\equiv CH$ | 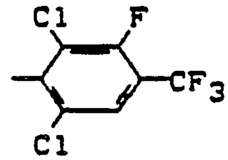 | 2 |
| H | $CCl_2F$ | $-N(CH_3)(CH_2-CH=CH_2)$ | 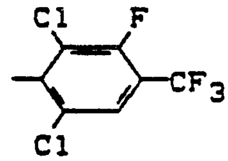 | 2 |
| H | $CCl_2F$ | $-N(CH_3)(CH_2-C\equiv CH)$ | 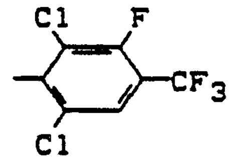 | 2 |
| H | $CCl_2F$ | $-NH-CH_2CH_2-CH_3$ | 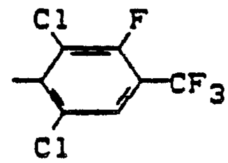 | 2 |
| H | $CCl_2F$ | $-NH-CH(CH_3)_2$ | 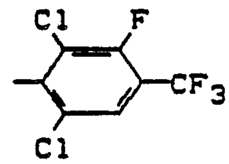 | 2 |
| H | $CCl_2F$ | $-N(CH_3)(CH_2-CH_2-CH_3)$ | 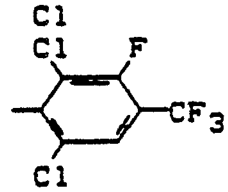 | 2 |
| H | $CCl_2F$ | $-N(CH_2-CH_2-CH_3)_2$ | 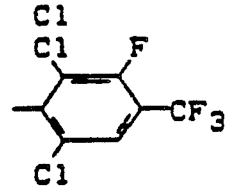 | 2 |
| H | $CCl_2F$ | $-NH-CH_2-CH=CH-CH_3$ | 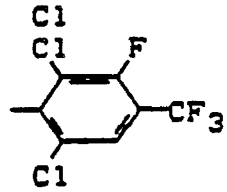 | 2 |
| H | $CCl_2F$ | $-NH-CH_2-C\equiv C-CH_3$ | 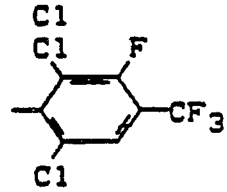 | 2 |

| $R^1$ | $R^2$ | $R^3$ | Ar | n |
|---|---|---|---|---|
| $CH_3$ | $CF_3$ | $-NH-C_2H_5$ | 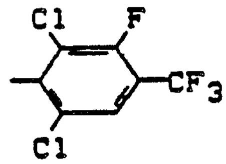 | 2 |
| $CH_3$ | $CF_3$ | $-NH-CH_3$ | 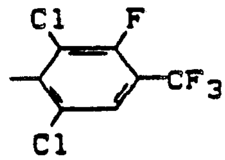 | 2 |
| $CH_3$ | $CF_3$ | $-N(CH_3)_2$ | 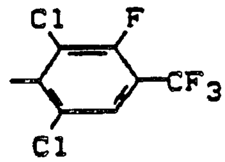 | 2 |
| $CH_3$ | $CF_3$ | $-N(C_2H_5)_2$ | 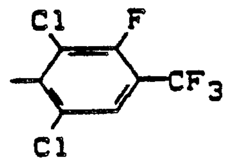 | 2 |
| $CH_3$ | $CF_3$ | $-N(CH_3)(C_2H_5)$ | 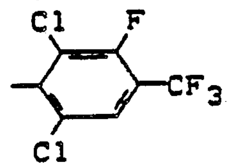 | 2 |
| $CH_3$ | $CF_3$ | $-NH-CH_2-CH=CH_2$ | 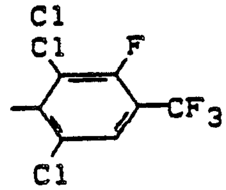 | 2 |
| $CH_3$ | $CF_3$ | $-N(CH_2-CH=CH_2)_2$ | 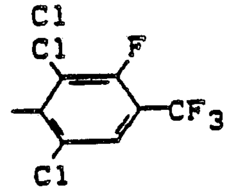 | 2 |
| $CH_3$ | $CF_3$ | $-N(CH_2-C \equiv CH)_2$ | 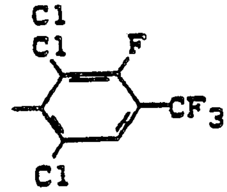 | 2 |

| $R^1$ | $R^2$ | $R^3$ | Ar | n |
|---|---|---|---|---|
| $CH_3$ | $CF_3$ | $-NH-CH_2-C\equiv CH$ | 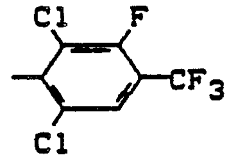 | 2 |
| $CH_3$ | $CF_3$ | $CH_3$<br>$-N$<br>$CH_2-CH=CH_2$ | 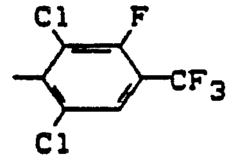 | 2 |
| $CH_3$ | $CF_3$ | $CH_3$<br>$-N$<br>$CH_2-C\equiv CH$ | 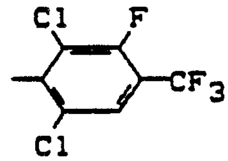 | 2 |
| $CH_3$ | $CF_3$ | $-NH-CH_2CH_2-CH_3$ | 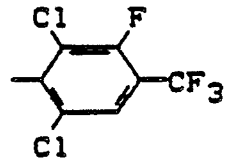 | 2 |
| $CH_3$ | $CF_3$ | $-NH-CH(CH_3)_2$ | 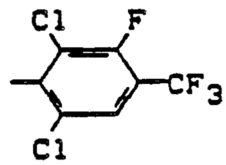 | 2 |
| $CH_3$ | $CF_3$ | $CH_3$<br>$-N$<br>$CH_2-CH_2-CH_3$ | 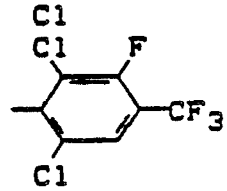 | 2 |
| $CH_3$ | $CF_3$ | $-N(CH_2-CH_2-CH_3)_2$ | 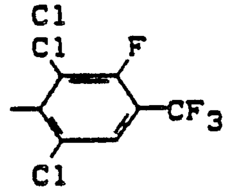 | 2 |
| $CH_3$ | $CF_3$ | $-NH-CH_2-CH=CH-CH_3$ | 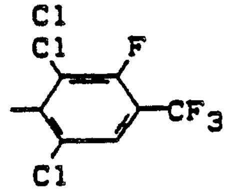 | 2 |
| $CH_3$ | $CF_3$ | $-NH-CH_2-C\equiv C-CH_3$ | 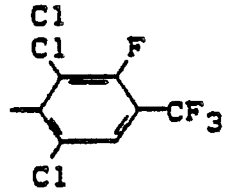 | 2 |

| $R^1$ | $R^2$ | $R^3$ | Ar | n |
|---|---|---|---|---|
| $CH_3$ | $CClF_2$ | $-NH-C_2H_5$ | 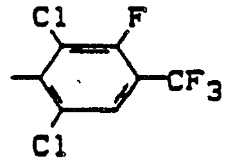 | 2 |
| $CH_3$ | $CClF_2$ | $-NH-CH_3$ | 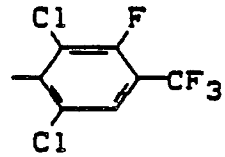 | 2 |
| $CH_3$ | $CClF_2$ | $-N(CH_3)_2$ | 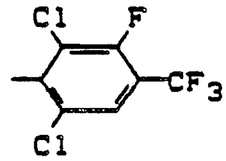 | 2 |
| $CH_3$ | $CClF_2$ | $-N(C_2H_5)_2$ | 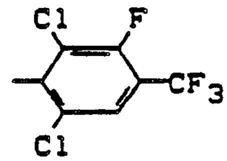 | 2 |
| $CH_3$ | $CClF_2$ | 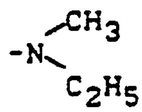 | 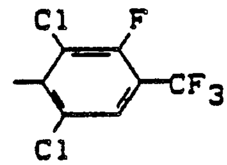 | 2 |
| $CH_3$ | $CClF_2$ | $-NH-CH_2-CH=CH_2$ | 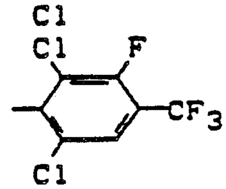 | 2 |
| $CH_3$ | $CClF_2$ | $-N(CH_2-CH=CH_2)_2$ | 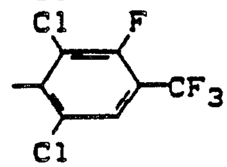 | 2 |
| $CH_3$ | $CClF_2$ | $-N(CH_2-C≡CH)_2$ | 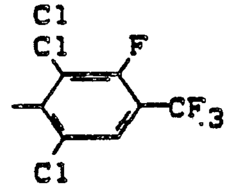 | 2 |

| $R^1$ | $R^2$ | $R^3$ | Ar | n |
|---|---|---|---|---|
| $CH_3$ | $CClF_2$ | $-NH-CH_2-C\equiv CH$ | 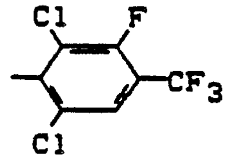 | 2 |
| $CH_3$ | $CClF_2$ | 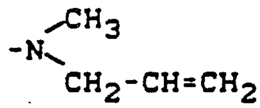 | 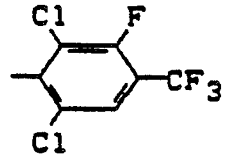 | 2 |
| $CH_3$ | $CClF_2$ | 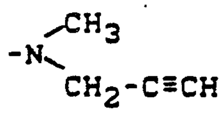 | 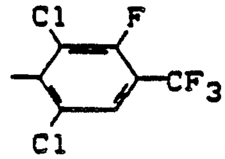 | 2 |
| $CH_3$ | $CClF_2$ | $-NH-CH_2CH_2-CH_3$ | 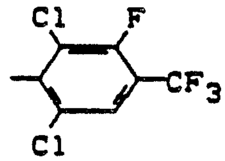 | 2 |
| $CH_3$ | $CClF_2$ | $-NH-CH(CH_3)_2$ | 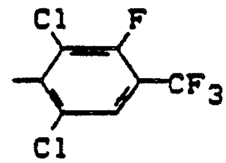 | 2 |
| $CH_3$ | $CClF_2$ | 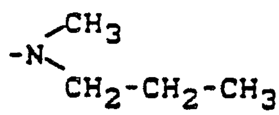 | 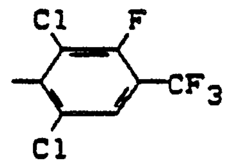 | 2 |
| $CH_3$ | $CClF_2$ | $-N(CH_2-CH_2-CH_3)_2$ | 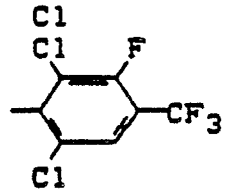 | 2 |
| $CH_3$ | $CClF_2$ | $-NH-CH_2-CH=CH-CH_3$ | 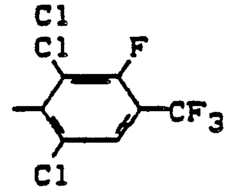 | 2 |
| $CH_3$ | $CClF_2$ | $-NH-CH_2-C\equiv C-CH_3$ | 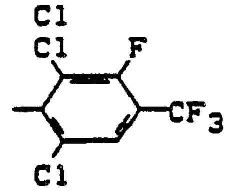 | 2 |

| $R^1$ | $R^2$ | $R^3$ | Ar | n |
|---|---|---|---|---|
| $CH_3$ | $CCl_2F$ | $-NH-C_2H_5$ | 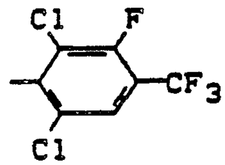 | 2 |
| $CH_3$ | $CCl_2F$ | $-NH-CH_3$ | 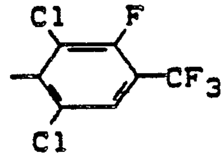 | 2 |
| $CH_3$ | $CCl_2F$ | $-N(CH_3)_2$ | 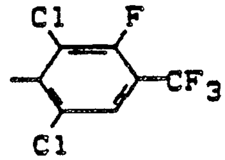 | 2 |
| $CH_3$ | $CCl_2F$ | $-N(C_2H_5)_2$ | 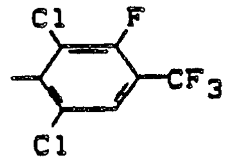 | 2 |
| $CH_3$ | $CCl_2F$ | $-N(CH_3)(C_2H_5)$ | 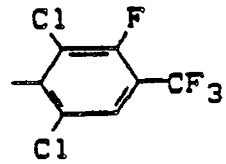 | 2 |
| $CH_3$ | $CCl_2F$ | $-NH-CH_2-CH=CH_2$ | 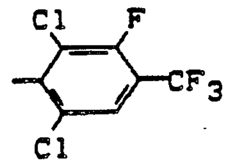 | 2 |
| $CH_3$ | $CCl_2F$ | $-N(CH_2-CH=CH_2)_2$ | 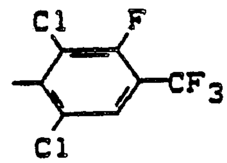 | 2 |
| $CH_3$ | $CCl_2F$ | $-N(CH_2-C\equiv CH)_2$ | 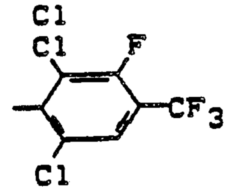 | 2 |

| $R^1$ | $R^2$ | $R^3$ | Ar | n |
|---|---|---|---|---|
| $CH_3$ | $CCl_2F$ | $-NH-CH_2-C\equiv CH$ | 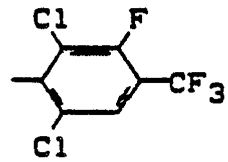 | 2 |
| $CH_3$ | $CCl_2F$ | $-N\begin{smallmatrix}CH_3\\CH_2-CH=CH_2\end{smallmatrix}$ | 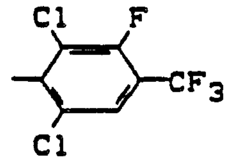 | 2 |
| $CH_3$ | $CCl_2F$ | $-N\begin{smallmatrix}CH_3\\CH_2-C\equiv CH\end{smallmatrix}$ | 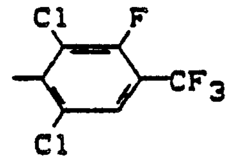 | 2 |
| $CH_3$ | $CCl_2F$ | $-NH-CH_2CH_2-CH_3$ | 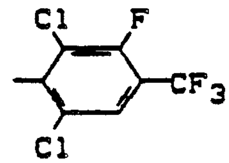 | 2 |
| $CH_3$ | $CCl_2F$ | $-NH-CH(CH_3)_2$ | 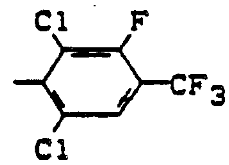 | 2 |
| $CH_3$ | $CCl_2F$ | $-N\begin{smallmatrix}CH_3\\CH_2-CH_2-CH_3\end{smallmatrix}$ | 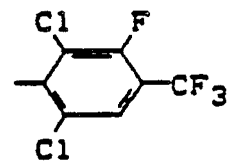 | 2 |
| $CH_3$ | $CCl_2F$ | $-N(CH_2-CH_2-CH_3)_2$ | 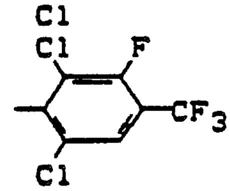 | 2 |
| $CH_3$ | $CCl_2F$ | $-NH-CH_2-CH=CH-CH_3$ | 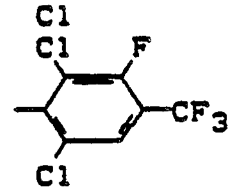 | 2 |
| $CH_3$ | $CCl_2F$ | $-NH-CH_2-C\equiv C-CH_3$ | 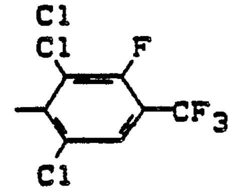 | 2 |

Verwendet man beispielsweise 5-Amino-1-(2-chlor-6-fluor-4-trifluormethylphenyl)-pyrazol und Dichlorfluormethansulfenylchlorid als Ausgangsstoffe, so läßt sich der Reaktionsablauf des erfindungsgemäßen Verfahrens (a) durch das folgende Formelschema darstellen:

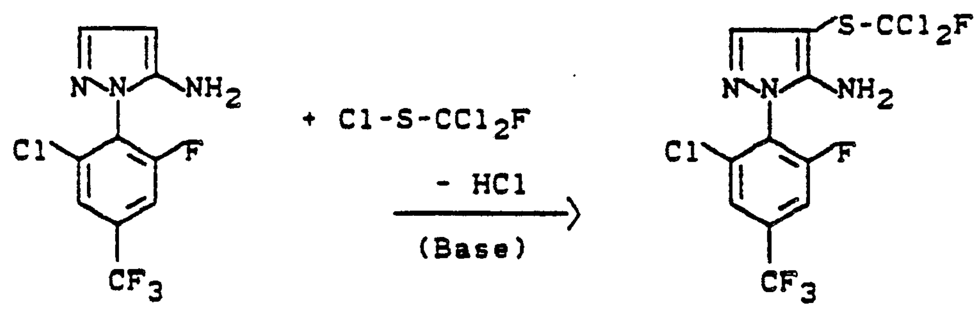


Verwendet man beispielsweise 3-Methyl-5-amino-1-(2,6-dichlor-3-fluor-4-trifluormethylphenyl)-pyrazolyl-3-thiocyanat und Methyliodid als Ausgangsstoffe und Natriumborhydrid als Reduktionsmittel, so läßt sich der Reaktionsablauf des erfindungsgemäßen Verfahrens (b-α) durch das folgende Formelschema darstellen:

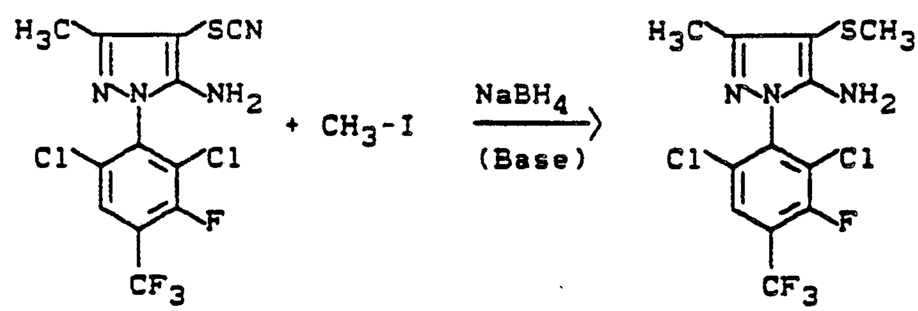


Verwendet man beispielsweise S,S′-Bis-[5-Amino-1-(2,3-difluor-6-chlor-4-trifluormethylphenyl)-pyrazol-3-yl]-disulfid und Ethylbromid als Ausgangsstoffe sowie Natriumdithionit als Reduktionsmittel, so läßt sich der Reaktionsablauf des erfindungsgemäßen Verfahrens (b-β) durch das folgende Formelschema darstellen:

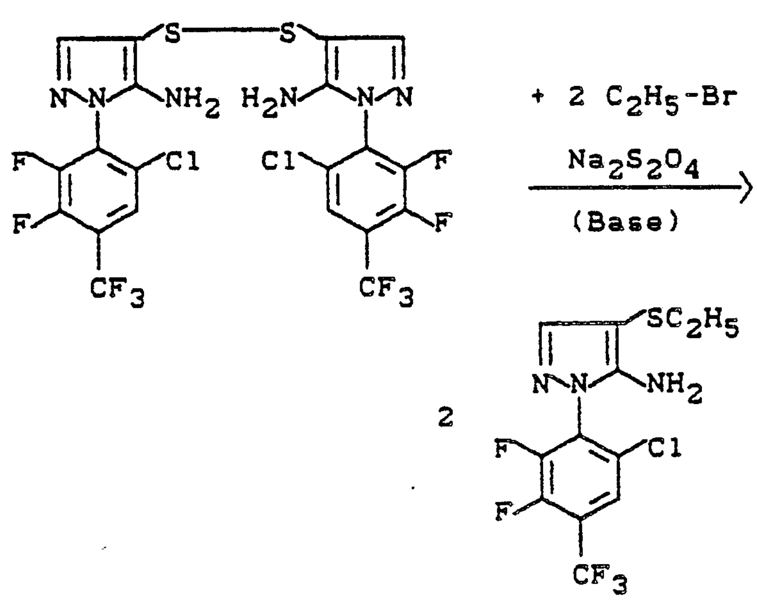


Verwendet man beispielsweise 5-Amino-4-dichlorfluormethylsulfenyl-1-(2-chlor-6-fluor-4-trifluormethylphenyl)-pyrazol als Ausgangsstoff und Wasserstoffperoxid als Oxidationsmittel, so läßt sich der Reaktionsablauf des erfindungsgemäßen Verfahrens (c) durch das folgende Formelschema darstellen:

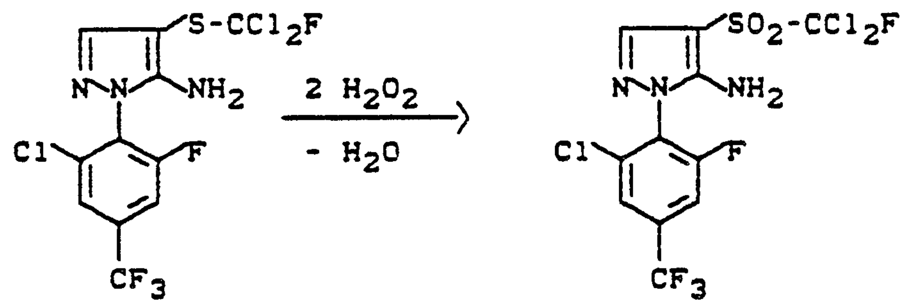


Verwendet man beispielsweise 5-Amino-4-trifluormethylsulfonyl-1-(2,6-dichlor-3-fluor-4-trifluormethylphenyl)-pyrazol und Dimethylsulfat als Ausgangsstoffe, so läßt ich der Reaktionsablauf des erfindungsgemäßen Verfahrens (d) durch das folgende Formelschema darstellen:

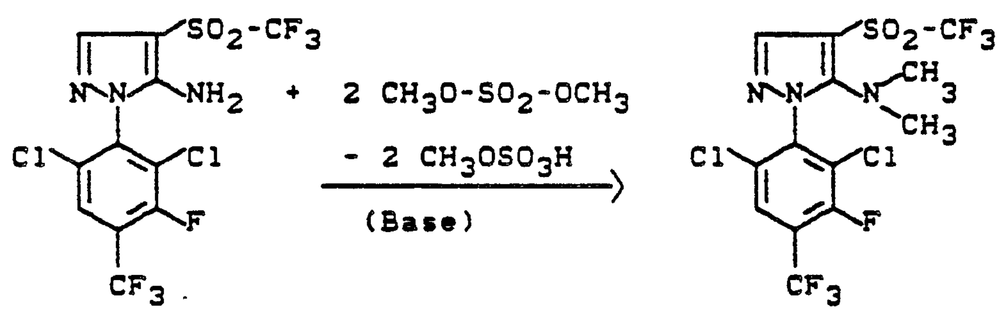


Verwendet man beispielsweise 5-Brom-3-methyl-4-trifluormethylsulfonyl-1-(2,3-difluor-6-chlor-4-trifluormethylphenyl)-pyrazol und Diethylamin als Ausgangsstoffe, so läßt sich der Reaktionsablauf des erfindungsgemäßen Verfahrens (e) durch das folgende Formelschema darstellen:

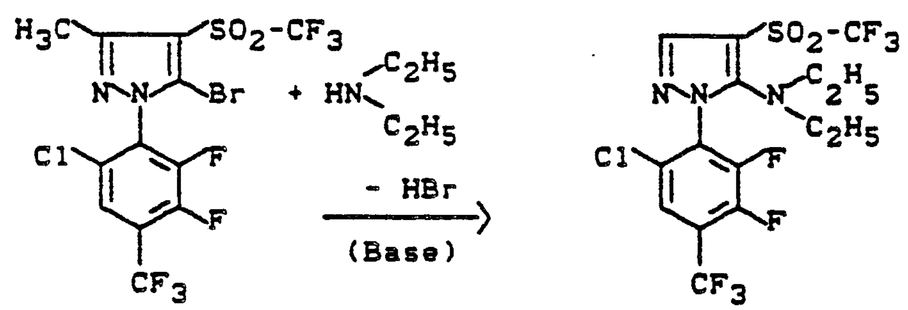


Verwendet man beispielsweise 5-Amino-4-difluorchlormethylsulfenyl-1-(2,3,5-trifluor-4-trifluormethylphenyl)-pyrazol, Natriumnitrit und Bromwasserstoffsäure als Ausgangsstoffe, so läßt sich der Reaktionsablauf des erfindungsgemäßen Verfahrens (f) durch das folgende Formelschema darstellen:

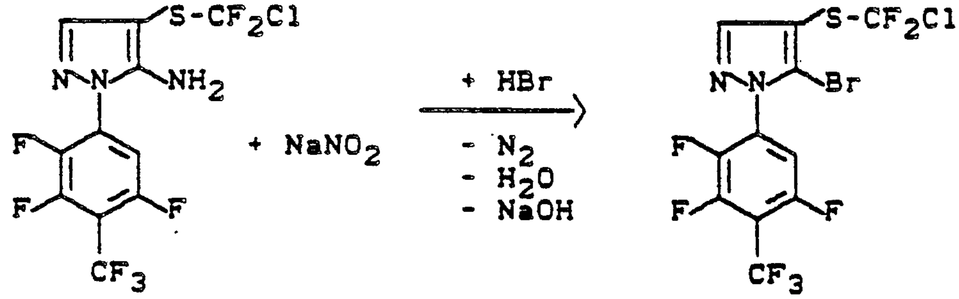

Die zur Durchführung des erfindungsgemäßen Verfahrens (a) als Ausgangsstoffe benötigten 4-unsubstituierten 1-Arylpyrazole sind durch die Formel (II) allgemein definiert. In dieser Formel (II) stehen $R^1$, $R^4$, $R^5$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 4-unsubstituierten 1-Arylpyrazole der Formel (II) sind noch nicht bekannt. Sie sind jedoch teilweise Gegenstand einer eigenen noch nicht publizierten Patentanmeldung (vergl. Deutsche Patentanmeldung P 36 17 977 vom 28.5.1985).

Man erhält sie beispielsweise, wenn man Arylhalogenide der Formel (IX),

$Ar\text{-}Hal^4$ (IX)

in welcher
$Hal^4$ für Halogen, insbesondere für Chlor oder Fluor steht und
Ar die oben angegebene Bedeutung hat,
zunächst in einer ersten Stufe mit Hydrazinhydrat gegebenenfalls in Gegenwart eines Verdünnungsmittels wie beispielsweise Ethanol oder Pyridin bei Temperaturen zwischen 20 °C und 120 °C umsetzt, dann die so erhältlichen Arylhydrazine der Formel (X),

$Ar\text{-}NH\text{-}NH_2$ (X)

in welcher
Ar die oben angegebene Bedeutung hat,
in einer 2. Stufe mit Cyanessigesterderivaten der Formel (XI),

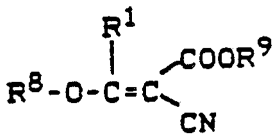

(XI)

in welcher
$R^1$ die oben angegebene Bedeutung hat und
$R^8$ und $R^9$ unabhängig voneinander für Alkyl, insbesondere für Methyl oder Ethyl stehen,
gegebenenfalls in Gegenwart eines Verdünnungsmittels wie beispielsweise Ethanol bei Temperaturen zwischen 20 °C und 120 °C umsetzt; dann die so erhältlichen 1-Arylpyrazol-4-carbonsäureester der Formel (XII),

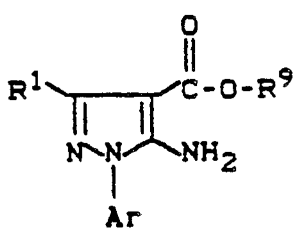

(XII)

in welcher
$R^1$, $R^9$ und Ar die oben angegebene Bedeutung haben,
in einer 3. Stufe mit Säuren wie beispielsweise wässriger Schwefelsäure bei Temperaturen zwischen 80 °C und 150 °C verseift und decarboxyliert und die so erhältlichen 1-Aryl-5-amino-pyrazole der Formel (IIa),

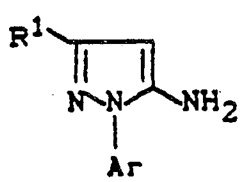

(IIa)

in welcher
$R^1$ und Ar die oben angegebene Bedeutung haben,
gegebenenfalls in einer 4.Stufe mit Alkylierungsmitteln der Formel (VII),

$R^{5-1}$-E (VII)

in welcher
$R^{5-1}$ für Alkyl, Alkenyl oder Alkinyl steht und
E für eine elektronenanziehende Abgangsgruppe wie beispielsweise Halogen, insbesondere Chlor, Brom oder Iod oder für jeweils gegebenenfalls substituiertes Alkoxysulfonyl oder Arylsulfonyloxy wie beispielsweise Methoxysulfonyloxy, Ethoxysulfonyloxy oder p-Toluolsulfonyloxy steht,
gegebenenfalls in Gegenwart eines Verdünnungsmittels wie beispielsweise Dichlormethan oder Acetonitril, gegebenenfalls in Gegenwart eines Säurebindemittels wie beispielsweise Natriumhydroxid und gegebenenfalls in Gegenwart eines Phasentransferkatalysators wie beispielsweise Tributylbenzylammoniumchlorid bei Temperaturen zwischen 20°C und 120°C alkyliert.

1-Aryl-5-aminopyrazole der Formel (IIa),

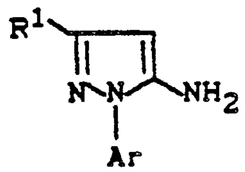

(IIa)

in welcher
$R^1$ und Ar die oben angegebene Bedeutung haben,
erhält man alternativ auch, wenn man Arylhydrazine der Formel (X),

$Ar-NH-NH_2$ (X)

in welcher
Ar die oben angegebene Bedeutung hat,
mit 2-Halogenacrylnitril-Derivaten dr Formel (XIII),

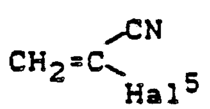

(XIII)

in welcher
$Hal^5$ für Halogen, insbesondere für Chlor oder Brom steht,
oder mit β-Aminoacrylnitrilderivaten der Formel (XIV),

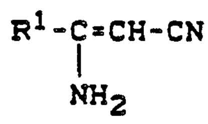

(XIV)

in welcher
$R^1$ die oben angegebene Bedeutung hat,
gegebenenfalls in Gegenwart eines Verdünnungsmittels wie beispielsweise Ethanol sowie gegebenenfalls in Gegenwart eines Reaktionshilfsmittels wie beispielsweise Schwefelsäure oder Trifluoressigsäure bei Temperaturen zwischen 50°C und 150°C umsetzt.

Arylhalogenide der Formel (IX) sind bekannt oder erhältlich in Analogie zu bekannten Verfahren (vergl. z.B. EP 187 023; EP 180 057; US 43 88 472; Zh. org. Khim. 20, 2187-2191 [1984] bzw. CA 102: 112944s; J.Fluorine Chem. 4, 317-326 [1974]; J.chem. Soc. C, 1969, 211-217 sowie die Herstellungsbeispiele).

Cyanessigesterderivate der Formel (XI), 2-Halogenacrylnitrilderivate und β-Aminoacrylnitrilderivate der Formel (XIII) sind allgemein bekannte Verbindungen der organischen Chemie.

Die zur Durchführung des erfindungsgemäßen Verfahrens (a) weiterhin als Ausgangsstoffe benötigten Sulfenylhalogenide sind durch die Formel (III) allgemein definiert. In dieser Formel (III) steht $R^2$ vorzugsweise für diejenigen Reste, die bereits im Zusammenhang mit der Beschreibung der erfindungsgemäßen Stoffe der Formel (I) als bevorzugt für diesen Substituenten genannt wurden.

$Hal^1$ steht für Chlor oder Brom.

Die Sulfenylhalogenide der Formel (III) sind ebenfalls allgemein bekannte Verbindungen der organischen Chemie.

Die zur Durchführung des erfindungsgemäßen Verfahrens (b-α) als Ausgangsstoffe benötigten Thiocyanate sind durch die Formel (IV) allgemein definiert. In dieser Formel (IV) stehen $R^1$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die Thiocyanate der Formel (IV) sind noch nicht bekannt. Man erhält sie jedoch in Analogie zu bekannten Verfahren (vergl. EP 201 852), beispielsweise, wenn man 4-unsubstituierte 1-Arylpyrazole der Formel (IIa),

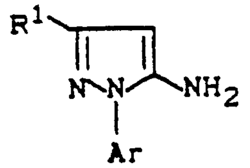

(IIa)

in welcher
$R^1$ und Ar die oben angegebene Bedeutung haben,
mit Ammoniumthiocyanat in Gegenwart von Brom und Essigsäure bei Temperaturen zwischen -20 °C und +20 °C umsetzt.

Die zur Durchführung des erfindungsgemäßen Verfahrens (b-β) als Ausgangsstoffe benötigten Disulfide sind durch die Formel (V) allgemein definiert. In dieser Formel (V) stehen $R^1$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die Disulfide der Formel (V) sind ebenfalls noch nicht bekannt. Man erhält sie jedoch ebenfalls in Analogie zu bekannten Verfahren (vergl. EP 201 852), beispielsweise wenn man Thiocyanate der Formel (IV),

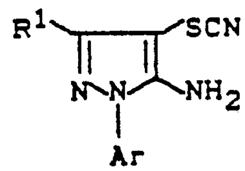

(IV)

in welcher
$R^1$ und Ar die oben angegebene Bedeutung haben,
mit wässriger Salzsäure gegebenenfalls in Gegenwart eines Verdünnungsmittels wie beispielsweise Ethanol bei Temperaturen zwischen 20 °C und 120 °C umsetzt.

Die zur Durchführung des erfindungsgemäßen Verfahrens (b) weiterhin als Ausgangsstoffe benötigten Halogenide sind durch die Formel (VI) allgemein definiert. In dieser Formel (VI) steht $R^2$ vorzugsweise für diejenigen Reste, die bereits im Zusammenhang mit der Beschreibung der erfindungsgemäßen Stoffe der Formel (I) als bevorzugt für diesen Substituenten genannt wurden.

$Hal^2$ steht vorzugsweise für Chlor, Brom oder Iod.

Die Halogenide der Formel (VI) sind allgemein bekannte Verbindungen der organischen Chemie.

Die zur Durchführung des erfindungsgemäßen Verfahrens (c) als Ausgangsstoffe benötigten 1-Arylpyrazole sind durch die Formel (Ia) allgemein definiert. In dieser Formel (Ia) stehen $R^1$, $R^2$, $R^4$, $R^5$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 1-Arylpyrazole der Formel (Ia) sind erfindungsgemäße Verbindungen und erhältlich mit Hilfe der erfindungsgemäßen Verfahren (a), (b), (d), oder (e).

Die zur Durchführung des erfindungsgemäßen Verfahrens (d) als Ausgangsstoffe benötigten 1-Arylpyrazole sind durch die Formel (Ig) allgemein definiert. In dieser Formel (Ig) stehen $R^1$, $R^2$, $R^4$, Ar und n vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden. Die 1-Arylpyrazole der Formel (Ig) sind ebenfalls erfindungsgemäße Verbindungen und erhältlich mit Hilfe der erfindungsgemäßen Verfahren (a), (b), (c) oder (e).

Die zur Durchführung des erfindungsgemäßen Verfahrens (d) und zur Synthese der Vorprodukte der Formel (II) weiterhin als Ausgangsstoffe benötigten Alkylierungsmittel sind durch die Formel (VII) allgemein definiert. In dieser Formel (VII) steht $R^{5-1}$ vorzugsweise für jeweils geradkettiges oder verzweigtes Alkyl, Alkenyl oder Alkinyl mit jeweils bis zu 6 Kohlenstoffatomen, insbe sondere für Methyl, Ethyl, n-oder i-Propyl, n-, i-, s-oder t-Butyl, Allyl, n- oder i-Butenyl, Propargyl sowie n- oder i-Butinyl.
E steht vorzugsweise für Halogen, insbesondere Chlor, Brom oder Iod oder für jeweils gegebenenfalls substituiertes Alkoxysulfonyl oder Arylsulfonyloxy wie beispielsweise Methoxysulfonyloxy, Ethoxysulfonyloxy oder p-Toluolsulfonyloxy steht.

Die Alkylierungsmittel der Formel (VII) sind allgemein bekannte Verbindungen der organischen Chemie.

Die zur Durchführung des erfindungsgemäßen Verfahrens (e) als Ausgangsstoffe benötigten 1-Arylpyrazole sind durch die Formel (Ih) allgemein definiert. In dieser Formel (Ih) stehen $R^1$, $R^2$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

$Hal^3$ steht vorzugsweise für Fluor, Chlor, Brom oder Iod, insbesondere für Chlor oder Brom.

Die 1-Arylpyrazole der Formel (Ih) sind erfindungsgemäße Verbindungen und erhältlich mit Hilfe des erfindungsgemäßen Verfahrens (f).

Die zur Durchführung des erfindungsgemäßen Verfahrens (e) weiterhin als Ausgangsstoffe benötigten Amine sind durch die Formel (VIII) allgemein definiert. In dieser Formel (VIII) stehen $R^4$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (VIII) sind allgemein bekannte Verbindungen der organischen Chemie.

Die zur Durchführung des erfindungsgemäßen Verfahrens (f) als Ausgangsstoffe benötigten 1-Arylpyrazole sind durch die Formel (Ii) allgemein definiert. In dieser Formel (Ii) stehen $R^1$, $R^2$, Ar und n vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

Die 1-Arylpyrazole der Formel (Ii) sind erfindungsgemäße Verbindungen und erhältlich mit Hilfe der erfindungsgemäßen Verfahren (a), (b) oder (c).

Als Verdünnungsmittel zur Durchführung des erfindungsgemäßen Verfahrens (a) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -di-ethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Säuren wie beispielsweise Essigsäure.

Das erfindungsgemäße Verfahren (a) wird gegebenenfalls in Gegenwart eines Reaktionshilfsmittels durchgeführt. Als solche kommen alle üblichen anorganischen oder organischen Basen in Frage. Hierzu gehören beispielsweise Alkalimetallhydroxide, wie Natriumhydroxdid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan(DABCO), Diazabicyclononen (DBN) oder Diazabicycloundecen(DBU).

Die Reaktionstemperaturen können bei der Durchführung des erfindungsgemäßen Verfahrens (a) in einem größeren Bereich variiert werden. Im allgemeinen arbeitet man bei Temperaturen zwischen -20 °C und +120 °C, vorzugsweise bei Temperaturen zwischen 0 °C und +50 °C.

Zur Durchführung des erfindungsgemäßen Verfahrens (a) setzt man pro Mol an 4-unsubstituierten 1-Aryl-pyrazol der Formel (II) im allgemeinen 1.0 bis 2.5 Mol, vorzugsweise 1.0 bis 1.5 Mol an Sulfenylhalogenid der Formel (III) und 1.0 bis 2.5 Mol, vorzugsweise 1.0 bis 1.5 Mol an Reaktionshilfsmittels ein. Die Reaktionsführung, Aufarbeitung und Isolierung der Reaktionsprodukte der Formel (Ia) erfolgt nach allgemein üblichen Verfahren.

Als Verdünnungsmittel zur Durchführung der erfindungsgemäßen Verfahrens (b-α) und (b-β) kommen ebenfalls inerte organische Lösungsmittel in Frage.

Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan,

Cyclohexan, Dichlormethan, Chloroform, Tetrachlorkohlenstoff, Ether, wie Diethylether, Dioxan, Tetrahydrofuran oder Ethylenglykoldimethyl- oder -diethylether oder Alkohole, wie Methanol, Ethanol oder Propanol. Mit besonderem Vorteil verwendet man als Verdünnungsmittel den im Alkylteil ($R^2$) dem Halogenid der Formel (VI) entsprechenden Alkohol, d.h. beispielsweise bei Verwendung von Methyliodid als Halogenid der Formel (VI) kommt mit besonderem Vorzug Methanol als Verdünnungsmittel in Frage.

Als Reduktionsmittel zur Durchführung des erfindungsgemäßen Verfahrens (b-α) verwendet man vorzugsweise komplexe Hydride wie Lithiumaluminiumhydrid, Lithiumborhydrid oder Natriumborhydrid. Besonders geeignet ist Natriumborhydrid.

Als Reduktionsmittel zur Durchführung des erfindungsgemäßen Verfahrens (b-β) kommen alle üblicherweise für Disulfidspaltungen verwendbaren Reduktionsmittel in Frage. Mit besonderem Vorzug verwendet man Dithionite, wie beispielsweise Natriumdithionit.

Die erfindungsgemäßen Herstellungsverfahren (b-α) und (b-β) werden vorzugsweise in Gegenwart einer geeigneten Base als Reaktionshilfsmittel durchgeführt. Als solche verwendet man Alkalimetallhydroxide oder -carbonate, wie beispielsweise Natrium- oder Kaliumhydroxid oder Natrium- oder Kaliumcarbonat.

Die Reaktionstemperaturen können bei der Durchführung der erfindungsgemäßen Verfahren (b-α) und (b-β) in einem größeren Bereich variiert werden. Im allgemeinen arbeitet man bei Temperaturen zwischen 0 °C und +120 °C, vorzugsweise bei Temperaturen zwischen +20 °C und +90 °C.

Zur Durchführung des erfindungsgemäßen Verfahrens (b-α) setzt man pro Mol Thiocyanat der Formel (IV) im allgemeinen 1,0 bis 5,0 Mol, vorzugsweise 1,8 bis 2,5 Mol an Halogenid der Formel (VI) und 1.0 bis 5,0 Mol, vorzugsweise 1,8 bis 2,0 Mol an Reduktionsmittel sowie gegebenenfalls 1,0 bis 5,0 Mol, vorzugsweise 1,5 bis 3,0 Mol an Reaktionshilfsmittel ein. Dabei setzt man zunächst das Thiocyanat der Formel (IV) in dem betreffenden Verdünnungsmittel unter Verwendung einer Stickstoff-Schutzgasatmosphäre mit dem Reduktionsmittel um und setzt nach beendeter Reaktion die als Reaktionshilfsmittel verwendete Base und das Halogenid der Formel (VI) zu. Die Aufarbeitung und Isolierung der Reaktionsprodukte der Formel (Ib) erfolgt nach üblichen Verfahren.

Zur Durchführung des erfindungsgemäßen Verfahrens (b-β) setzt man pro Mol an Disulfid der Formel (V) im allgemeinen 1,0 bis 5,0 Mol, vorzugsweise 1,8 bis 2,5 Mol an Halogenid der Formel (VI) und 1,0 bis 5,0 Mol, vorzugsweise 1,8 bis 2,0 Mol an Reduktionsmittel sowie gegebenenfalls 1,0 bis 5,0 Mol, vorzugsweise 1,5 bis 3,0 Mol an Reaktionshilfsmittel ein. Dabei setzt man zunächst das Disulfid der Formel (V) in dem betreffenden Verdünnungsmittel in Gegenwart der als Reaktionshilfsmittel verwendeten Base bei der entsprechenden Reaktionstemperatur mit dem Reduktionsmittel um, setzt nach einigen Stunden das Halogenid der Formel (VI) zu und erwärmt eine weitere Stunde auf die erforderliche Reaktionstemperatur. Die Aufarbeitung und Isolierung der Reaktionsprodukte der Formel (Ib) erfolgt nach üblichen Verfahren.

Als Oxidationsmittel zur Durchführung des erfindungsgemäßen Verfahrens (c) kommen alle üblichen zur Schwefeloxidation verwendbaren Oxidationsmittel infrage. Insbesondere geeignet sind Wasserstoffperoxid, organische Persäuren, wie beispielsweise Peressigsäure, m-Chlorperbenzoesäure, p-Nitroperbenzoesäure oder Luftsauerstoff.

Als Verdünnungsmittel zur Durchführung des erfindungsgemäßen Verfahrens (c) kommen ebenfalls organische oder auch anorganische Lösungsmittel in Frage. Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Säuren, wie Essigsäure, Propionsäure oder Schwefelsäure, oder dipolar aprotische Lösungsmittel, wie Acetonitril, Aceton, Essigsäureethylester oder Dimethylformamid.

Das erfindungsgemäße Verfahren (c) kann gegebenenfalls in Gegenwart eines Reaktionshilfsmittels durchgeführt werden. Als solche kommen alle üblicherweise verwendbaren organischen und anorganischen Säurebindemittel in Frage. Vorzugsweise verwendet man Erdalkali- oder Alkalimetallhydroxide, -acetate oder -carbonate, wie beispielsweise Calciumhydroxid, Natriumhydroxid, Natriumacetat oder Natriumcarbonat.

Das erfindungsgemäße Verfahren (c) kann gegebenenfalls in Gegenwart eines geeigneten Katalysators durchgeführt werden. Als solche kommen alle üblicherweise für derartige Schwefeloxidationen üblichen Katalysatoren in Frage. Beispielhaft genannt seien in diesem Zusammenhang Schwermetallkatalysatoren wie Ammoniummolybdat.

Die Reaktionstemperaturen können bei der Durchführung des erfindungsgemäßen Verfahrens (c) in einem größeren Bereich variiert werden. Im allgemeinen arbeitet man bei Temperaturen zwischen -20 °C und +70 °C, vorzugsweise bei Temperaturen zwischen 0 °C und +50 °C.

Zur Durchführung des erfindungsgemäßen Verfahrens (c) setzt man pro Mol an 1-Aryl-pyrazol der Formel (Ia) im allgemeinen 0.8 bis 1.2 Mol, vorzugsweise äquimolare Mengen Oxidationsmittel ein, wenn

man die Oxidation des Schwefels auf der Sulfoxidstufe unterbrechen will. Zur Oxidation zum Sulfon setzt man pro Mol an 1-Aryl-pyrazol der Formel (Ia) im allgemeinen 1.8 bis 3.0 Mol, vorzugsweise doppelt molare Mengen an Oxidationsmittel ein. Die Reaktionsführung, Aufarbeitung und Isolierung der End produkte der Formel (Ic) erfolgt nach üblichen Verfahren.

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische, gegebenenfalls halogenierte Hohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (d) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyl-dimethylammoniummethylsulfat, Dimethyl-$C_{12}$/$C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Als Reaktionshilfsmittel zur Durchführung des erfindungsgemäßen Verfahrens (d) kommen alle üblicherweise verwendbaren anorganischen und organischen Basen infrage, Vorzugsweise verwendet man Alkalimetallhydride, -hydroxide, -amide, -alkoholate, -carbonate oder hydrogencarbonate, wie beispielsweise Natriumhydrid, Natriumamid, Natriummethylat, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (d) in einem größeren Bereich variiert werden. Im allgemeinen arbeitet man zwischen -20 °C und +150 °C, vorzugsweise zwischen 0 °C und + 100 °C.

Zur Durchführung des erfindungsgemäßen Verfahrens (d) setzt man pro Mol 1-Aryl-pyrazol der Formel (Ig) im allgemeinen 1.0 bis 20.0 Mol, vorzugsweise 1.0 bis 15.0 Mol an Alkylierungsmittel der Formel (VII) und gegebenenfalls 1.0 bis 3.0 Mol, vorzugsweise 1.0 bis 2.0 Mol an Reaktionshilfsmittel sowie gegebenenfalls 0,01 bis 1,0 Mol an Phasentransferkatalysator ein. Die Reaktionsdurchführung, Aufarbeitung und Isolierung der Reaktionsprodukte der Formel (Id) erfolgt in allgemein üblicher Art und Weise.

Als Verdünnungsmittel zur Durchführung des erfindungsgemäßen Verfahrens (e) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (e) kann gegebenenfalls in Gegenwart eines geeigneten Reaktionshilfsmittels durchgeführt werden. Als solche kommen alle üblichen anorganischen oder organischen Basen in Frage. Hierzu gehören beispielsweise Alkalimetallhydroxide, wie Natriumhydroxdid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist jedoch auch möglich, einen entsprechenden Überschuß an dem als reaktionspartner eingesetzten Amin der Formel (VIII) gleichzeitig als Reaktionshilfsmittel zu verwenden.

Die Reaktionstemperaturen können bei der Durchführung des erfindungsgemäßen Verfahrens (e) in einem größeren Bereich variiert werden. Im allgemeinen arbeitet man bei Temperaturen zwischen -20 °C und +200 °C, vorzugsweise bei Temperaturen zwischen 0 °C und +150 °C.

Zur Durchführung des erfindungsgemäßen Verfahrens (e) setzt man pro Mol an 1-Arylpyrazol der Formel (Ih) im allgemeinen 1.0 bis 10.0 Mol, vorzugsweise 1.0 bis 5.0 Mol an Amin der Formel (VIII) ein. Die Reaktionsdurchführung, Aufarbeitung und Isolierung der Reaktionsprodukte der Formel (Ie) erfolgt nach allgemein üblichen Verfahren.

Als Verdünnungsmittel zur Durchführung des erfindungsgemäßen Verfahrens (f) kommen alle üblicherweise für derartige Diazotierungsreaktionen üblichen Lösungsmittel in Frage. Vorzugsweise verwendet man Halogenkohlenwasserstoffe wie Chloroform oder Bromoform oder wässrige Säuren wie beispielsweise Halogenwasserstoffsäuren oder Schwefelsäure, wobei die Säurekomponente gleichzeitig als Reagenz und/oder als Reaktionshilfsmittel fungiert. Bei der Verwendung von Bromoform als Verdünnungsmittel erhält man in der Regel die entsprechenden 5-Brom-pyrazole, wobei das Bromoform gleichzeitig als Verdünnungsmittel und als Reagenz fungiert.
Die entsprechende Reaktion in Gegenwart von Chloroform als Verdünnungsmittel ergibt im allgemeinen eine Mischung aus 5-Chlor-pyrazolverbindungen der Formel (If) und den analogen reduzierten Verbindungen der Formel (If) die in der 5-Position des Pyrazolringes einen Wasserstoffrest tragen. Diese Mischungen lassen sich destillativ auftrennen.

Das erfindungsgemäße Verfahren (f) wird in Gegenwart eines anorganischen oder organischen Nitrits durchgeführt. Als solche kommen alle üblicherweise für derartige Diazotierungsreaktionen üblichen Nitritverbindungen in Frage. Besonders bevorzugt verwendet man Alkalimetallnitrite, wie beispielsweise Natriumnitrit oder Alkylnitrite wie beispielsweise t-Butylnitrit oder n-Pentylnitrit.

Das erfindungsgemäße Verfahren (f) wird gegebenenfalls in Gegenwart einer Halogenwasserstoffsäure durchgeführt. In diesem Fall erhält man als Reaktionsprodukte 1-Aryl-pyrazole der Formel (If), bei welchen der Rest $R^6$ für einen Halogenrest steht, der dem Anion der verwendeten Halogenwasserstoffsäure entspricht. Vorzugsweise verwendet man jeweils wässrige Lösungen der Fluorwasserstoffsäure, Chlorwasserstoffsäure, Bromwasserstoffsäure oder Iodwasserstoffsäure.

Das erfindungsgemäße Verfahren (f) wird üblicherweise in Gegenwart eines Reaktionshilfsmittels durchgeführt. Als solche kommen insbesondere starke Mineralsäuren wie Schwefelsäure oder Phosphorsäure oder die oben aufgeführten Halogenwasserstoffsäuren in Frage, die in diesem Fall gleichzeitig als Reagenz und als Katalysator wirken.

Das erfindungsgemäße Verfahren (f) kann gegebenenfalls in Gegenwart eines geeigneten Reduktionsmittels durchgeführt werden. In diesem Fall erhält man als Reaktionsprodukte 1-Aryl-pyrazole der Formel (If), bei welchen der Rest $R^6$ für Wasserstoff steht. Als Reduktionsmittel verwendet man in diesen Fällen besonders bevorzugt unterphosphorige Säure ($H_3PO_2$).

Die Reaktionstemperaturen können bei der Durchführung des erfindungsgemäßen Verfahrens (f) in einem größeren Bereich variiert werden. Im allgemeinen arbeitet man bei Temperaturen zwischen -30 °C und +60 °C, vorzugsweise bei Temperaturen zwischen -20 °C und +40 °C.

Zur Durchführung des erfindungsgemäßen Verfahrens (f) setzt man pro Mol an 1-Arylpyrazol der Formel (Ii) im allgemeinen 1,0 bis 1,8 Mol an Nitrit, gegebenenfalls 1,0 bis 20,0 Mol, vorzugsweise 1,0 bis 10,0 Mol an Halogenwasserstoffsäure, gegebenenfalls 1,0 bis 50,0 Mol, vorzugsweise 1,0 bis 20,0 Mol an Reduktionsmittel und gegebenenfalls 1,0 bis 20,0 Mol, vorzugsweise 1,0 bis 10,0 Mol an als Reaktionshilfsmittel verwendete Mineralsäure ein.

Dabei setzt man üblicherweise der Reaktionsmischung bestehend aus 1-Arylpyrazol der Formel (Ii), Mineralsäure, Verdünnungsmittel und Halogenwasserstoffsäure bzw. Reduktionsmittel das Nitrit in kleinen Portionen gegebenenfalls in geeignetem Verdünnungsmittel gelöst zu.

Die Aufarbeitung und Isolierung der Reaktionsprodukte der Formel (If) erfolgt nach üblichen Methoden z.B durch Abfiltrieren von kristallinen Produkten oder durch Extraktion mit einem geeigneten organischen Lösungsmittel. Die Identifizierung erfolgt durch Schmelzpunkt oder Protonen-Kernresonanz-Spektrum.

Erfindungsgemäße Verbindungen der Formel ($If_1$),

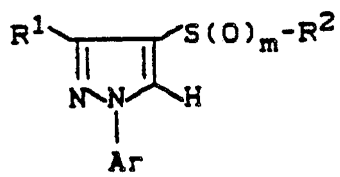

($If_1$)

in welcher
$R^2$ und Ar die oben angegebene Bedeutung haben, und
m für eine Zahl 1 oder 2 steht,
erhält man alternativ auch aus den erfindungsgemäßen Verbindungen der Formel ($If_2$),

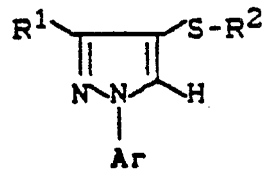

(If2)

in welcher
$R^2$ und Ar die oben angegebene Bedeutung haben,
wenn man in üblicher Art und Weise mit Oxidationsmitteln in Analogie zur Durchführung des erfindungsgemäßen Verfahrens (c) gegebenenfalls in Gegenwart eines Verdünnungsmittels wie beispielsweise Dichlormethan und gegebenenfalls in Gegenwart eines Katalysators wie beispielsweise Ammoniummolybdat und gegebenenfalls in Gegenwart eines Reaktionshilfsmittels wie beispielsweise Natriumcarbonat oder Natriumbicarbonat bei Temperaturen zwischen 0° C und 50° C am Schwefel der Sulfenylgruppe in 4-Position des Pyrazolringes oxidiert.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aud der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossy piella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica·alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium

pharaonis, Vespa spp.

Aus der Ordnung der diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räubemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensible und resistente Arten und Stämme, sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide Wirksamkeit aus. Sie lassen sich mit besonders gutem Erfolg zur Bekämpfung von pflanzenschädigenden Insekten, wie beispielsweise gegen die schwarze Bohnenblattlaus (Aphis fabae) oder gegen die Larven der Meerettichblattkäfer (Phaedon cochleariae) oder gegen die grüne Pfirsichblattlaus (Myzus persicae) ebenso wie zur Bekämpfung von Bodeninsekten, wie beispielsweise gegen die Maden der Zwiebelfliege (Phorbia antiqua) oder von Diabrotica balteata-Larven im Boden einsetzen. Dabei zeigen die erfindungsgemäßen Wirkstoffe sowohl wurzelsystemische als auch blattsystemische Eigenschaften.

Außerdem besitzen die erfindungsgemäß verwendbaren Wirkstoffe eine hohe Wirkung gegen Hygiene- und Vorratsschädlinge und lassen sich beispielsweise zur Bekämpfung der deutschen Schabe (Blattella germanica) oder zur Bekämpfung der gemeinen Stubenfliege (Musca domestica) einsetzen. Darüber hinaus lassen sich die erfindungsgemäßen Wirkstoffe mit besonders gutem Erfolg zur Bekämpfung von parasitisch lebenden Warmblüterschädlingen, wie beispielsweise gegen Larven der Goldfliege (Lucilia cuprina), gegen rinderzecken (Boophilus microplus), gegen Stechfliegen (Stomoxys calcitrans), gegen die Weideviehfliege (Musca autumnalis) oder gegen den endoparasitisch lebenden Nematoden der Gattung Caenorhabditis elegans einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylke ton, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer use. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Die biologische Wirksamkeit der erfindungsgemäßen Verbindungen soll anhand der folgenden Beispiele erläutert werden:

Herstellungsbeispiele:

Beispiel 1:

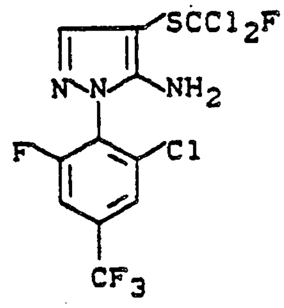

(Verfahren a)

Zu 10 g (0,036 Mol) 5-Amino-1-(2-chlor-6-fluor-4-trifluormethylphenyl)-pyrazol in 50 ml Eisessig gibt man bei 15 °C-20 °C 4,5 ml (0,043 Mol) Dichlorfluormethansulfenylchlorid, rührt 7 Stunden bei Raumtemperatur, tropft dann die Mischung in 300 ml Eiswasser, filtriert den so entstandenen Niederschlag ab und trocknet im Vakuum bei 50 °C.

Man erhält 13,1 g (89 % der Theorie) an 5-Amino-4-dichlorfluormethylthio-1-(2-chlor-6-fluor-4-trifluormethylphenyl)-pyrazol vom Schmelzpunkt 98 °C bis 100 °C.

Beispiel 2:

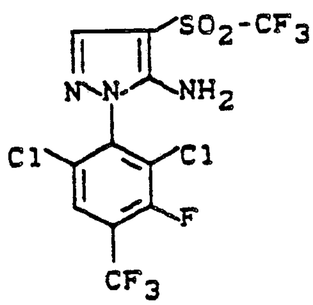

(Verfahren c)

Zu einer Lösung von 6,0 g (0,0145 Mol) 5-Amino-4-trifluormethylthio-1-(2,6-dichlor-3-fluor-4-trifluormethylphenyl)-pyrazol in 30 ml 80-prozentiger Schwefelsäure gibt man bei Raumtemperatur 4,5 ml (0,05 Mol) 35-prozentige Wasserstoffperoxid-Lösung, rührt 20 Stunden bei Raumtemperatur, tropft dann die Mischung in 150 ml Eiswasser, filtriert den so entstandenen Niederschlag ab, wäscht mit Wasser nach und trocknet im Vakuum bei 50 °C.

Man erhält 6,0 g (93 % der Theorie) an 5-Amino-4-trifluormethylsulfonyl-1-(2,6-dichlor-3-fluor-4-trifluormethylphenyl)-pyrazol vom Schmelzpunkt 134 °C-139 °C.

Beispiel 3:

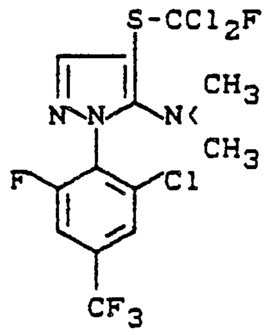

(Verfahren d)

30 g (0.0073 Mol) 5-Amino-4-dichlorfluormethylthio-1-(2-chlor-6-fluor-4-trifluormethyl-phenyl)-pyrazol werden in 550 ml Dichlormethan gelöst und nacheinander mit 150 ml 40-prozentiger wässriger Natronlauge, drei Spatelspitzen Tributylbenzylammomiumchlorid und 1,6 ml (0,016 Mol) 97-prozentigem Dimethylsulfat versetzt und 16 Stunden bei Raumtemperatur gerührt. Die wässrige Phase wird abgetrennt, die organische Phase mit Wasser gewaschen und im Vakuum vom Lösungsmittel befreit. Der Rückstand wird in 30 ml Ethanol aufgenommen, mit 3 ml 25-prozentigem wässrigen Ammoniak versetzt und 5 bis 10 Stunden gerührt. Danach wird das Lösungsmittel im Vakuum entfernt, der Rückstand in 100 ml Dichlormethan gelöst und die organische Phase nacheinander mit wässriger Ammoniumchlorid- und Kochsalzlösung gewaschen, über Magnesiumsulfat getrocknet und im Vakuum eingeengt.

Man erhält 2,9 g (91 % der Theorie) an 4-Dichlorfluormethylthio-5-dimethylamino-1-(2-chlor-6-fluor-4-trifluormethyl-phenyl)-pyrazol als Öl.

$^1$H-NMR ($CDCl_3$/TMS): $\delta$ = 2,83 (s); 7,48 (dd); 7,67 (m); 7,79 (d).

Beispiel 4:

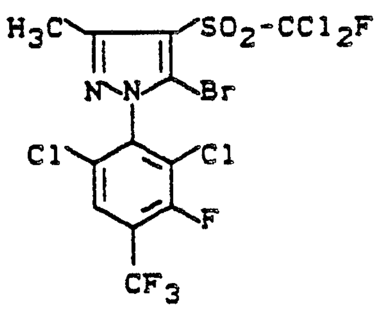

(Verfahren f)

Zu einer Lösung aus 6,9 g (0,014 Mol) 5-Amino-3-methyl-4-dichlorfluormethylsulfonyl-1-(2,6-dichlor-3-fluor-4-trifluormethyl-phenyl)-pyrazol in 65 ml Bromoform gibt man tropfenweise unter Rühren bei Raumtemperatur 5,0 ml (0,042 Mol) t-Butylnitrit, rührt nach beendeter Zugabe weitere 20 Stunden bei Raumtemperatur und engt im Hochvakuum ein.

Man erhält 6,2 g (80 % der Theorie) und 5-Brom-3-methyl-4-dichlorfluormethylsulfonyl-1-(2,6-dichlor-3-fluor-4-trifluormethyl-phenyl)-pyrazol vom Schmelzpunkt 98 °C bis 100 °C.

Beispiel 5:

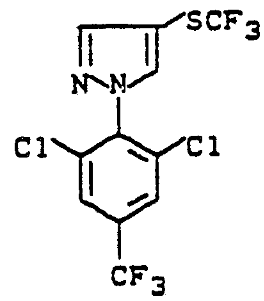

(Verfahren f)

Zu 4 g (0,01 Mol) 5-Amino-4-trifluormethylthio-1-(2-chlor-6-fluor-4-trifluormethyl-phenyl)-pyrazol in 30 ml Tetrahydrofuran gibt man bei Raumtemperatur 2,8 ml (0,021 Mol) n-Pentylnitrit, erhitzt 3 Stunden auf Rückflußtemperatur, destilliert das Lösungsmittel im Vakuum ab und destilliert den Rückstand im Hochvakuum (Kugelrohrdestillation).

Man erhält 3 g (78 % der Theorie) an 4-Trifluormethylthio-1-(2-chlor-6-fluor-4-trifluormethylphenyl)-pyrazol vom Brechungsindex $n_D^{20}$ : 1.4762.

In entsprechender Weise und gemäß den allgemeinen Angaben zur Herstellung erhält man die folgenden 1-Arylpyrazole der allgemeinen Formel (I):

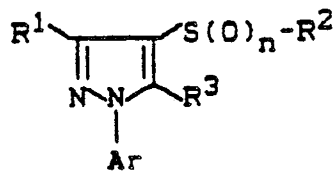

(I)

| Bsp. Nr. | $R^1$ | $-S(O)_n-R^2$ | $R^3$ | Ar | physikalische Eigenschaften |
|---|---|---|---|---|---|
| 6 | H | $-SCF_3$ | $-NH_2$ | 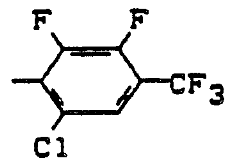 | Fp:86° C |
| 7 | H | $-SCCl_2F$ | $-NH_2$ | 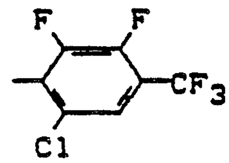 | Fp:85° C |
| 8 | H | $-\overset{O}{\overset{\|}{S}}-CCl_2F$ | $-NH_2$ | 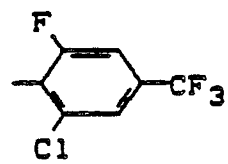 | Fp:73-78° C |
| 9 | H | $-SO_2-CCl_2F$ | $-NH_2$ | 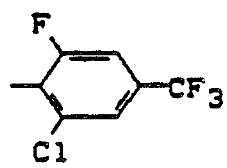 | Fp:111-114° C |
| 10 | H | $-SCF_3$ | $-NH_2$ | 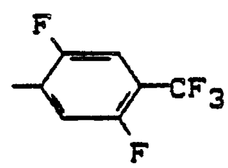 | $^1$H-NMR*): 7,7 (1H) |
| 11 | H | $-SCF_3$ | $-NH_2$ | 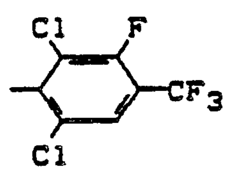 | Fp:110-112° C |
| 12 | H | $-SCCl_2F$ | $-NH_2$ | 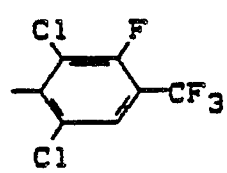 | Fp:115-117° C |

| Bsp. Nr. | $R^1$ | $-S(O)_n-R^2$ | $R^3$ | Ar | physikalische Eigenschaften |
|---|---|---|---|---|---|
| 13 | H | $-S(O)-CCl_2F$ | $-NH_2$ | 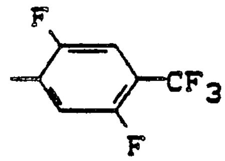 | Fp:93-100° C |
| 14 | H | $-SCF_3$ | $-NH_2$ | 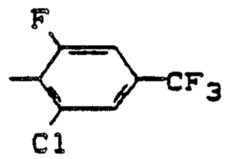 | Fp:75-78° C |
| 15 | H | $-S(O)-CF_3$ | $-NH_2$ | 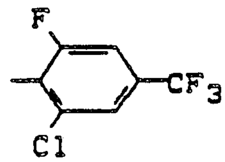 | Fp:128-129° C |
| 16 | H | $-SO_2-CF_3$ | $-NH_2$ | 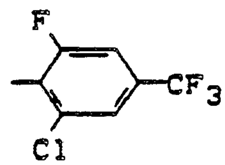 | Fp:140-141° C |
| 17 | H | $-SCF_3$ | H | 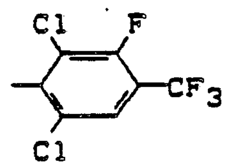 | $n_D^{20}$ : 1,489 |
| 18 | H | $-SCF_3$ | H | 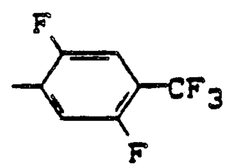 | Kp:98° C/ 0,3mbar |
| 19 | H | $-S(O)-CF_3$ | $-NH_2$ | 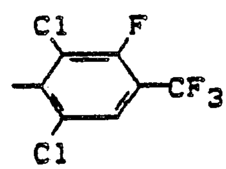 | Fp:76-90° C |

| Bsp. Nr. | $R^1$ | $-S(O)_n-R^2$ | $R^3$ | Ar | physikalische Eigenschaften |
|---|---|---|---|---|---|
| 20 | H | 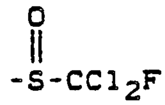 | $-NH_2$ | 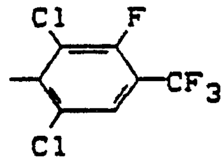 | Fp:85-109° C |
| 21 | H | $-SO_2-CCl_2F$ | $-NH_2$ | 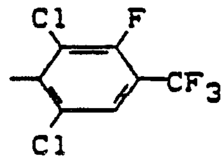 | Fp:172-175° C |
| 22 | H | $-SCClF_2$ | $-NH_2$ | 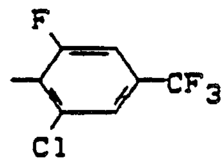 | Fp:107° C |
| 23 | H | 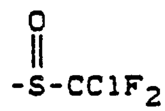 | $-NH_2$ | 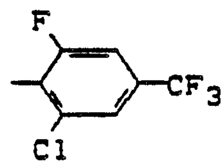 | Fp:58-64° C |
| 24 | H | $-SO_2-CClF_2$ | $-NH_2$ | 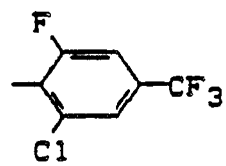 | Fp:121° C |
| 25 | $CH_3$ | $-SCCl_2F$ | $-NH_2$ | 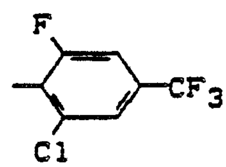 | Fp:88-90° C |
| 26 | $CH_3$ | $-SCClF_2$ | $-NH_2$ | 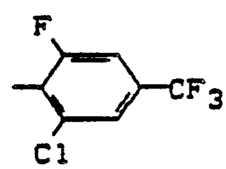 | Fp:108° C |

| Bsp. Nr. | $R^1$ | $-S(O)_n-R^2$ | $R^3$ | Ar | physikalische Eigenschaften |
|---|---|---|---|---|---|
| 27 | $CH_3$ | $-SCF_3$ | $-NH_2$ | 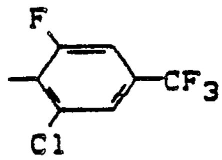 | Fp: 106° C |
| 28 | $CH_3$ | $-\overset{O}{\overset{\|}{S}}-CF_3$ | $-NH_2$ | 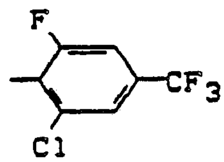 | Fp: 72-74° C |
| 29 | $CH_3$ | $-SO_2-CF_3$ | $-NH_2$ | 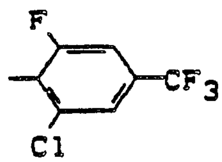 | Fp: 127-129° C |
| 30 | $CH_3$ | $-SCF_3$ | H | 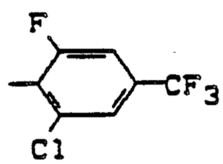 | Fp: 52-55° C |
| 31 | $CH_3$ | $-SCF_3$ | $-NH-CH_3$ | 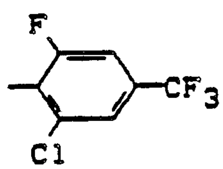 | Fp: 94-95° C |
| 32 | $CH_3$ | $-SCF_3$ | $-NH-C_2H_5$ | 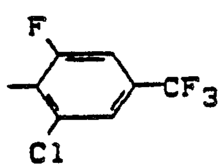 | Fp: 88-89° C |
| 33 | $CH_3$ | $-SCF_3$ | $-NH_2$ | 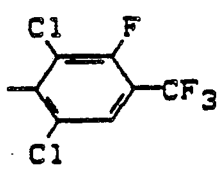 | Fp: 115° C |

| Bsp. Nr. | $R^1$ | $-S(O)_n-R^2$ | $R^3$ | Ar | physikalische Eigenschaften |
|---|---|---|---|---|---|
| 34 | $CH_3$ | 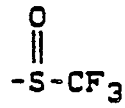 | H | 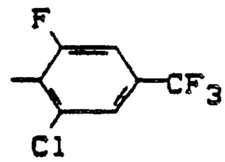 | Fp:72-75° C |
| 35 | $CH_3$ | $-SO_2-CF_3$ | H | 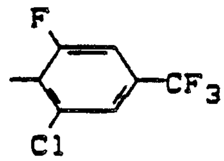 | Fp:89° C |
| 36 | $CH_3$ | $-SCF_3$ | H | 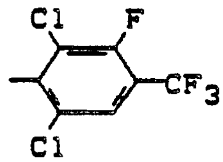 | $n_D^{20}$ :1,4968 |
| 37 | $CH_3$ | $-SCF_3$ | $-NH-CH_3$ | 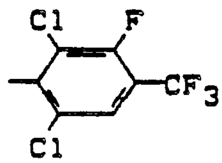 | Fp:67-68° C |
| 38 | $CH_3$ | 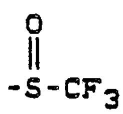 | $-NH_2$ | 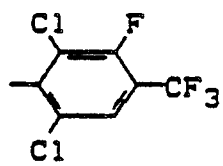 | Fp:134° C |
| 39 | $CH_3$ | $-SO_2-CF_3$ | $-NH_2$ | 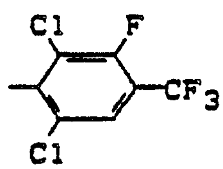 | Fp:144° C |
| 40 | $CH_3$ | 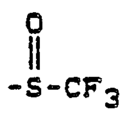 | H | 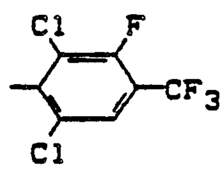 | Fp:78-79° C |

| Bsp. Nr. | $R^1$ | $-S(O)_n-R^2$ | $R^3$ | Ar | physikalische Eigenschaften |
|---|---|---|---|---|---|
| 41 | $CH_3$ | $-SO_2-CF_3$ | H | 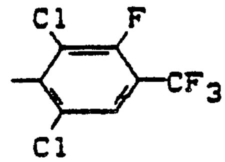 | Fp: 68-70° C |
| 42 | $CH_3$ | $-S(=O)-CClF_2$ | $-NH_2$ | 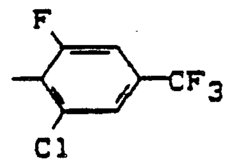 | Fp: 67-69° C |
| 43 | $CH_3$ | $-SO_2-CClF_2$ | $-NH_2$ | 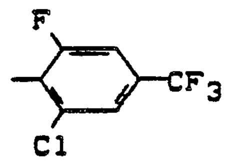 | Fp: 127-129° C |
| 44 | $CH_3$ | $-SCCl_2F$ | $-NH_2$ | 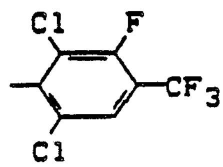 | Fp: 121-122° C |
| 45 | $CH_3$ | $-SCF_3$ | $-N(C_2H_5)_2$ | 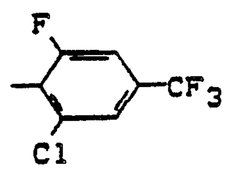 | $n_D^{20}$ : 1,4900 |
| 46 | $CH_3$ | $-SCF_3$ | $-N(CH_3)_2$ | 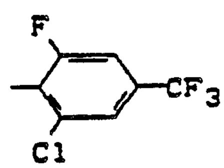 | $n_D^{20}$ : 1,4925 |
| 47 | $CH_3$ | $-S(=O)-CF_3$ | $-NH_2$ | 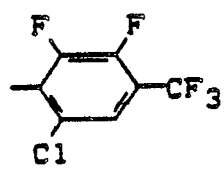 | Fp: 71-73° C |

| Bsp. Nr. | $R^1$ | $-S(O)_n-R^2$ | $R^3$ | Ar | physikalische Eigenschaften |
|---|---|---|---|---|---|
| 48 | $CH_3$ | $-SO_2-CF_3$ | $-NH_2$ | 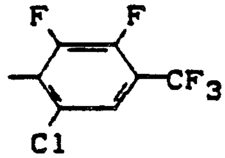 | Fp:71-73° C |
| 49 | $CH_3$ | $-SCF_3$ | H | 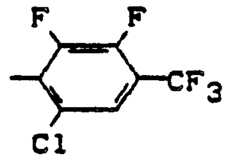 | Fp:48-50° C |
| 50 | $CH_3$ | $-S(=O)-CF_3$ | H | 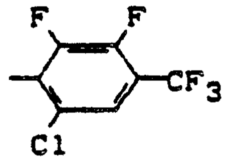 | Fp:83-85° C |
| 51 | $CH_3$ | $-SO_2-CF_3$ | H | 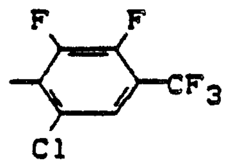 | $n_D^{20}$ :1,4755 |
| 52 | $CH_3$ | $-SO_2-CF_3$ | $-NH-CH_3$ | 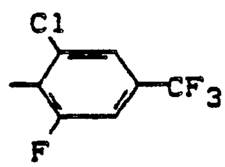 | Fp:101-102° C |
| 53 | $CH_3$ | $-SO_2-CF_3$ | $-NH-C_2H_5$ | 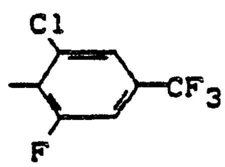 | Fp:109-110° C |
| 54 | $CH_3$ | $-SCCl_2F$ | $-NH_2$ | 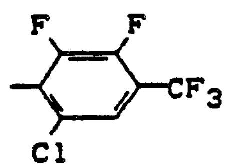 | Fp:85-87° C |
| 55 | $CH_3$ | $-S-CClF_2$ | $-NH_2$ | 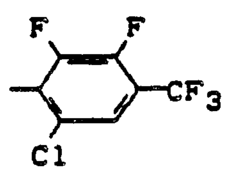 | Fp:92-94° C |

| Bsp. Nr. | $R^1$ | $-S(O)_n-R^2$ | $R^3$ | Ar | physikalische Eigenschaften |
|---|---|---|---|---|---|
| 56 | $CH_3$ | $-S-CF_3$ | $-NH_2$ | 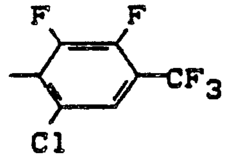 | Fp:80-82° C |
| 57 | $CH_3$ | $-S-CF_3$ | $-NH-CH_3$ | 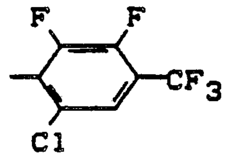 | Fp:103° C |
| 58 | $CH_3$ | $-\overset{\overset{O}{\Vert}}{S}-CCl_2F$ | $-NH_2$ | 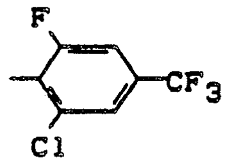 | Fp:74-76° C |
| 59 | $CH_3$ | $-SO_2-CCl_2F$ | $-NH_2$ | 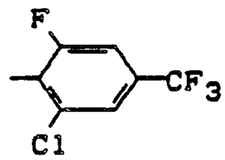 | Fp:119-20° C |
| 60 | H | $-S-CCl_2F$ | $-NH_2$ | 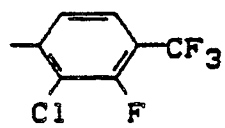 | Fp:73-76° C |
| 61 | H | $-SO-CCl_2F$ | $-NH_2$ | 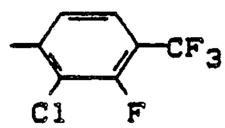 | Fp:75-78° C |
| 62 | H | $-SO_2-CCl_2F$ | $-NH_2$ | 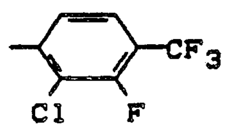 | Fp:154-57° C |
| 63 | $CH_3$ | $-\overset{\overset{O}{\Vert}}{S}-CCl_2F$ | $-NH_2$ | 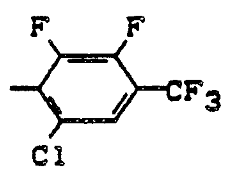 | Fp: 75-77° C |

| Bsp. Nr. | $R^1$ | $-S(O)_n-R^2$ | $R^3$ | Ar | physikalische Eigenschaften |
|---|---|---|---|---|---|
| 64 | $CH_3$ | $-SO_2-CCl_2F$ | $-NH_2$ | 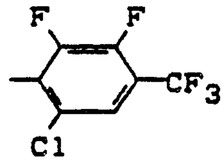 | Fp:73-77° C |
| 65 | $CH_3$ | $-SCF_3$ | $-NH-CH_2-CH=CH_2$ | 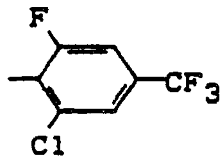 | Fp:85-86° C |
| 66 | $CH_3$ | $-SCF_3$ | $-NH-CH_2-C\equiv CH$ | 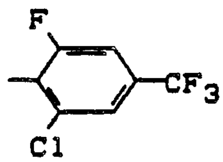 | Fp:79-80° C |
| 67 | $CH_3$ | $-SCF_3$ | $-N(CH_2-CH=CH_2)_2$ | 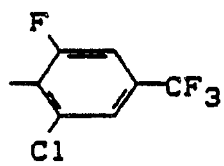 | Fp:138-139° C |
| 68 | $CH_3$ | $-SCF_3$ | $-N(CH_2-C\equiv CH)_2$ | 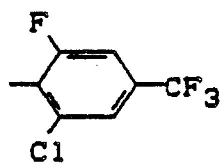 | Fp:137° C |
| 69 | H | $-SCCl_2F$ | $-NH-CH_2-C\equiv CH$ | 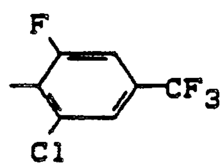 | Fp:69-73° C |
| 70 | H | $-SCCl_2F$ | $-N(CH_2-C\equiv CH)_2$ | 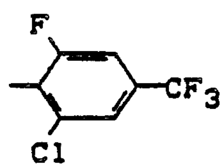 | $^1H$-NMR: 7.78 (s) |

| Bsp. Nr. | $R^1$ | $-S(O)_n-R^2$ | $R^3$ | Ar | physikalische Eigenschaften |
|---|---|---|---|---|---|
| 71 | H | $-SCF_3$ | $-NH-CH_3$ | 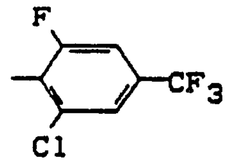 | Fp:80-81° C |
| 72 | H | $-SCCl_2F$ | $-NH-CH_3$ | 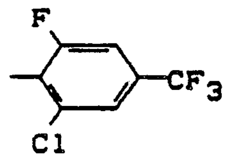 | Fp:124-126° C |
| 73 | H | 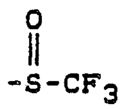 | $-NH-CH_3$ | 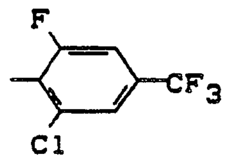 | Fp:129-130° C |
| 74 | H | 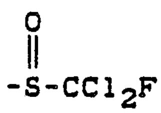 | $-NH-CH_3$ | 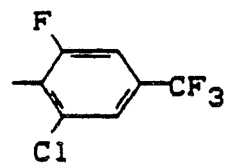 | Fp:70° C |
| 75 | H | $-SO_2-CF_3$ | $-NH-CH_3$ | 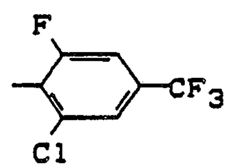 | Fp:97-100° C |
| 76 | H | $-SO_2-CCl_2F$ | $-NH-CH_3$ | 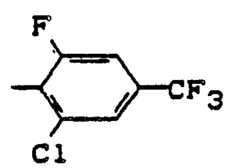 | Fp:104-108° C |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Herstellung der Ausgangsverbindungen:

Beispiel II-1:

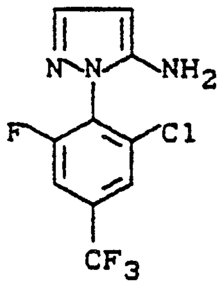

250 g (0,71 Mol) 5-Amino-4-ethoxycarbonyl-1-(2-chlor-6-fluor-4-trifluormethylphenyl)-pyrazol werden in 650 ml 50-%iger wässriger Schwefelsäure 2 Stunden auf 120 °C erhitzt, wobei flüchtige Bestandteile abdestillieren. Man erhitzt für weitere 5 Stunden auf 115 °C bis 120 °C, kühlt dann ab, gibt 3 l Eiswasser zu, stellt mit wäßriger Natronlauge auf pH 8 bis 9 ein, filtriert den entstandenen Niederschlag ab, wäscht mit Wasser nach und trocknet bei 50 °C im Vakuum.

Man erhält 163 g (82 % der Theorie) an 5-Amino-1-(2-chlor-6-fluor-4-trifluormethylphenyl)-pyrazol vom Schmelzpunkt 87-90 °C.

In entsprechender Weise und gemäß den allgemeinen Angaben zur Herstellung erhält man die folgenden 1-Arylpyrazole der allgemeinen Formel (II):

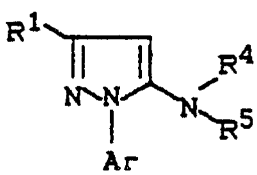

(II)

| Bsp. Nr. | $R^1$ | $-N\langle^{R^4}_{R^5}$ | Ar | Schmelzpunkt /° C |
|---|---|---|---|---|
| II-2 | H | $-NH_2$ | 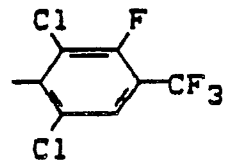 | 69-79 |
| II-3 | H | $-NH_2$ | 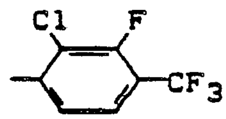 | 112-114 |
| II-4 | H | $-NH_2$ | 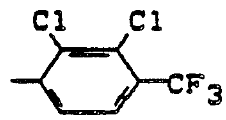 | 139-140 |
| II-5 | H | $-NH_2$ | 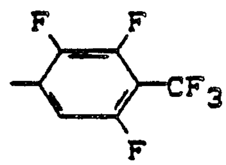 | 116-117 |
| II-6 | H | $-NH_2$ | 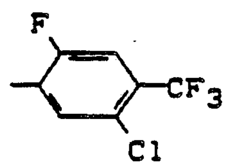 | 62-64 |
| II-7 | H | $-NH_2$ | 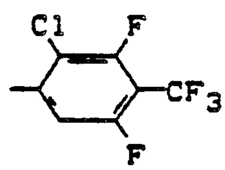 | 93-97 |
| II-8 | H | $-NH_2$ | 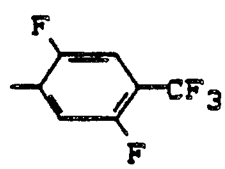 | 95-97 |
| II-9 | H | $-NH_2$ | 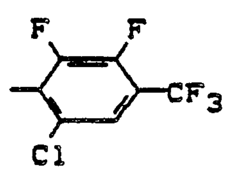 | 85-89 |

| Bsp. Nr. | $R^1$ | $-N\langle^{R^4}_{R^5}$ | Ar | Schmelzpunkt /°C Brechungs-index |
|---|---|---|---|---|
| II-10 | $CH_3$ | $-NH_2$ | 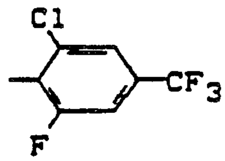 | 61-62 |
| II-11 | $CH_3$ | $-NH-C_2H_5$ | 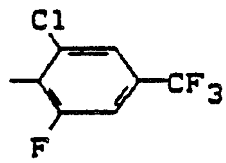 | $n_D^{20}$ :1,5255 |
| II-12 | $CH_3$ | $-NH(C_2H_5)_2$ | 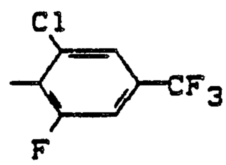 | $n_D^{20}$ :1,5016 |
| II-13 | $CH_3$ | $-NH_2$ | 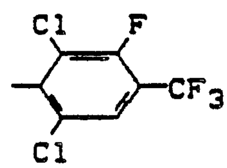 | $n_D^{20}$ :1,5122 |
| II-14 | $CH_3$ | $-NH_2$ | 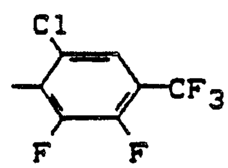 | $^1$H-NMR*) 2,23; 7,59 |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Beispiel XII-1:

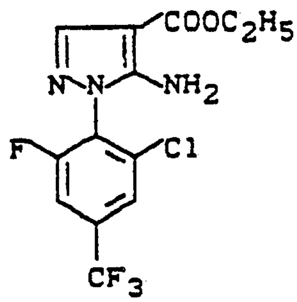

200 g (0,88 Mol) 2-Chlor-6-fluor-4-trifluormethylphenylhydrazin und 151 g (0,88 Mol) Ethoxymethylencyanessigsäureethylester in 400 ml Ethanol werden 30 Stunden auf Rückflußtemperatur erwärmt, dann auf ca. die Hälfte des Volumens eingeengt, abgekühlt, der so erhaltene Niederschlag abfiltriert, mit wenig kaltem Ethanol gewaschen und getrocknet.

Man erhält 250 g (81 % der Theorie) an 5-Amino-1-(2-chlor-6-fluor-4-trifluormethylphenyl)-pyrazol-4-yl-carbonsäureethylester vom Schmelzpunkt 147-149 °C.

In entsprechender Weise und gemäß den allgemeinen Angaben zur Herstellung erhält man die folgenden 1-Arylpyrazol-4-carbonsäureester der allgemeinen Formel (XII):

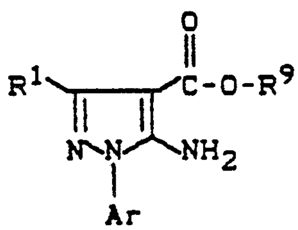

(XII)

| Bsp. Nr. | $R^1$ | $R^9$ | Ar | physikalische Eigenschaften |
|---|---|---|---|---|
| XII-2 | H | $C_2H_5$ | 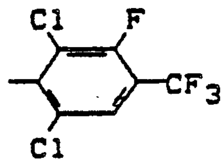 | Fp: 70-74° C |
| XII-3 | H | $C_2H_5$ | 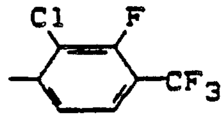 | Fp: 119-126° C |
| XII-4 | H | $C_2H_5$ | 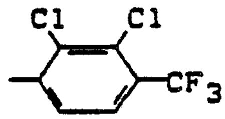 | Fp: 174-175° C |
| XII-5 | H | $C_2H_5$ | 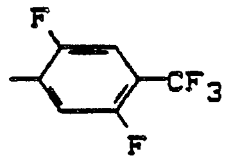 | Fp: 132-133° C |
| XII-6 | H | $C_2H_5$ | 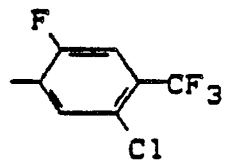 | Fp: 139° C |
| XII-7 | H | $C_2H_5$ | 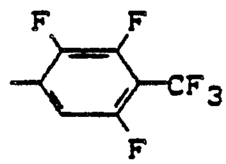 | $^1$H-NMR*): 7,87 |
| XII-8 | H | $C_2H_5$ | 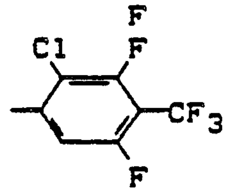 | Fp: 171-175° C |
| XII-9 | H | $C_2H_5$ | 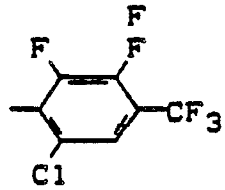 | Fp: 88° C |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Beispiel X-1:

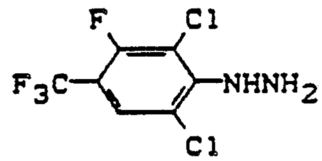

In 1000 ml Ethanol werden 470 g (1,87 Mol) 3,5-Dichlor-2,4-difluor-benzotrifluorid vorgelegt und 142 g (2,84 Mol) Hydrazinhydrat zudosiert und anschließend für 3 Stunden zum Rückfluß erhitzt. Danach wird das Lösungsmittel unter reduziertem Druck abdestilliert und der Rückstand in 1000 ml kaltes Wasser eingerührt. Nach 30 Minuten wird abgesaugt und das Festprodukt im Umluftschrank getrocknet.
Man erhält 445 g (90 % der Theorie) 2,6-Dichlor-3-fluor-4-trifluomethyl-phenylhydrazin mit einem Schmelzpunkt von 50 bis 51 °C.

Beispiel X-2:

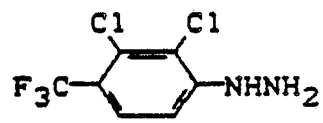

Es werden 100 g (0,4 Mol) 2,3,4-Trichlorbenzotrifluorid vorgelegt, 200 ml Pyridin und anschließend 100 g (2,0 Mol) Hydrazinhydrat zugefügt und dann 12 Stunden auf Rückflußtemperatur erhitzt. Danach wird das Pyridin zu 90 % abdestilliert und der verbleibende Rückstand in 250 ml Wasser eingerührt. Das kristalline Produkt wird abge saugt, mit etwas Wasser gewaschen und getrocknet. Man erhält 78 g (80 % der Theorie) 2,3-Dichlor-4-trifluormethyl-phenylhydrazin mit einem Schmelzpunkt von 79 bis 80 °C.

In entsprechender Weise und gemäß den allgemeinen Angaben zur Herstellung erhält man die folgenden Arylhydrazine der Formel (X):

$Ar - NH - NH_2$ (X)

| Bsp. Nr. | Ar | Schmelzpunkt /° C |
|---|---|---|
| X-3 | 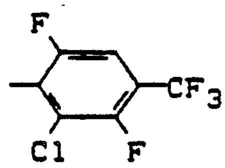 | 72-83 |
| X-4 | 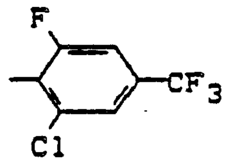 | 60-61 |
| X-5 | 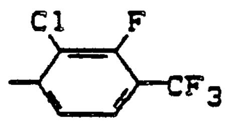 | 102-103 |
| X-6 | 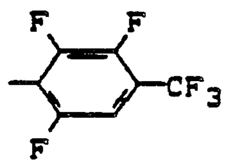 | 60-62 |
| X-7 | 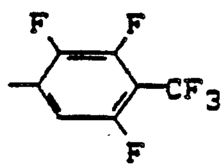 | 69-70 |

| Bsp. Nr. | Ar | Schmelzpunkt /°C |
|---|---|---|
| X-8 | 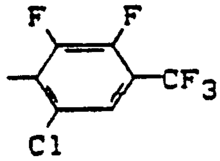 | 80 |
| X-9 | 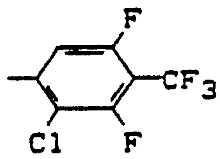 | 82-84 |
| X-10 | 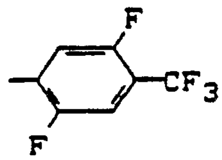 | 41 |
| X-11 | 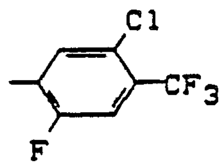 | 69-70 |

Beispiel IX-1/IX-2:

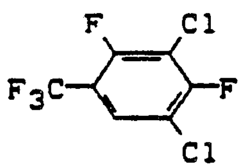

+

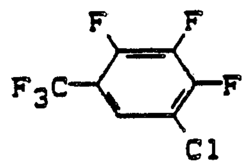

Zu 800 g (13,8 Mol) Kaliumfluorid in 1800 ml trockenem Tetramethylensulfon gibt man 900 g (3,17 Mol) 2,3,4,5-Tetrachlorbenzotrifluorid (vgl. z.B. EP 150 587) und erhitzt 10 Stunden auf 200 °C. Zur Aufarbeitung wird im Vakuum eingeengt und destilliert.

Man erhält zunächst 50 g (6,8 % der Theorie) an 2,3,4-Trifluor-5-chlor-benzotrifluorid vom Siedepunkt (Kp) 31-38 °C bei 10 mbar und vom Brechungsindex $n_D^{20}$ = 1,4130 und als 2.Fraktion 490 g (61,6 % der Theorie) an 2,4-Difluor-3,5-dichlorbenzotrifluorid vom Siedepunkt 57-59 °C bei 10 mbar und vom Brechungsindex $n_D^{20}$ = 1,4510.

Beispiel IX-3:

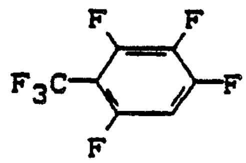

252,5 g (1 Mol) 3-Chlor-2,4,5,6-tetrafluorbenzotrifluorid (vergl. z.B. Zh. obshch. Khim. 37, 1686-1687 [1967] und 86 g (1,05 Mol) Natriumacetat in 1000 ml Eisessig werden in Gegenwart von 10 g Palladium auf Aktivkohle (5%ig) bei einem Wasserstoffdruck von 30 bis 50 bar und 120 °C bis zur Druckkonstanz hydriert. Zur Aufarbeitung filtriert man aus der erkalteten Reaktionsmischung den Katalysator ab und destilliert den Rückstand.

Man erhält 210 g (96 % der Theorie) an 2,3,4,6-Tetrafluorbenzotrifluorid vom Siedepunkt 105-106 °C und vom Brechungsindex $n_D^{20}$ = 1,3770.

In entsprechender Weise erhält man die folgenden Arylhalogenide der formel (IX):

$$Ar - Hal^4 \qquad (IX)$$

| Bsp. Nr. | Ar | $Hal^4$ | Siedepunkt/mbar Brechungsindex $n_D^{20}$ |
|---|---|---|---|
| IX-4 | 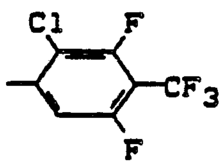 | F | Kp: 145° C/760<br>$n_D^{20}$ = 1,4170 |
| IX-5 | 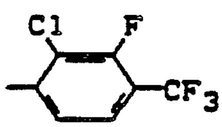 | F | Kp: 37° C/16<br>$n_D^{20}$ = 1,4268 |
| IX-6 | 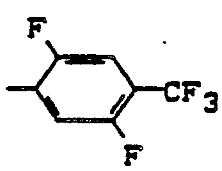 | F | Kp: 104° C/760<br>$n_D^{20}$ = 1,3862 |
| IX-7 | 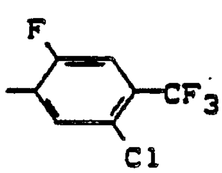 | F | Kp: 130-140° C/760<br>$n_D^{20}$ = 1,4250 |

Anwendungsbeispiele:

In den folgenden Anwendungsbeispielen wurden die nachstehend aufgeführten Verbindungen als Vergleichssubstanzen eingesetzt:

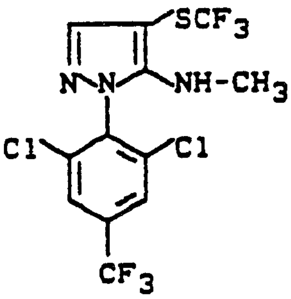

(A)

5-Methylamino-4-trifluormethylthio-1-(2,6-dichlor-4-trifluormethyl-phenyl)-pyrazol

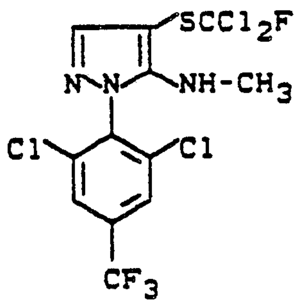

;

(B)

5-Methylamino-4-dichlorfluormethylthio-1-(2,6-dichlor-4-trifluormethyl-phenyl)-pyrazol
(beide bekannt aus EP 201 852)

Beispiel A

$LT_{100}$-Test für Dipteren

Testtiere: Musca domestica, resistent
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterm Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100

%igen knock down-Effekt notwendig ist.

Bei diesem Test zeigen z.B. die folgenden Verbindungen der Herstellungsbeispiele überlegene Wirkung gegenüber dem Stand der Technik: 5, 6, 14, 15, 16, 17, 22, 23, 25, 26, 27, 28, 30, 31, 32, 34, 35 und 36.

Beispiel B

$LD_{100}$-Test

Testtiere: Blattella germanica
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man 10 Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden; 0 % bedeutet, daß keine Testtiere abgetötet wurden.

Bei diesem Test zeigen z.B. die folgenden Verbindungen der Herstellungsbeispiele überlegene Wirkung gegenüber dem Stand der Technik: 6, 8, 10, 11, 14, 15, 22, 23, 26, 27, 30, 31, 32, 33, 34 und 35.

Beispiel C

Aphis-Test (systemische Wirkung)

Lösungsmittel: 7 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Aphis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 15, 23, 28 und 29.

Beispiel D

Grenzkonzentrations-Test / Bodeninsekten

Testinsekt: Phorbia antiqua-Maden (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.
Bei diesem Test zeigen z.B. die folgenden Verbindungen der Herstellungsbeispiele überlegene Wirksamkeit gegenüber dem Stand der Technik: 25, 28, 30, 31 und 34.

## Beispiel E

Grenzkonzentrationstest / Bodeninsekten

Testtiere: Diabrotica balteata - Larven im Boden
Lösungsmittel: 3 Gewichtsteile Aceton
Emulgator; 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration. Die Wirkstoffzubereitung wird innig mit dem Boden vermischt. Dabei spielt die Konzentration des Wirkstoffes in der Zubereitung praktisch keine Rolle, entsprechend ist allein die Wirkstoffgewichtsmenge pro Volumeneinheit im Boden, welche in ppm (mg/l) angegeben wird. Man füllt den Boden in 0,5 l Töpfe und läßt diese bei 20° C stehen.

Sofort nach dem Ansatz werden je Topf 6 vorgekeimte Maiskörner gelegt. Nach 2 Tagen werden in den behandelten Boden die entsprechenden Testinsekten gesetzt. Nach weiteren 7 Tagen wird der Wirkungsgrad des Wirkstoffes durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie in der unbehandelten Kontrolle.
Bei diesem Test zeigen z.B. die folgenden Verbindungen der Herstellungsbeispiele überlegene Wirksamkeit gegenüber dem Stand der Technik: 28.

## Beispiel F

Grenzkonzentrations-Test / Wurzelsystemische Wirkung

Testinsekt: Phaedon cochleariae-Larven
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 5, 8, 9, 11, 13, 19, 20, 26, 27, 28, 29, 31, 33, 34 und 38.

## Beispiel G

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirkung gegenüber dem Stand der Technik: 1, 8, 9, 15, 16, 22, 23, 24, 27, 28, 29, 31, 32 und 43.

## Beispiel H

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele

Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 8, 9, 14, 19, 27 und 28.

Beispiel I

Test mit Lucilia cuprina resistent-Larven

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 1, 2, 5, 8, 9, 11, 14, 16, 19, 21, 22, 23, 24, 26, 28, 29, 34 und 43.

Beispiel K

Test mit parasitierenden, adulten Stechfliegen (Stomoxys calcitrans)

Lösungsmittel: Cremophor

Zur Herstellung einer zweckmäßigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

10 adulte Stechfliegen (Stomoxys calcitrans) werden in Petrischalen, die Sandwiches enthalten, die einen Tag vor Versuchsbeginn mit 1 ml de zu testenden Wirkstoffzubereitung durchtränkt wurden, gebracht. Nach 3 Stunden wird der Abtötungsgrad in Prozent bestimmt, wobei 100 % bedeuten, daß alle und 0 %, daß keine Fliegen abgetötet worden sind.

Bei diesem Test zeigen z.B. die folgenden Verbindungen der Herstellungsbeispiele überlegene Wirkung gegenüber dem Stand der Technik: 2, 26 und 34.

Beispiel L

Facefly - Test (Musca autumnalis)

Lösungsmittel: 35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

10 adulte Facefiles (Musca autumnalis) werden in Petrischalen gebracht, die Filterpapierscheiben entsprechender Größe enthalten, die einen Tag vor Versuchsbeginn mit 1 ml der zu testenden Wirkstoffzubereitung durchtränkt wurden. Nach 3 Stunden wird der Abtötungsgrad in Prozent bestimmt, wobei 100%

bedeuten, daß alle und 0%, daß keine Fliegen abgetötet worden sind.

Bei diesem Test zeigt z.B. die folgende Verbindung der Herstellungsbeispiele überlegene Wirkung gegenüber dem Stand der Technik: 2, 11, 26 und 34.

Beispiel: M

Zeckentest (Boophilus microplus)/Hemmung der Eiablage

Lösungsmittel: 35 Gewichtsteile Ethylenglykolmonomethylether
35 Gewichtsteile Nonylphenolpolyether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

In der Wirkstoffzubereitung werden adulte, vollgesogene Zeckenweibchen der Arten Boophilus microplus (OP-resistenter Biarra Stamm) eine Minute lang getaucht. Nach dem Tauchen von je 10 weiblichen Exemplaren der verschiedenen Zeckenarten überführt man diese in Petrischalen, deren Boden mit einer entsprechend großen Filterscheibe belegt ist.

Nach 10 Tagen wird die Wirksamkeit der Wirkstoffzubereitung bestimmt durch Ermittlung der Hemmung der Eiablage gegenüber unbehandelten Kontrollzecken. Die Wirkung drückt man in Prozent aus, wobei 100 % bedeutet, daß keine Eier mehr abgelegt wurden und 0 % besagt, daß die Zecken Eier in normaler Menge ablegen.

Bei diesem Test zeigen z.B. die folgenden Verbindungen der Herstellungsbeispiele überlegene Wirkung gegenüber dem Stand der Technik: 1, 2, 8, 9, 11, 14, 15, 16, 19, 22, 23, 24, 25, 26, 28, 29, 42 und 43.

Beispiel :N

In vitro Nematodentest

Caenorhabditis elegans

$10^{-4}$ g Wirkstoff werden in 1 ml Wasser oder 0,1 ml Dimethylsulfoxid (DMSO) gelöst. Diese Lösung wird auf eine Replica-Platte gegeben. Dazu gibt man 2 ml einer E.coli-Suspension gegeben, zu der man vorher 10-20 weibliche Tiere oder Larven von Caenorhabditis elegans in 0,5 ml sterile M9 Pufferlösung gegeben hat. Die E.coli-Suspension wird hergestellt indem man 300 ml einer Übernachtkultur einer Uracilbedürftigen E.coli-Stammes mit 1,8 l steriler M9 Pufferlösung versetzt.

Der Versuchsansatz wird 7 Tage bei 22° C inkubiert und danach ausgewertet. Es wurde bewertet, inwieweit der Wirkstoff die Vermehrung beeinträchtigt und die Konzentration angegeben bei der die Vermehrung verhindert wird.

Bei diesem Test zeigen z.B. die folgenden Verbindungen der Herstellungsbeispiele eine mindestens 95 %ige Hemmung der Vermehrung des Nematoden C.elegans.

- bei einer Konzentration von ≦ 100 µg/ml:
1, 5, 10, 13, 14, 15, 22, 23, 25, 28, 34, 38 und 42
- bei einer Konzentration von ≦ 10 µg/ml:
2, 8, 9, 11, 12, 16, 17, 21, 24, 26, 29, 31, 33, 36, 37, 39 und 43.

## Ansprüche

1) 1-Arylpyrazole der allgemeinen Formel

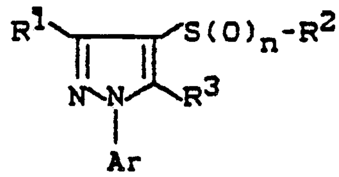 (I)

in welcher
$R^1$ für Wasserstoff oder Alkyl steht,
$R^2$ für Alkyl oder Halogenalkyl steht,
$R^3$ für Wasserstoff, Halogen,
oder für einen Rest

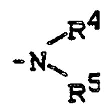

steht,
wobei
$R^4$ und $R^5$ unabhängig voneinander jeweils für Wasserstoff, Alkyl, Alkenyl oder Alkinyl stehen,
Ar für einen der Reste

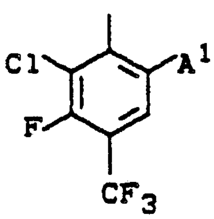 ; 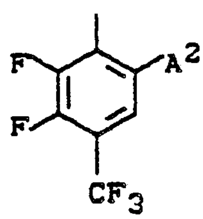 ; 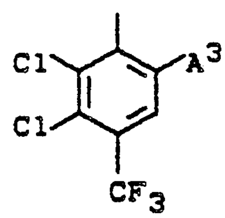 ;

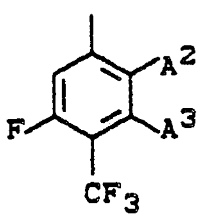 ; 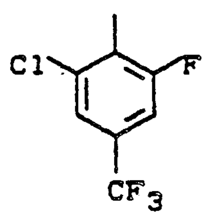 oder 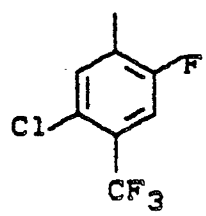

steht, wobei
$A^1$ für Wasserstoff, Fluor oder Chlor steht,
$A^2$ für Fluor oder Chlor steht und
$A^3$ für Wasserstoff oder Fluor steht und
n für eine Zahl 0, 1 oder 2 steht,
mit Ausnahme der Verbindung 5-Amino-1-(2,5-difluor-4-trifluormethyl-phenyl)-4-dichlorfluormethylthiopyrazol.

2) 1-Arylpyrazole der Formel (I) gemäß Anspruch 1), wobei
$R^1$ für Wasserstoff oder für geradkettiges oder verzweigtes Alkyl mit 1 bis 4 Kohlenstoffatomen steht,
$R^2$ für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,
$R^3$ für Wasserstoff, Fluor, Chlor, Brom oder für einen Rest

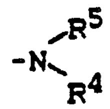

steht, wobei
$R^4$ und $R^5$ unabhängig voneinander jeweils für Wasserstoff oder für jeweils geradkettiges oder verzweigtes Alkyl, Alkenyl oder Alkinyl mit jeweils bis zu 6 Kohlenstoffatomen stehen,
Ar für einen der Reste

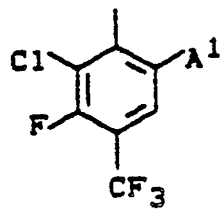 ; 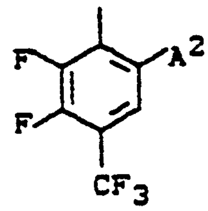 ; 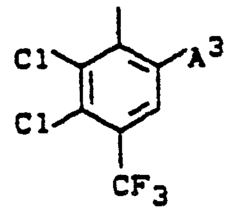 ;

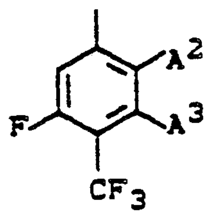 ; 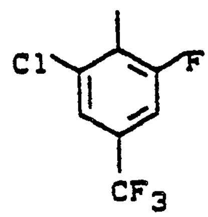 oder 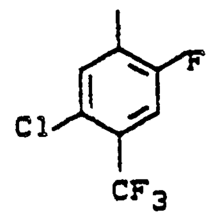

steht, wobei
$A^1$ für Wasserstoff, Fluor oder Chlor steht,
$A^2$ für Fluor oder Chlor steht und
$A^3$ für Wasserstoff oder Fluor steht und
n für eine Zahl 0, 1 oder 2 steht,
mit Ausnahme der Verbindung 5-Amino-1-(2,5-difluor-4-trifluormethyl-phenyl)-4-dichlorfluormethylthiopyrazol.

3) 1-Arylpyrazole der allgemeinen Formel (I) gemäß Anspruch 1), in welcher
$R^1$ für Wasserstoff, Methyl oder Ethyl steht,
$R^2$ für Methyl, Ethyl, n- oder i-Propyl, n-, i-, s-oder t-Butyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluorichlorethyl, Tetrafluorchlorethyl, Heptafluorpropyl, Chlorethyl, Bromethyl, Chlorpropyl oder Brompropyl steht,
$R^3$ für Wasserstoff, Fluor, Chlor oder Brom oder für einen Rest

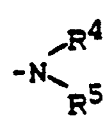

steht,
wobei
$R^4$ und $R^5$unabhängig voneinander für Wasserstoff, Methyl, Ethyl, n- oder i-Propyl, n-, i-, s-oder t-Butyl, Allyl, n- oder i-Butenyl, Propargyl sowie n- oder i-Butinyl stehen,
Ar für einen der Reste

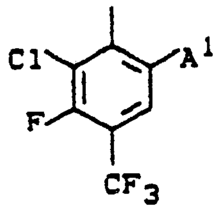 ; 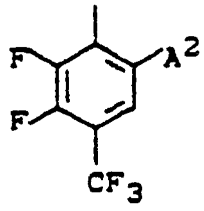 ; 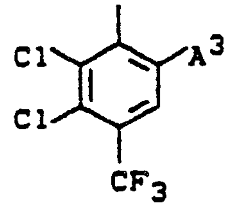 ;

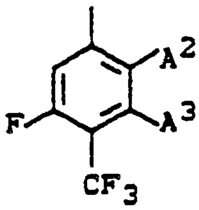 ; 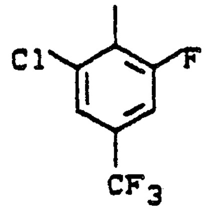 oder 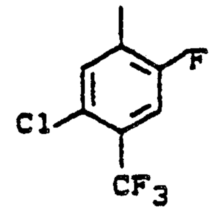

steht, wobei
$A^1$ für Wasserstoff, Fluor oder Chlor steht,
$A^2$ für Fluor oder Chlor steht und
$A^3$ für Wasserstoff oder Fluor steht und
n für eine Zahl 0, 1 oder 2 steht,
mit Ausnahme der Verbindung 5-Amino-1-(2,5-difluor-4-trifluormethyl-phenyl)-4-dichlorfluormethylthiopyrazol.

4) 1-Arylpyrazole der allgemeinen Formel (I) gemäß Anspruch 1), in welcher
$R^1$ für Wasserstoff oder Methyl steht,
$R^2$ für Methyl, Ethyl, Trifluormethyl, Dichlorfluormethyl oder Difluorchlormethyl steht,
$R^3$ für Wasserstoff, Chlor, Brom oder für einen Rest

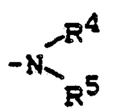

steht,
$R^4$ und $R^5$ unabhängig voneinander für Wasserstoff, Methyl, Ethyl, n- oder i-Propyl, n-, i-, s-Butyl, Allyl, n- oder i-Butenyl, Propargyl sowie n- oder i-Butinyl stehen,
Ar für einen der Reste

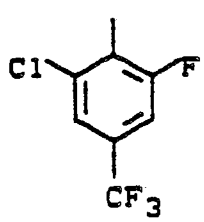 ; 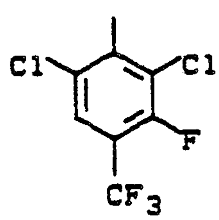 oder 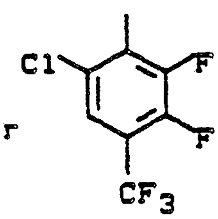

steht und n für eine Zahl 0, 1 oder 2 steht.

5) Verfahren zur Herstellung von 1-Arylpyrazolen der allgemeinen Formel (I)

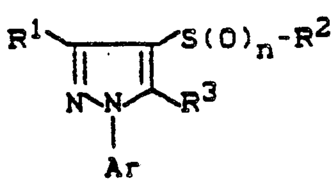 (I)

in welcher

$R^1$ für Wasserstoff oder Alkyl steht,
$R^2$ für Alkyl oder Halogenalkyl steht,
$R^3$ für Wasserstoff, Halogen,
oder für einen Rest

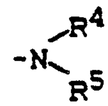

steht,
wobei
$R^4$ und $R^5$ unabhängig voneinander jeweils für Wasserstoff, Alkyl, Alkenyl oder Alkinyl stehen,
Ar für einen der Reste

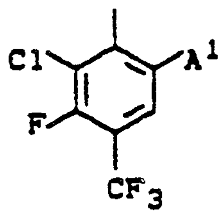 ; 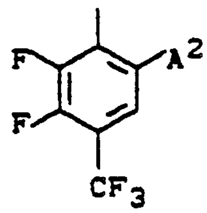 ; 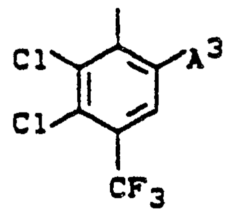 ;

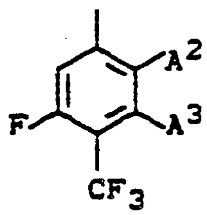 ; 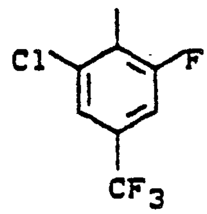 oder 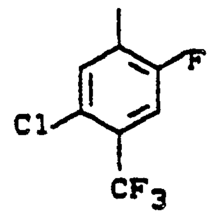

steht, wobei
$A^1$ für Wasserstoff, Fluor oder Chlor steht,
$A^2$ für Fluor oder Chlor steht und
$A^3$ für Wasserstoff oder Fluor steht und
n für eine Zahl 0, 1 oder 2 steht,
mit Ausnahme der Verbindung 5-Amino-1-(2,5-difluor-4-trifluormethyl-phenyl)-4-dichlorfluormethylthiopyrazol,
dadurch gekennzeichnet, daß man
(a) zum Erhalt von 1-Arylpyrazolen der Formel (Ia),

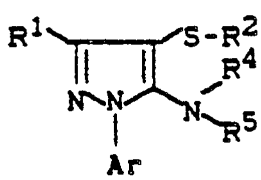 (Ia)

in welcher
$R^1$, $R^2$, $R^4$, $R^5$ und Ar die oben angegebene Bedeutung haben,
4-unsubstituierte 1-Arylpyrazole der Formel (II),

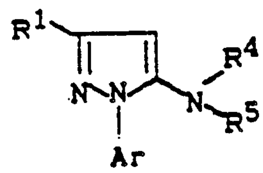

(II)

in welcher
$R^1$, $R^4$, $R^5$ und Ar die oben angegebene Bedeutung haben,
mit Sulfenylhalogeniden der Formel (III),

$R^2$-S-$Hal^1$ (III)

in welcher
$Hal^1$ für Halogen steht und
$R^2$ die oben angegebene Bedeutung hat,
gegebenenfalls in Gegenwart eines Verdünnungsmittels und gegebenenfalls in Gegenwart eines Reaktionshilfsmittels umsetzt;

(b) oder daß man zum Erhalt von 1-Arylpyrazolen der Formel (Ib),

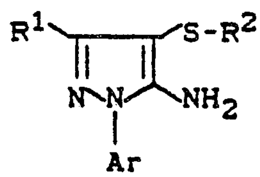

(Ib)

in welcher
$R^1$, $R^2$ und Ar die oben angegebene Bedeutung haben,
($\alpha$) Thiocyanate der Formel (IV),

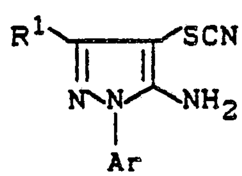

(IV)

in welcher
$R^1$ und Ar die oben angegebene Bedeutung haben,
oder
($\beta$) Disulfide der Formel (V),

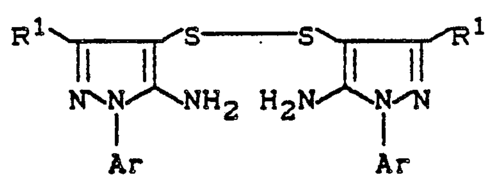

(V)

in welcher
$R^1$ und Ar die oben angegebene Bedeutung haben,
mit Halogeniden der Formel (VI),

$R^2$-$Hal^2$ (VI)

in welcher
$Hal^2$ für Halogen steht und

$R^2$ die oben angegebene Bedeutung hat, in Gegenwart eines geeigneten Reduktionsmittels gegebenenfalls in Gegenwart eines Verdünnungsmittels und gegebenenefalls in Gegenwart eines Reaktionshilfsmittels umsetzt;

(c) oder daß man zum Erhalt von 1-Arylpyrazolen der Formel (Ic),

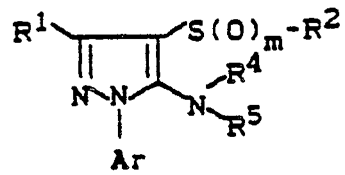

(Ic)

in welcher $R^1$, $R^2$, $R^4$, $R^5$ und Ar die oben angegebene Bedeutung haben und m für eine Zahl 1 oder 2 steht, 1-Arylpyrazole der Formel (Ia),

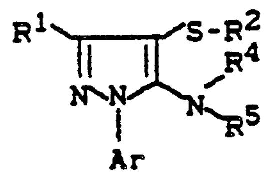

(Ia)

in welcher $R^1$, $R^2$, $R^4$, $R^5$ und Ar die oben angegebene Bedeutung haben, mit einem geeigneten Oxidationsmittel, gegebenenfalls in Gegenwart eines Verdünnungsmittels und gegebenenfalls in Gegenwart eines Reaktionshilfsmittels sowie gegebenenfalls in Gegenwart eines geeigneten Katalysators, umsetzt;

(d) oder daß man zum Erhalt von 1-Arylpyrazolen der Formel (Id),

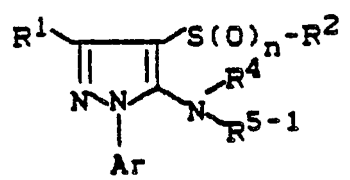

(Id)

in welcher $R^1$, $R^2$, $R^4$, Ar und n die oben angegebene Bedeutung haben und $R^{5-1}$ für Alkyl, Alkenyl oder Alkinyl steht, 1-Arylpyrazole der Formel (Ig),

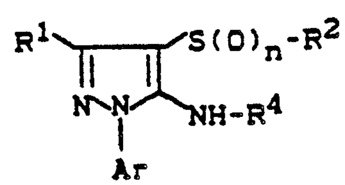

(Ig)

in welcher $R^1$, $R^2$, $R^4$, Ar und n die oben angegebene Bedeutung haben, mit Alkylierungsmitteln der Formel (VII),

$R^{5-1}$-E (VII)

in welcher $R^{5-1}$ für Alkyl, Alkenyl oder Alkinyl steht

und
E für eine elektronenanziehende Abgangsgruppe steht, gegebenenfalls in Gegenwart eines Verdünnungsmittels und gegebenenfalls in Gegenwart eines Reaktionshilfsmittels umsetzt;

(e) oder daß man zum Erhalt von 1-Arylpyrazolen der Formel (Ie),

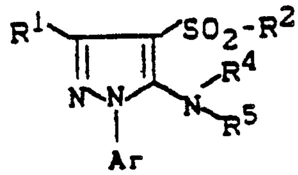

(Ie)

in welcher
$R^1$, $R^2$, $R^4$, $R^5$ und Ar die oben angegebene Bedeutung haben,
1-Arylpyrazole der Formel (Ih),

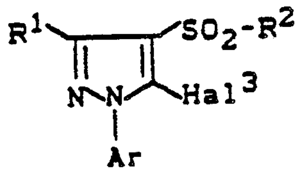

(Ih)

in welcher
$Hal^3$ für Halogen steht und
$R^1$,$R^2$ und Ar die oben angegebene Bedeutung haben,
mit Aminen der Formel (VIII),

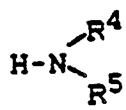

(VIII)

in welcher
$R^4$ und $R^5$ die oben angegebene Bedeutung haben,
gegebenenfalls in Gegenwart eines Verdünnungsmittels und gegebenenfalls in Gegenwart eines Reaktionshilfsmittels umsetzt;

(f) oder daß man zum Erhalt von 1-Arylpyrazolen der Formel (If),

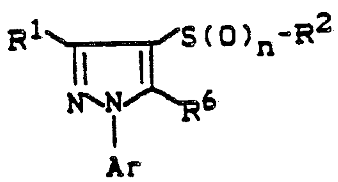

(If)

in welcher
$R^1$, $R^2$, Ar und n die oben angegebene Bedeutung haben und
$R^6$ für Wasserstoff oder Halogen steht,
1-Arylpyrazole der Formel (Ii),

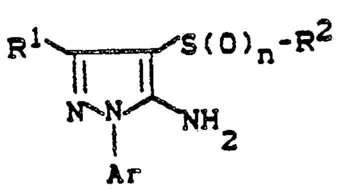

(Ii)

in welcher
$R^1$, $R^2$, Ar und n die oben angegebene Bedeutung haben,
mit einem anorganischen oder organischen Nitrit, gegebenenfalls in Gegenwart eines Reaktionshilfsmittels und gegebenenfalls in Gegenwart einer Halogenwasserstoffsäure sowie gegebenenfalls in Gegenwart eines Verdünnungsmittels, umsetzt.

6) Schädlingsbekämpfungsmittel, gekennzeichnet durch einen Gehalt an mindestens einem 1-Arylpyrazol der Formel (I).

7) Insektizide, akarizide und nematizide Mittel, gekennzeichnet durch einen Gehalt an mindestens einem 1-Arylpyrazol der Formel (I).

8) Verfahren zur Bekämpfung von Insekten und/oder Spinnentieren und/oder Nematoden, dadurch gekennzeichnet, daß man 1-Arylpyrazole der Formel (I) auf Insekten und/oder Spinnentieren und/oder Nematoden und/oder deren Lebensraum einwirken läßt.

9) Verwendung von 1-Arylpyrazolen der Formel (I) zur Bekämpfung von Insekten und/oder Spinnentieren und/oder Nematoden.

10) Verfahren zur Herstellung von Schädlingsbekämpfungsmitteln, dadurch gekennzeichnet, daß man 1-Arylpyrazole der Formel (I) mit Streckmitteln und/oder oberflächenaktiven Mitteln vermischt.